# EUROPEAN PATENT APPLICATION

(11) **EP 3 138 577 A1**
(43) Date of publication of application: **08.03.2017**
(21) Application number: 16001869.3
(22) Date of filing: 29.02.2012
(51) Int. Cl.: A61K 39/00, A61K 9/00

(54) **VACCINATION IN ELDERLY PATIENTS**

(30) Priority: 02.03.2011 WO PCT/EP2011/001043
(62) Divisional of application: 12706489.7
(71) Applicant: CureVac AG, 72076 Tübingen (DE)
(72) Inventor: KALLEN, Karl-Josef, 50226 Königsdorf (DE); KRAMPS, Thomas, 72070 Tübingen (DE); SCHNEE, Margit, 78467 Konstanz (DE); PETSCH, Benjamin, 72070 Tübingen (DE); STITZ, Lothar, 72108 Rottenburg (DE)
(74) Representative: Graf von Stosch, Andreas

(57) **Abstract**

The present invention relates to vaccines comprising at least one mRNA encoding at least one antigen for use in the treatment of a disease in an elderly patient preferably exhibiting an age of at least 50 years, more preferably of at least 55 years, 60 years, 65 years, 70 years, or older, wherein the treatment comprises vaccination of the patient and eliciting an immune response in said patient. The present invention is furthermore directed to kits and kits of parts comprising such a vaccine and/or its components and to methods applying such a vaccine or kit.

## Description

The present invention relates to vaccines comprising at least one mRNA encoding at least one antigen for use in the treatment of a disease in an elderly patient preferably exhibiting an age of at least 50 years, more preferably of at least 55 years, 60 years, 65 years, 70 years, or older, wherein the treatment comprises vaccination of the patient and eliciting an immune response in said patient. The present invention is furthermore directed to kits and kits of parts comprising such a vaccine and/or its components and to methods applying such a vaccine or kit.

As evidenced over the last decades human beings reach an ever older age and even unexpected maximal ages. This, however, is accompanied by an apparent increase of the emergence of many diseases associated with aging. As widely reviewed by Fülöp *et al.* (Fülöp et al., Clinical Interventions in Aging, 2007:2(I), 33-54) the incidence of infections, cancers, and chronic inflammatory diseases such as atherosclerosis and neurodegenerative diseases increases with age. Although one still does not know what the exact cause of aging is, it may be recognized that changes of the immune system play an important role both in the aging process and in the increase of age-related diseases.

The primary role of the immune system is to protect the organism against pathogens, but age-associated alterations to immunity increase the susceptibility of the elderly to infectious diseases but also cancers and autoimmune disorders. Evidently, the immune system is a complex interactive system composed of many different players. However, these components are not all altered in the same manner and do not contribute equally to aging. One may perhaps conceptualize immunosenescence as a dysregulation of a homeostatically constantly adapting system, the inputs and outputs of which are still only crudely defined, let alone the pathways linking these. At the whole organism level, many studies have documented changes in endocrine and neural function, cardiovascular, muscle, and skeletal health, as well as regulation of glucose metabolism. It must be borne in mind that these diverse physiological changes also affect the immune system, although very few investigations address these issues, especially in humans. Although numerous studies on age-associated immune alterations exist (collectively known as "immunosenescence") the exact nature of these is still controversial because of differences between species, the lack of definition of physiological aging rendering difficult the exclusion of some latent disease states, nutritional, genetic, and environmental differences, amongst other factors. However, the clinical consequences of the decreased immune response with aging seem quite clear. These are mainly the increased incidence and severity of infections, but also cancers and autoimmune disorders. Many elderly subjects actually die from infections even if the cause of death given by the attending physician is very different.

The exact nature of these changes is still controversial, but the use of screening procedures, such as the SENIEUR protocol to exclude underlying illness, helped to better characterize the changes actually related to physiological aging rather than pathology (see Fülöp *et al.* (2007, supra)). The hallmark of immunosenescence is the overwhelming decrease in T cell function with aging. In this context, it is generally agreed that the most marked changes occur in the cellular immune response reflecting profound alterations in T cells. Much of this is due to thymic involution as well as changes in the proportions of T cell subpopulations resulting from antigen exposure, and altered T cell activation pathways. There are also changes in the other parts of the immune system, but they are much less marked, and may often be secondary to changes in the T cells (not only the T cell-dependent B cells, but also innate components sensitive to T cell feedback, especially antigen-presenting cells). Such age-related changes of the immune response are multifactorial, but it is reasonable to think that the extra-cellular milieu is very important. Furthermore, a body of data indicates that innate immune responses, including the critical bridge between innate and adaptive immunity, and antigen presenting capacity are not completely resistant to senescence processes. The consequences of all these alterations are an increased incidence of infections, as well as possibly cancers, autoimmune disorders, and chronic inflammatory diseases (see Fülöp *et al.* (2007, supra)).

Profound alterations in T cells as mentioned above in particular concern CD4+ T and CD8+ T cells. Whereas the function of aged CD4+ T cells has been extensively investigated and distinct defects defined, the impact of aging on CD8+ T cell function is poorly understood. It has been suggested that apparent declines in CD8+ T cell effector function may instead be the consequence of age-associated changes in the composition of the CD8+ T cell pool, consistent with reports that naive CD8+ T cells from aged mice are fully functional. The ability of individuals to generate effective T cell responses to newly encountered infections and to respond to vaccination requires the maintenance of a diverse repertoire of T cells. Thus, it has been speculated that declining T cell repertoire diversity associated with aging is a contributing factor to the impaired ability of aged individuals to mount effective immune responses to infections and vaccines. The functional diversity of the αβTCR repertoire in the spleens of young mice has been estimated to be - 2 x 10⁶ clones, with - 10 cells per clone. However, several age-associated changes are thought to lead to reductions in both the size and diversity of the naive T cell repertoire. Fewer T cells are produced in the thymus, leading to reduced numbers of naive T cells in the periphery. The naive T cell repertoire also becomes increasingly constrained by the progressive accumulation of peripheral T cells exhibiting a memory phenotype, believed to be the result of the accumulated antigen experience of the individual. The diversity of the memory repertoire is further compromised by the development of age-associated CD8+ T cell clonal expansions, which can comprise 70 - 80% or more of the total CD8+ T cell compartment in some aged animals. Collectively, declining numbers and diversity of naive T cells emerging from the aged thymus, progressive increase in the proportion of antigen-experienced compared with naive T cells, and the development of large clonal expansions result in substantially reduced diversity among CD8+ T cells in aged mice (see Yager et al., JEM, Vol. 205, March 17, 2008).

In this context, Yager *et al.* (2008, supra) show in a study with a well-characterized influenza virus model the impact of aging on the diversity of the antiviral CD8+ T cell response. The data of Yager *et al.* (2008, supra) provide experimental evidence that the age-associated decline in CD8+ T cell repertoire diversity can greatly impact the response to new infections, and the development of heterosubtypic immunity. Importantly, perturbations in the repertoire of T cells specific for influenza virus epitopes for which there is a low precursor frequency and limited TCR diversity, lead to the selective development of holes in the repertoire for a typically immunodominant viral epitope. T cell repertoire is frequently characterized by either exhaustive sequence analysis of individual cells or spectratype analysis of bulk populations of T cells, and different information is obtained by the two techniques. Further to alterations in the CD4+ and CD8+ T cell response a shift from Th1 cytokines (including IFN-γ) to Th2 cytokines (including IL-10) with aging has been observed and associated with reduced CTL activity and diminished protection against influenza virus challenge. To be noted, influenza is cleared by CTL through granule-mediated killing of virus-infected host cells. In this process, granzyme B (GrB) is regarded as a key cytolytic mediator and an early marker of the CTL response to influenza infection. Virus-specific CTL are recruited to influenza-infected lungs by a Th1 response, specifically due to the production of IFN-γ. Although influenza specific Th2 cytokines including IL-10 do not promote recovery from influenza infection, these cytokines continue to be expressed in high levels at the site of influenza infection. Thus, a balance between Th1 and Th2 cytokines also appears to be important for viral clearance (see McElhaney et al., The Journal of Immunology, 2006, 176: 6333-6339).

Not only immunosenescence in T cell responses but also in B-cell responses appear to exhibit a severe impact on immunoprotection in elderly patients. B-cell responses in later life are mainly characterized by their serological profiles (see Claire-Anne Siegrist and Richard Aspinall, Nature Reviews|Immunology, Volume 9, March 2009, pp. 185-194). As discussed by Siegrist and Aspinall (2009), successful recovery from viral and bacterial infections typically requires a good humoral immune response, as shown by the increased levels of specific antibodies following infection. Surviving to old age requires a successful response to a wide range of potential pathogens and the generation of a prodigious 'reservoir' of immunological memory. Measurable titres of serum antibody are stable and have the capacity to be maintained for prolonged periods. Antibody responses to viruses, such as varicellazoster, measles and mumps, have half-lives of 50 years or more, whereas antibody responses to non-replicating protein antigens, such as tetanus and diphtheria toxins, decline with half-lives of 10-20 years. This suggests that the persistence of antigen contributes to the continued production of antibody, as indicated by studies in mice. Unfortunately, the capacity to respond to new potential pathogens does not increase at an exponential rate throughout life. Indeed, an inability to respond to new antigens can be found in elderly patients, albeit for mechanistically different reasons. Evidence for this in older individuals comes from the measurement of changes in specific antibody levels at defined times after vaccination. As an example, the ability of influenza vaccine to induce protection is related to age, with an efficacy between 70% and 90% in those under 65 years of age, but of 30% to 40% at best for those over 65 years of age. Similarly, responses to pneumococcal polysaccharides and hepatitis B vaccines are compromised by old age, and antibody responses are of shorter duration in healthy older people (see Siegrist and Aspinall (2009, supra)).

Epidemiological studies furthermore have shown that populations of older individuals have increasing titres of autoantibody as they age. These autoantibody titres are not usually associated with autoimmune disease, possibly because of the low affinity of the antibodies. More than half of healthy older individuals have antibodies for non-organspecific autoantigens (such as nucleoprotein or IgG), which might result from their generation as a byproduct of responses to other antigens. Indeed, an early study showed that shortly after vaccination with tetanus toxoid, there was a significant increase in the frequency of B cells producing rheumatoid factor, accompanied by an increase in plasma levels of IgM rheumatoid factor. There is also an age-dependent increase in the prevalence of organspecific antibodies (such as thyroid-specific antibodies). The increase does not seem to be exponential, as studies showed that the prevalence of organ-specific auto antibodies in centenarians is similar to that found in individuals under 50 years of age. Further changes in the serological profile in older individuals were revealed by the presence of a single spike on electrophoresis gels within the region of the gel that is associated with immunoglobulins. These paraproteins are produced by a clone of plasma cells with a defined single specificity - a condition known as benign monoclonal gammopathy. The prevalence of this condition increases with age, with 3.2% of individuals over 50 years of age suffering from the disease, 5.3% of those over 70 years of age and 7.5% of those over 85 years of age. The differences in the serum antibody profile between these age groups include the increase in the amount of serum immunoglobulin between elderly individuals, the increased presence of autoantibodies and low affinity antibodies in the elderly and the occurrence of an over-representation of specific classes of antibody from individual B-cell clones in some older individuals (see Siegrist *et al.* (2009, supra).

Alterations in T cell responses and also in B-cell responses as discussed above exhibit a significant effect not only on immunoprotection towards pathogens but also to vaccination strategies in elderly patients when combating infectious diseases but possibly also cancers and autoimmune disorders. Accordingly, it was not surprising that similar observations were also made in the treatment of elderly cancer patients, preferably when using vaccines of the art.

One previous approach to overcome theses deficiencies in the context of virus-based influenza vaccines refers to the administration of hemagglutinin (HA) and/or nucleoprotein (NP) influenza viral genes (see Radu et al., Viral Immunology, Vol. 12, Number 3, 1999). Radu *et al.* (1999) discuss the finding that despite the greater antibody- and cell-mediated immune responses and complete protection afforded by the recombinant vaccinia vaccines to old mice, concerns about the toxicity of such vaccinia based vaccines have prevented their development as human vaccines. Accordingly, Radu *et al.* (1999) suggest that immunization with a recombinant vaccinia virus expressing the influenza hemagglutinin gene may overcome the age-associated defects in the immune response of old-mice and may elicit complete protection from influenza challenge in old mice. Radu *et al.* (1999) provided DNA-based plasmid vaccines expressing hemagglutinin (HA) and nucleoprotein (NP) influenza viral genes and argue that such DNA-based plasmid vaccines (pHA and pNP, respectively) have been shown to be safe and effective in inducing protective immunity to influenza in various animal species. They conclude that old and adult mice vaccinated with pHA were protected to a comparable extend from lethal challenge with live WSN influenza virus. They furthermore conclude that continuous production of antigen after DNA vaccination primes B and T cells in old and young mice to a comparable extent although the diversity of the antibody response in old mice was limited compared to adult mice. Such a strategy however, even though promising at the time of carrying out the above experiments, still requires administration of DNA based vectors, which have been encountered as dangerous due to unwanted insertion into the genome. Such DNA based vectors may even lead to interruption of functional genes and cancer or the formation of anti-DNA antibodies and are therefore out of focus as of today.

One further more actual strategy to combat e.g. infectious diseases with vaccines in elderly patients is based on the delivery of current vaccines or new vaccine candidates in combination with adjuvants. However, there are only few adjuvants approved for human use. One major adjuvant approved for human use is e.g. Alum, an aluminium salt based adjuvant. However, although approved for human use, such aluminium salts failed to provide satisfactory augmentation of immune responses for seasonal influenza vaccines in early human clinical trials, an effect, which may be expected likewise during other vaccination strategies. Further licensed adjuvanted influenza vaccines include to date Fluad^{®} (Novartis Vaccines), containing MF59 in combination with a subunit vaccine formulation, and the virosomal vaccines Inflexal^{®}V (Berna Biotech, a Crucell company) and Invivac^{®} (Solvay). Although animal studies and human clinical trials revealed a higher immunogenicity profile - defined as increased antibody responses - with the MF59-adjuvanted influenza vaccine, MF59 is not a potent adjuvant for the induction of type 1 driven cellular immune responses. Unlike Fluad^{®}, the virosomal vaccines represent reconstituted influenza virus envelopes containing the functional influenza surface proteins haemagglutinin and neuraminidase in their phospholipid bilayer. The immunogenicity and local tolerability of virosome-based influenza vaccines has been shown in several studies. However, the development of virosomal formulations is very complex and the costs of goods are high.

In this context, Riedl *et al.* (see Riedl. et al., Vaccine 26 (2008), 3461-3468) reported the development of a synthetic two component adjuvant IC31^{®} with characteristics that are likely to contribute to improved influenza vaccines. IC31^{®} is a mixture of a novel immunostimulatory oligodeoxynucleotide containing deoxy-Inosine/deoxy-Cytosine (ODN1a) and the peptide KLKL5KLK (KLK). Apart from effective vaccine depot formation mediated by KLK, IC31^{®} induces activation of antigen presenting cells and strongly stimulates both T and B cell responses with type 1 dominance when combined with different types of antigens. In these studies the immunostimulatory effect of IC31^{®} on seasonal subunit influenza vaccines was investigated. Riedl *et al.* (2008, supra) present evidence for antigen dose-sparing and induction of type 1 humoral and cellular responses that are long lasting and apparent even in aged animals. As shown in their experiments no immune enhancement was found at day 21 or 80 by measuring HI antibodies upon single vaccination of aged mice with low amounts of influenza vaccine in combination with IC31^{®}. However, boosting on day 21 with the same amount of IC31^{®}-cantaining influenza vaccine dramatically increased HI titres against all three strains 3 weeks post booster immunisation compared to administration of the vaccine alone. Although the immune response was significantly increased, the strategy as outlined by Riedl *et al.* (2008, supra) still requires booster administrations following administration of IC31^{®} improved influenza vaccines. Furthermore, the effect appears to be at least in part dependent on prior exposition of the patient to be treated to specific influenza strains and thus may not resemble an immune response to an entirely novel pathogen. It may also not reflect the requirements of vaccination strategies as e.g. in the treatment of cancer or autoimmune diseases.

Investigations have also been carried out with respect to cancer vaccines. Gravekamp (see Claudia Gravekamp, Exp Gerontol. 2007 May; 42(5): 441-450) reviews the current knowledge of T cell unresponsiveness in cancer patients and elderly, the results of cancer vaccination in preclinical models and in clinical trials, and recent data of cancer vaccination at old age in preclinical models. Finally, Gravekamp (2007, supra) proposes experimental approaches to provide cancer vaccines more effective at older age. They developed a DNA vaccine of Mage-b and tested this vaccine at young and old age in a syngeneic mouse tumour model, 4TO7cg. This mouse tumour model is moderately metastatic (range: 2-20 metastases per mouse) and overexpresses Mage-b in primary tumour and metastases (Sypniewska *et al.,* 2005). Preventive vaccination of young and old mice with pcDNA3.1-Mage-b protected 90% of the young mice from metastases, while only 60% of the old mice remained free of metastases. Analysis of spleen cells of tumour-bearing mice after in vitro restimulation, showed high levels of IL-2 and IFN-γ at young age but undetectable levels at old age, indicating a poor immune response in old mice. Gravekamp (2007, supra) also repeated this vaccine study in a much more aggressive metastatic model 4T1 (range: 5-300 metastases per mouse), also overexpressing Mage-b, and mixed the pcDNA3.1-Mage-b DNA vaccine with plasmid DNA secreting GM-CSF. To recruit APC more effectively to the peritoneal cavity (pc), thioglycollate was injected into the pc, prior to each vaccination. Although the effect in the young mice was stronger than in the old, a significant reduction in the frequency of metastases was observed in both young and old mice. However, when analyzing the draining lymph nodes of tumour-bearing mice, moderate levels of IL-2 and IFN-γ still were detected after restimulation at young age but not at old age. FACS analysis of the draining lymph nodes of Mage-b vaccinated tumour-bearing mice at young and old age after restimulation, also showed CD4+ and CD8+ responses (intracellular IL-2 and/or IFN-γ production) at young age but not at old age. At old age macrophages and NK cells were more active (intracellular production of IFN-γ and IL-2 receptor expression), suggesting that the innate immune response may have contributed to the antitumour response in mice. Gravekamp (2007, supra) thus show that cancer vaccines may not be very effective in cancer patients, which are usually elderly, unless the vaccines are optimized for older age. Summarizing the above, there is an urgent need for vaccines optimized for patients of older age. More precisely, vaccines are required, which lead to good immune response and do not bear the problems shown in the prior art or at least diminish these problems to a significant extent. Furthermore, it is highly envisaged to provide vaccines, which allow inducing Th1 immune responses in elderly patients, preferably without leading to a shift from Th1 to Th2 immune responses subsequent to administration. Likewise, the administration of DNA based vaccines should be avoided due to possible insertion of DNA into the genome, possible interruption of genes and formation of anti-DNA antibodies.

The object underlying the present invention is solved by the subject matter of the attached claims, more preferably as outlined in the following.

According to a first embodiment, the object underlying the present invention is solved by a vaccine comprising at least one mRNA encoding at least one antigen for use in the prophylaxis and/or treatment of a disease in an elderly patient exhibiting an age of at least 50 years, more preferably of at least 55 years, 60 years, 65 years, 70 years, or older, wherein the treatment comprises vaccination of the patient and eliciting an immune response in said patient.

Without being bound to theory RNA vaccines elegantly integrate adjuvanticity and antigen expression, thereby mimicking relevant aspects of viral infections. This increases their efficacy compared to other inactivated (dead) vaccines, that require the use of advanced adjuvants in the elderly, simplifying handling and production. RNA can address a range of dedicated immunologic pattern recognition receptors, including toll-like receptors 3, 7, and 8, RIG-I, MDA5, PKR, and others that may act synergistically and serve to enhance the induction of antigen-specific adaptive B and T cell responses. Importantly, by antigen synthesis in transfected host cells, mRNA vaccines directly introduce antigen into cellular antigen processing and presentation pathways, granting access to MHC molecules and triggering T cell responses, irrespective of the hosts MHC haplotype. This enables the induction of polyclonal T cell responses that may act synergistically with other immune responses, including B cells. Also, presenting the full spectrum of MHC-binding epitopes may circumvent limitations imposed by "holes" in the elderly T cell repertoire. Also, endogenous production of antigen ensures faithful posttranslational modification (e.g. proteolytic processing, glycosylation, etc.) that may positively impact immunogenicity. Also, RNA vaccines exhibit safety features that make them superior for use in the elderly. For example, the increased reactogenicity of live attenuated vaccines generally prevents use in this highly relevant target group, i.e. persons of at least 50 years of age, but also persons suffering from chronic conditions such as asthma or from a severe disease, such as cancer. However, considering the short persistence and traceless decay of the vaccine vector within a matter of days the observed good immunogenicity is unexpected and contrasts claims for plasmid DNA vaccines that variously linked efficacy to the persistent expression of antigen.

The at least one mRNA of the inventive vaccine as defined in the first embodiment of the present invention, encoding at least one antigen, may be selected from any antigen, known to a skilled person, preferably suitable to elicit an antigen-specific immune response in a patient. According to the present invention, the term "antigen" refers to a substance which is recognized by the immune system and is capable of triggering an antigen-specific immune response, e.g. by formation of antibodies or antigen-specific T cells as part of an adaptive immune response. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by CD8+ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which do not recognize and bind antigen directly, but instead recognize short peptide fragments e.g. of pathogens' protein antigens, which are bound to MHC molecules on the surfaces of other cells.

In the context of the present invention, antigens as encoded by the at least one mRNA of the inventive vaccine typically comprise any antigen, falling under the above definition, more preferably protein and peptide antigens. In accordance with the invention, antigens as encoded by the at least one mRNA of the inventive vaccine may be antigens generated outside the cell, more typically antigens not derived from the host organism (e.g. a human) itself (i.e. non-self antigens) but rather derived from host cells outside the host organism, e.g. pathogenic antigens, particularly viral antigens, bacterial antigens, fungal antigens, protozoological antigens, animal antigens (preferably selected from animals or organisms as disclosed herein), allergy antigens, etc. Antigens as encoded by the at least one mRNA of the inventive vaccine may be furthermore antigens generated inside the cell, the tissue or the body, e.g. by secretion of proteins, their degradation, metabolism, etc. Such antigens include antigens derived from the host organism (e.g. a human) itself, e.g. tumour antigens, self-antigens or auto-antigens, such as auto-immune self-antigens, etc., but also (non-self) antigens as defined above, which have been originally been derived from host cells outside the host organism, but which are fragmented or degraded inside the body, tissue or cell, e.g. by (protease) degradation, metabolism, etc.

Pathogenic antigens particularly comprise e.g. antigens from influenza, preferably influenza A, influenza B, influenza C or thogotovirus, preferably influenza antigens haemagglutinin (HA) and/or neuraminidase (NA), preferably influenza antigens derived from haemagglutinin subtypes H1, H2, H3, H4, H5, H6, H7, H8, H9, H10, H11, H12, H13, H14 or H15, and/or neuraminidase subtypes N1, N2, N3, N4, N5, N6, N7, N8 or N9, or preferably selected from influenza-A subtypes H1N1, H1N2, H2N2, H2N3, H3N1, H3N2, H3N3, H5N1, H5N2, H7N7 or H9N2, or any further combination, or from matrix protein 1 (M1), ion channel protein M2 (M2), nucleoprotein (NP), etc; or e.g. antigens from respiratory syncytial virus (RSV), including F-protein, G-protein, etc.

One further class of antigens as encoded by the at least one mRNA of the inventive vaccine comprises allergy antigens. Allergy antigens are typically antigens, which cause an allergy in a human and may be derived from either a human or other sources. Such allergy antigens may be selected from antigens derived from different sources, e.g. from animals, plants, fungi, bacteria, etc. Allergens in this context also include antigens derived from e.g. grasses, pollens, molds, drugs, or numerous environmental triggers, etc. Allergy antigens typically belong to different classes of compounds, such as proteins or peptides and their fragments, carbohydrates, polysaccharides, sugars, lipids, phospholipids, etc. Of particular interest in the context of the present invention are antigens, which are encoded by the at least one mRNA of the inventive vaccine, i.e. protein or peptide antigens and their fragments or epitopes, or nucleic acids and their fragments, particularly nucleic acids and their fragments, encoding such protein or peptide antigens and their fragments or epitopes.

Particularly preferred, antigens derived from animals, which are encoded by the at least one mRNA of the inventive vaccine, may include antigens derived from, without being limited thereto, insects, such as mite (e.g. house dust mites), mosquito, bee (e.g. honey bee, bumble bee), cockroach, tick, moth (e.g. silk moth), midge, bug, flea, wasp, caterpillar, fruit fly, migratory locust, grasshopper, ant aphide, from crustaceans, such as shrimps, crab, krill, lobster, prawn, crawfish, scampi, from birds, such as duck, goose, seagull, turkey, ostrich, chicken, from fishes, such as eel, herring, carp, seabream, codfish, halibut, catfish, beluga, salmon, flounder, mackerel, cuttlefish, perch, form molluscs, such as scallop, octopus, abalone, snail, whelk, squid, clam, mussel, from spiders, from mammals, such as cow, rabbit, sheep, lion, jaguar, leopard, rat, pig, buffalo, dog, loris, hamster, guinea pig, fallow deer, horse, cat, mouse, ocelot, serval, from arthropod, such as spider, or silverfish, from worms, such as nematodes, from trichinella species, or roundworm, from amphibians, such as frogs, or from sea squirt, etc. Antigens derived from animals may also comprise antigens contained in animal products, preferably contained in animal products derived from animals as defined above, e.g. milk, eggs, meat, etc., but also from excrements or precipitates of any kind, derived from any of these animals.

Most preferably, antigens derived from animals, which are encoded by the at least one mRNA of the inventive vaccine, may include antigens derived from such animals, causing a disease as defined herein, preferably an infectious disease or an autoimmune disease as defined herein, or any further disease as defined herein.

Antigens derived from plants, which are encoded by the at least one mRNA of the inventive vaccine, may include antigens derived from, without being limited thereto, fruits, such as kiwi, pineapple, jackfruit, papaya, lemon, orange, mandarin, melon, sharon fruit, strawberry, lychee, apple, cherry paradise apple, mango, passion fruit, plum, apricot, nectarine, pear, passion fruit, raspberry, grape, from vegetables, such as garlic, onion, leek, soya bean, celery, cauliflower, turnip, paprika, chickpea, fennel, zucchini, cucumber, carrot, yam, bean, pea, olive, tomato, potato, lentil, lettuce, avocado, parsley, horseradish, chirimoya, beet, pumkin, spinach, from spices, such as mustard, coriander, saffron, pepper, aniseed, from crop, such as oat, buckwheat, barley, rice, wheat, maize, rapeseed, sesame, from nuts, such as cashew, walnut, butternut, pistachio, almond, hazelnut, peanut, brazil nut, pecan, chestnut, from trees, such as alder, hornbeam, cedar, birch, hazel, beech, ash, privet, oak, plane tree, cypress, palm, from flowers, such as ragweed, carnation, forsythia, sunflower, lupine, chamomile, lilac, passion flower, from grasses, such as quack grass, common bent, brome grass, Bermuda grass, sweet vernal grass, rye grass, or from other plants, such as opium poppy, pellitory, ribwort, tobacco, asparagus, mugwort, cress, etc.

Antigens derived from fungi, which are encoded by the at least one mRNA of the inventive vaccine, may include antigens derived from, without being limited thereto, e.g. *Alternia sp., Aspergillus sp., Beauveria sp., Candida sp., Cladosporium sp., Endothia sp., Curcularia sp., Embellisia sp., Epicoccum sp., Fusarium sp., Malassezia sp., Penicillum sp., Pleospora sp., Saccharomyces sp.,* etc.

Antigens derived from bacteria, which are encoded by the at least one mRNA of the inventive vaccine, may include antigens derived from, without being limited thereto, e.g. *Bacillus tetani, Staphylococcus aureus, Streptomycesgriseus,* etc.

One further class of antigens as encoded by the at least one mRNA of the inventive vaccine comprises tumour antigens. "Tumour antigens" are preferably located on the surface of the (tumour) cell. Tumour antigens may also be selected from proteins, which are overexpressed in tumour cells compared to a normal cell. Furthermore, tumour antigens also include antigens expressed in cells which are (were) not themselves (or originally not themselves) degenerated but are associated with the supposed tumour. Antigens which are connected with tumour-supplying vessels or (re)formation thereof, in particular those antigens which are associated with neovascularization, e.g. growth factors, such as VEGF, bFGF etc., are also included herein. Antigens connected with a tumour furthermore include antigens from cells or tissues, typically embedding the tumour. Further, some substances (usually proteins or peptides) are expressed in patients suffering (knowingly or not-knowingly) from a cancer disease and they occur in increased concentrations in the body fluids of said patients. These substances are also referred to as "tumour antigens", however they are not antigens in the stringent meaning of an immune response inducing substance. The class of tumour antigens can be divided further into tumour-specific antigens (TSAs) and tumour-associated-antigens (TAAs). TSAs can only be presented by tumour cells and never by normal "healthy" cells. They typically result from a tumour specific mutation. TAAs, which are more common, are usually presented by both tumour and healthy cells. These antigens are recognized and the antigen-presenting cell can be destroyed by cytotoxic T cells. Additionally, tumour antigens can also occur on the surface of the tumour in the form of, e.g., a mutated receptor. In this case, they can be recognized by antibodies. According to the invention, the terms "cancer diseases" and "tumour diseases" are used synonymously herein.

Examples of tumour antigens as encoded by the at least one mRNA of the inventive vaccine may comprise e.g. antigens selected from the group comprising, without being limited thereto, 5T4, 707-AP (707 alanine proline), 9D7, AFP (alpha-fetoprotein), AlbZIP HPG1, alpha5beta1-Integrin, alpha5beta6-Integrin, alpha-methylacyl-coenzyme A racemase, ART-4 (adenocarcinoma antigen recognized by T cells 4), B7H4, BAGE-1 (B antigen), BCL-2, BING-4, CA 15-3/CA 27-29, CA 19-9, CA 72-4, CA125, calreticulin, CAMEL (CTL-recognized antigen on melanoma), CASP-8 (caspase-8), cathepsin B, cathepsin L, CD19, CD20, CD22, CD25, CD30, CD33, CD40, CD52, CD55, CD56, CD80, CEA (carcinoembryonic antigen), CLCA2 (calcium-activated chloride channel-2), CML28, Coactosin-like protein, Collagen XXIII, COX-2, CT-9/BRD6 (bromodomain testis-specific protein), Cten (C-terminal tensin-like protein), cyclin B1, cyclin D1, cyp-B (cyclophilin B), CYPB1 (cytochrom P450 181), DAM-10/MAGE-B1 (differentiation antigen melanoma 10), DAM-6/MAGE-B2 (differentiation antigen melanoma 6), EGFR/Her1, EMMPRIN (tumour cell-associated extracellular matrix metalloproteinase inducer/), EpCam (epithelial cell adhesion molecule), EphA2 (ephrin type-A receptor 2), EphA3 (ephrin type-A receptor 3), ErbB3, EZH2 (enhancer of Zeste homolog 2), FGF-5 (fibroblast growth factor-5), FN (fibronectin), Fra-1 (Fos-related antigen-1), G250/CAIX (glycoprotein 250), GAGE-1 (G antigen 1), GAGE-2 (G antigen 2),

GAGE-3 (G antigen 3), GAGE-4 (G antigen 4), GAGE-5 (G antigen 5), GAGE-6 (G antigen 6), GAGE-7b (G antigen 7b), GAGE-8 (G antigen 8), GDEP (gene differentially expressed in prostate), GnT-V (N-acetylglucosaminyltransferase V), gp100 (glycoprotein 100 kDa), GPC3 (glypican 3), HAGE (helicase antigen), HAST-2 (human signet ring tumour-2), hepsin, Her2/neu/ErbB2 (human epidermal receptor-2/neurological), HERV-K-MEL, HNE (human neutrophil elastase), homeobox NKX 3.1, HOM-TES-14/SCP-1, HOM-TES-85, HPV-E6, HPV-E7, HST-2, hTERT (human telomerase reverse transcriptase), iCE (intestinal carboxyl esterase), IGF-1R, IL-13Ra2 (interleukin 13 receptor alpha 2 chain), IL-2R, IL-5, immature laminin receptor, kallikrein 2, kallikrein 4, Ki67, KIAA0205, KK-LC-1 (Kita-kyushu lung cancer antigen 1), KM-HN-1, LAGE-1 (L antigen), livin, MAGE-A1 (melanoma antigen-A1), MAGE-A10 (melanoma antigen-A10), MAGE-A12 (melanoma antigen-A12), MAGE-A2 (melanoma antigen-A2), MAGE-A3 (melanoma antigen-A3), MAGE-A4 (melanoma antigen-A4), MAGE-A6 (melanoma antigen-A6), MAGE-A9 (melanoma-antigen-A9), MAGE-B1 (melanoma-antigen-B1), MAGE-B10 (melanoma-antigen-B10), MAGE-B16 (melanoma-antigen-B16), MAGE-B17 (melanoma-antigen-B17), MAGE-B2 (melanoma-antigen-B2), MAGE-B3 (melanoma-antigen-B3), MAGE-B4 (melanoma-antigen-B4), MAGE-B5 (melanoma-antigen-B5), MAGE-B6 (melanoma-antigen-B6), MAGE-C1 (melanoma-antigen-C1), MAGE-C2 (melanoma-antigen-C2), MAGE-C3 (melanoma-antigen-C3), MAGE-D1 (melanoma-antigen-D1), MAGE-D2 (melanoma-antigen-D2), MAGE-D4 (melanoma-antigen-D4), MAGE-E1 (melanoma-antigen-E1), MAGE-E2 (melanoma-antigen-E2), MAGE-F1 (melanoma-antigen-F1), MAGE-H1 (melanoma-antigen-H1), MAGEL2 (MAGE-like 2), mammaglobin A, MART-1/Melan-A (melanoma antigen recognized by T cells-1/melanoma antigen A), MART-2 (melanoma antigen recognized by T cells-2), matrix protein 22, MC1R (melanocortin 1 receptor), M-CSF (macrophage colony-stimulating factor gene), mesothelin, MG50/PXDN, MMP 11 (M-phase phosphoprotein 11), MN/CA IX-antigen, MRP-3 (multidrug resistance-associated protein 3), MUC1 (mucin 1), MUC2 (mucin 2), NA88-A (NA cDNA clone of patient M88), N-acetylglucos-aminyltransferase-V, Neo-PAP (Neo-poly(A) polymerase), NGEP, NMP22, NPM/ALK (nucleophosmin/anaplastic lymphoma kinase fusion protein), NSE (neuron-specific enolase), NY-ESO-1 (New York esophageous 1), NY-ESO-B, OA1 (ocular albinism type 1 protein), OFA-iLRP (oncofetal antigen-immature laminin receptor), OGT (O-linked N-acetylglucosamine transferase gene), OS-9, osteocalcin, osteopontin, p15 (protein 15), p15, p190 minor bcr-abl, p53, PAGE-4 (prostate GAGE-like protein-4), PAI-1 (plasminogen acitvator inhibitor 1), PAI-2 (plasminogen acitvator inhibitor 2), PAP (prostate acic phosphatase), PART-1, PATE, PDEF, Pim-1-Kinase, Pin1 (Propyl isomerase), POTE, PRAME (preferentially expressed antigen of melanoma), prostein, proteinase-3, PSA (prostate-specific antigen), PSCA, PSGR, PSM, PSMA (prostate-specific membrane antigen), RAGE-1 (renal antigen), RHAMM/CD168 (receptor for hyaluronic acid mediated motility), RU1 (renal ubiquitous 1), RU2 (renal ubiquitous 1), S-100, SAGE (sarcoma antigen), SART-1 (squamous antigen rejecting tumour 1), SART-2 (squamous antigen rejecting tumour 1), SART-3 (squamous antigen rejecting tumour 1), SCC (squamous cell carcinoma antigen), Sp17 (sperm protein 17), SSX-1 (synovial sarcoma X breakpoint 1), SSX-2/HOM-MEL-40 (synovial sarcoma X breakpoint), SSX-4 (synovial sarcoma X breakpoint 4), STAMP-1, STEAP (six transmembrane epithelial antigen prostate), surviving, survivin-2B (intron 2-retaining survivin), TA-90, TAG-72, TARP, TGFb (TGFbeta), TGFbRII (TGFbeta receptor II), TGM-4 (prostate-specific transglutaminase), TRAG-3 (taxol resistant associated protein 3), TRG (testin-related gene), TRP-1 (tyrosine related protein 1), TRP-2/6b (TRP-2/novel exon 6b), TRP-2/INT2 (TRP-2/intron 2), Trp-p8, Tyrosinase, UPA (urokinase-type plasminogen activator), VEGF (vascular endothelial growth factor), VEGFR-2/FLK-1 (vascular endothelial growth factor receptor-2), WT1 (Wilm' tumour gene), or may comprise e.g. mutant antigens expressed in cancer diseases selected from the group comprising, without being limited thereto, alpha-actinin-4/m, ARTC1/m, bcr/abl (breakpoint cluster region-Abelson fusion protein), beta-Catenin/m (beta-Catenin), BRCA1/m, BRCA2/m, CASP-5/m, CASP-8/m, CDC27/m (cell-division-cycle 27), CDK4/m (cyclin-dependent kinase 4), CDKN2A/m, CML66, COA-1/m, DEK-CAN (fusion protein), EFTUD2/m, ELF2/m (Elongation factor 2), ETV6-AML1 (Ets variant gene6/acute myeloid leukemia 1 gene fusion protein), FN1/m (fibronectin 1), GPNMB/m, HLA-A*0201-R170] (arginine to isoleucine exchange at residue 170 of the alpha-helix of the alpha2-domain in the HLA-A2 gene), HLA-A11/m, HLA-A2/m, HSP70-2M (heat shock protein 70-2 mutated), KIAA0205/m, K-Ras/m, LDLR-FUT (LDR-Fucosyltransferase fusion protein), MART2/m, ME1/m, MUM-1/m (melanoma ubiquitous mutated 1), MUM-2/m (melanoma ubiquitous mutated 2), MUM-3/m (melanoma ubiquitous mutated 3), Myosin class I/m, neo-PAP/m, NFYC/m, N-Ras/m, OGT/m, OS-9/m, p53/m, PmI/RARa (promyelocytic leukemia/retinoic acid receptor alpha), PRDX5/m, PTPRK/m (receptor-type protein-tyrosine phosphatase kappa), RBAF600/m, SIRT2/m, SYT-SSX-1 (synaptotagmin I/synovial sarcoma X fusion protein), SYT-SSX-2 (synaptotagmin I/synovial sarcoma X fusion protein), TEL-AML1 (translocation Ets-family leukemia/acute myeloid leukemia 1 fusion protein), TGFbRII (TGFbeta receptor II), TPI/m (triosephosphate isomerase). According to a specific aspect, however, mRNAs encoding antigens gp100, MAGE-A1, MAGE-A3, MART-1/melan-A, survivin, and/or tyrosinase, more preferably mRNAs encoding antigens gp100, MAGE-A1, MAGE-A3, MART-1/melan-A, survivin, and/or tyrosinase, wherein the mRNAs have been complexed with or stabilized with protamine (e.g. in a ratio of about 80 µg mRNA and 128 µg protamine), may be excluded from the scope of invention. In a preferred aspect the tumour antigens as encoded by the at least one mRNA of the inventive vaccine are selected from the group consisting of 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD40, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R171, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class l/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, S1RT2/m, Sp17, SSX-1, SSX-2/HOM-MEL-40, SSX-4, STAMP-1, STEAP, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGF, VEGFR-2/FLK-1, and WT1.

According to a particularly preferred aspect, tumour antigens as encoded by the at least one mRNA of the inventive vaccine are selected from the group consisting of MAGE-A1 (e.g. MAGE-A1 according to accession number M77481), MAGE-A2, MAGE-A3, MAGE-A6 (e.g. MAGE-A6 according to accession number NM_005363), MAGE-C1, MAGE-C2, melan-A (e.g. melan-A according to accession number NM_0055111), GP100 (e.g. GP100 according to accession number M77348), tyrosinase (e.g. tyrosinase according to accession number NM_000372), survivin (e.g. survivin according to accession number AF077350), CEA (e.g. CEA according to accession number NM_004363), Her-2/neu (e.g. Her-2/neu according to accession number M11730), WT1 (e.g. WT1 according to accession number NM_000378), PRAME (e.g. PRAME according to accession number NM_006115), EGFRI (epidermal growth factor receptor 1) (e.g. EGFRI (epidermal growth factor receptor 1) according to accession number AF288738), MUC1, mucin-1 (e.g. mucin-1 according to accession number NM_002456), SEC61G (e.g. SEC61G according to accession number NM_014302), hTERT (e.g. hTERT accession number NM_198253), 5T4 (e.g. ST4 according to accession number NM_006670), NY-Eso-1 (e.g. NY-Eso1 according to accession number NM_001327), TRP-2 (e.g. TRP-2 according to accession number NM_001922), STEAP, PCA, PSA, PSMA, etc.

Particulary preferred are antigens derived from Influenza A virus (HA, NA, NP, M2, M1 antigens), influenza B virus (HA, NA antigens), respiratory syncytial virus (F, G, M, SH antigens), parainfluenza virus (glycoprotein antigens), varicella-zoster virus/herpes zoster, human papillomavirus (L1, L2, E6, E7), human immunodeficiency virus (gp120, gag, env antigens), SARS CoV (spike protein), Staphylococcus aureus (IsdA, IsdB, toxin antigens), Bordetella pertussis (toxin), polio virus (VP1-4), Plasmodium (NANP, CSP protein, ssp2, ama1, msp142 antigens), Streptococcus pneumoniae (Pht, PcsB, StkP antigens), Corynebacterium diphtheriae, Clostridium tetani, Measles, Mumps, Rubella, Rabies virus (G, N antigens), Staphylococcus aureus (toxin antigen), Clostridium difficile (toxin antigen), Mycobacterium tuberculosis (dormant antigens), Candida albicans.

Antigens as encoded by the at least one mRNA of the inventive vaccine may furthermore comprise fragments of such antigens as mentioned herein, particularly of protein or peptide antigens. Fragments of such antigens in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form.

Fragments of antigens as defined herein may also comprise epitopes of those antigens. Epitopes (also called "antigen determinants") are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

According to a further particularly preferred aspect, the tumour antigens as encoded by at least one mRNA of the inventive vaccine may form a cocktail of antigens, e.g. in an active (immunostimulatory) composition or a kit of parts (wherein preferably each antigen is contained in one part of the kit), preferably for eliciting an (adaptive) immune response for the treatment of a disease or disorder as defined herein. For this purpose, the inventive vaccine may comprise at least one mRNA, wherein each mRNA may encode at least one, preferably two, three, four or even more (preferably different) antigens as mentioned herein. Alternatively, the inventive vaccine may contain at least one, two, three, four or even more (preferably different) mRNAs, wherein each mRNA encodes at least one antigen as mentioned herein.

Such a cocktail of antigens, as encoded by the least one mRNA of the inventive vaccine may be used e.g. in the treatment of e.g. prostate cancer (PCa), preferably in the treatment of neoadjuvant and/or hormone-refractory prostate cancers, and diseases or disorders related thereto. For this purpose, the inventive vaccine may comprise at least one mRNA, wherein each mRNA may encode at least one, preferably two, three, four or even more (preferably different) antigens as mentioned herein. Alternatively, the inventive vaccine may contain at least one, two, three, four or even more (preferably different) mRNAs, wherein each mRNA encodes at least one antigen as mentioned herein. Preferably, the antigens are selected from PSA (Prostate-Specific Antigen) = KLK3 (Kallikrein-3), PSMA (Prostate-Specific Membrane Antigen), PSCA (Prostate Stem Cell Antigen), and/or STEAP (Six Transmembrane Epithelial Antigen of the Prostate).

Furthermore, such a cocktail of antigens, as encoded by the at least one mRNA of the inventive vaccine may be used in the treatment of e.g. non-small cell lung cancers (NSCLC), preferably selected from the three main sub-types squamous cell lung carcinoma, adenocarcinoma and large cell lung carcinoma, or of disorders related thereto. For this purpose, the inventive vaccine may comprise at least one mRNA, wherein each mRNA may encode at least one, preferably two, three, four, five, six, seven, eight, nine, ten eleven or twelve (preferably different) antigens as mentioned herein. Alternatively, the inventive vaccine may contain at least one, preferably two, three, four, five, six, seven, eight, nine, ten, eleven or twelve (preferably different) mRNAs, wherein each mRNA encodes at least one antigen as mentioned herein. Preferably, such antigens are selected from hTERT, WT1, MAGE-A2, 5T4, MAGE-A3, MUC1, Her-2/neu, NY-ESO-1, CEA, Survivin, MAGE-C1, and/or MAGE-C2.

In the above aspects, each of the above defined antigens may be encoded by one (monocistronic) mRNA. In other words, in this case the at least one mRNA of the inventive vaccine may comprise at least two (three, four, etc.) (monocistronic) mRNAs, wherein each of these at least two (three, four, etc.) (monocistronic) mRNAs may encode, e.g. just one (preferably different) antigen, preferably selected from one of the above mentioned antigen combinations.

According to a particularly preferred aspect, the at least one mRNA of the inventive vaccine may comprise (at least) one bi- or even multicistronic mRNA, preferably mRNA, i.e. (at least) one mRNA which carries, e.g. two or even more of the coding sequences of at least two (preferably different) antigens, preferably selected from one of the above mentioned antigen combinations. Such coding sequences, e.g. of the at least two (preferably different) antigens, of the (at least) one bi- or even multicistronic mRNA may be separated by at least one IRES (internal ribosomal entry site) sequence, as defined below. Thus, the term "encoding at least two (preferably different) antigens" may mean, without being limited thereto, that the (at least) one (bi- or even multicistronic) mRNA, may encode e.g. at least two, three, four, five, six, seven, eight, nine, ten, eleven or twelve or more (preferably different) antigens of the above mentioned group(s) of antigens, or their fragments or variants, etc. In this context, a so-called IRES (internal ribosomal entry site) sequence as defined herein can function as a sole ribosome binding site, but it can also serve to provide a bi- or even multicistronic RNA as defined herein which codes for several proteins, which are to be translated by the ribosomes independently of one another. Examples of IRES sequences which can be used according to the invention are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukemia virus (MLV), simian immunodeficiency viruses (SIV) or cricket paralysis viruses (CrPV).

According to a further particularly preferred aspect, the at least one mRNA of the inventive vaccine may comprise a mixture of at least one monocistronic mRNA as defined herein, and at least one bi- or even multicistronic RNA, preferably mRNA, as defined herein. The at least one monocistronic RNA and/or the at least one bi- or even multicistronic RNA preferably encode different antigens, or their fragments or variants, the antigens preferably being selected from one of the above mentioned antigens, more preferably in one of the above mentioned combinations. However, the at least one monocistronic RNA and the at least one bi- or even multicistronic RNA may preferably also encode (in part) identical antigens selected from one of the above mentioned antigens, preferably in one of the above mentioned combinations, provided that the at least one mRNA of the inventive vaccine as a whole provides at least two (preferably different) antigens, as defined herein. Such an aspect may be advantageous e.g. for a staggered, e.g. time dependent, administration of one or several of the at least one mRNA of the inventive vaccine to a patient in need thereof. The components of such a vaccine may be contained in (different parts of) a kit of parts composition or may be e.g. administered separately as components of the same inventive vaccine as defined according to the present invention.

In a further preferred embodiment the at least one mRNA of the inventive vaccine (or any further nucleic acid as defined herein) may also occur in the form of a modified nucleic acid.

According to a first aspect, the at least one mRNA of the inventive vaccine (or any further nucleic acid as defined herein) may be provided as a "stabilized nucleic acid" that is essentially resistant to *in vivo* degradation (e.g. by an exo- or endo-nuclease).

In this context, the at least one mRNA of the inventive vaccine (or any further nucleic acid as defined herein) may contain backbone modifications, sugar modifications or base modifications. A backbone modification in connection with the present invention is a modification in which phosphates of the backbone of the nucleotides contained in the at least one mRNA of the inventive vaccine (or any further nucleic acid as defined herein) are chemically modified. A sugar modification in connection with the present invention is a chemical modification of the sugar of the nucleotides of the at least one mRNA of the inventive vaccine (or any further nucleic acid as defined herein). Furthermore, a base modification in connection with the present invention is a chemical modification of the base moiety of the nucleotides of the at least one mRNA of the inventive vaccine (or any further nucleic acid as defined herein).

According to a further aspect, the at least one mRNA of the inventive vaccine (or any further nucleic acid as defined herein) can contain a lipid modification. Such a lipid-modified nucleic acid typically comprises a nucleic acid as defined herein, e.g. an mRNA or any further nucleic acid. Such a lipid-modified mRNA of the inventive vaccine (or any further lipid-modified nucleic acid as defined herein) typically further comprises at least one linker covalently linked with that nucleic acid molecule, and at least one lipid covalently linked with the respective linker. Alternatively, the lipid-modified mRNA of the inventive vaccine (or any further lipid-modified nucleic acid as defined herein) comprises at least one nucleic acid molecule as defined herein, e.g. an mRNA or any further nucleic acid, and at least one (bifunctional) lipid covalently linked (without a linker) with that nucleic acid molecule. According to a third alternative, the lipid-modified mRNA of the inventive vaccine (or any further lipid-modified nucleic acid as defined herein) comprises a nucleic acid molecule as defined herein, e.g. an mRNA or any further nucleic acid, at least one linker covalently linked with that nucleic acid molecule, and at least one lipid covalently linked with the respective linker, and also at least one (bifunctional) lipid covalently linked (without a linker) with that nucleic acid molecule.

The at least one mRNA of the inventive vaccine (or any further nucleic acid as defined herein) may likewise be stabilized in order to prevent degradation of the mRNA (or any further nucleic acid molecule) by various approaches. It is known in the art that instability and (fast) degradation of RNA in general may represent a serious problem in the application of RNA based compositions. This instability of RNA is typically due to RNA-degrading enzymes, "RNAases" (ribonucleases), wherein contamination with such ribonucleases may sometimes completely degrade RNA in solution. Accordingly, the natural degradation of RNA in the cytoplasm of cells is very finely regulated and RNase contaminations may be generally removed by special treatment prior to use of said compositions, in particular with diethyl pyrocarbonate (DEPC). A number of mechanisms of natural degradation are known in this connection in the prior art, which may be utilized as well. E.g., the terminal structure is typically of critical importance particularly for an mRNA. As an example, at the 5' end of naturally occurring mRNAs there is usually a so-called "cap structure" (a modified guanosine nucleotide), and at the 3' end is typically a sequence of up to 200 adenosine nucleotides (the so-called poly-A tail).

According to another aspect, the at least one mRNA of the inventive vaccine may be modified and thus stabilized by modifying the G/C content of the mRNA, preferably of the coding region thereof.

In a particularly preferred aspect of the present invention, the G/C content of the coding region of the at least one mRNA of the inventive vaccine is modified, particularly increased, compared to the G/C content of the coding region of its particular wild type coding sequence, i.e. the unmodified mRNA. The encoded amino acid sequence of the mRNA is preferably not modified compared to the coded amino acid sequence of the particular wild type mRNA.

The modification of the G/C-content of the at least one mRNA of the inventive vaccine is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. According to the invention, the codons of the coding sequence or mRNA are therefore varied compared to its wild type coding sequence or mRNA, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage).

Preferably, the G/C content of the coding region of the at least one mRNA of the inventive vaccine is increased by at least 7%, more preferably by at least 15%, particularly preferably by at least 20%, compared to the G/C content of the coded region of the wild type mRNA. According to a specific aspect at least 5%, 10%, 20%, 30%, 40%, 50%, 60%, more preferably at least 70 %, even more preferably at least 80% and most preferably at least 90%, 95% or even 100% of the substitutable codons in the region coding for a protein or peptide as defined herein or its fragment or variant thereof or the whole sequence of the wild type mRNA sequence or coding sequence are substituted, thereby increasing the G/C content of said sequence.

In this context, it is particularly preferable to increase the G/C content of the at least one mRNA of the inventive vaccine to the maximum (i.e. 100% of the substitutable codons), in particular in the region coding for a protein, compared to the wild type sequence.

According to the invention, a further preferred modification of the at least one mRNA of the inventive vaccine, especially if the nucleic acid is in the form of an mRNA or codes for an mRNA, is based on the finding that the translation efficiency is also determined by a different frequency in the occurrence of tRNAs in cells. Thus, if so-called "rare codons" are present in the at least one mRNA of the inventive vaccine to an increased extent, the corresponding modified mRNA is translated to a significantly poorer degree than in the case where codons coding for relatively "frequent" tRNAs are present.

Preferably, the coding region of the at least one mRNA of the inventive vaccine is modified compared to the corresponding region of the wild type mRNA or coding sequence such that at least one codon of the wild type sequence which codes for a tRNA which is relatively rare in the cell is exchanged for a codon which codes for a tRNA which is relatively frequent in the cell and carries the same amino acid as the relatively rare tRNA. By this modification, the sequences of the at least one mRNA of the inventive vaccine, especially if the nucleic acid is in the form of an mRNA or codes for an mRNA, is modified such that codons for which frequently occurring tRNAs are available are inserted. In other words, according to the invention, by this modification all codons of the wild type sequence which code for a tRNA which is relatively rare in the cell can in each case be exchanged for a codon which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA which occurs the most frequently in the (human) cell, are particularly preferred.

According to the invention, it is particularly preferable to link the sequential G/C content which is increased, in particular maximized, in the modified at least one mRNA of the inventive vaccine with the "frequent" codons without modifying the amino acid sequence of the protein encoded by the coding region of the mRNA. This preferred aspect allows provision of a particularly efficiently translated and stabilized (modified) at least one mRNA of the inventive vaccine.

According to a further preferred aspect of the invention, the at least one mRNA of the inventive vaccine as defined herein or any further nucleic acid molecule as defined herein preferably has at least one 5' and/or 3' stabilizing sequence. These stabilizing sequences in the 5' and/or 3' untranslated regions have the effect of increasing the half-life of the nucleic acid in the cytosol. These stabilizing sequences can have 100% sequence identity to naturally occurring sequences which occur in viruses, bacteria and eukaryotes, but can also be partly or completely synthetic. The untranslated sequences (UTR) of the (alpha-)globin gene, e.g. from *Homo sapiens* or *Xenopus laevis* may be mentioned as an example of stabilizing sequences which can be used in the present invention for a stabilized nucleic acid. Another example of a stabilizing sequence has the general formula (C/U)CCANₓCCC(U/A)PyₓUC(C/U)CC (SEQ ID NO: 383), which is contained in the 3'UTR of the very stable RNA which codes for (alpha-)globin, type(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art.

Nevertheless, substitutions, additions or eliminations of bases are preferably carried out with the at least one mRNA of the inventive vaccine or any further nucleic acid molecule as defined herein, especially if the nucleic acid is in the form of an mRNA, using a DNA matrix for preparation of the nucleic acid molecule by techniques of the well known site directed mutagenesis or with an oligonucleotide ligation strategy (see e.g. Maniatis et al., Molecular Cloning: A Laboratory Manual, Cold Spring Harbor Laboratory Press, 3rd ed., Cold Spring Harbor, NY, 2001). In such a process, for preparation of the at least one mRNA of the inventive vaccine as defined herein a corresponding DNA molecule may be transcribed *in vitro.* This DNA matrix preferably comprises a suitable promoter, e.g. a T7 or SP6 promoter, for *in vitro* transcription, which is followed by the desired nucleotide sequence for the at least one mRNA to be prepared and a termination signal for *in* vitro transcription. The DNA molecule, which forms the matrix of the at least one mRNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM^{®} series, e.g. pGEM^{®}-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

Nucleic acid molecules used according to the invention and as defined herein, e.g. the at least one mRNA of the inventive vaccine or any further nucleic acid molecule as defined herein, may be modified as outlined above for the at least one mRNA of the inventive vaccine.

Additionally, nucleic acid molecules used according to the invention and as defined herein, e.g. the at least one mRNA of the inventive vaccine or any further nucleic acid molecule as defined herein, may be prepared using any method known in the art, including synthetic methods such as e.g. solid phase synthesis, as well as *in vitro* methods, such as *in vitro tran*scription reactions.

According to one preferred aspect of the present invention the at least one mRNA of the inventive vaccine may be administered naked without being associated with any further vehicle, transfection or complexation agent for increasing the transfection efficiency of the at least one mRNA.

In a further preferred aspect of the present invention the at least one mRNA of the inventive vaccine is associated with a vehicle, transfection or complexation agent for increasing the transfection efficiency of the at least one mRNA. Particularly preferred agents in this context suitable for increasing the transfection efficiency are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia),* pAntp, plsl, FGF, Lactoferrin, Transportan, Buforin-2, Bac715-24, SynB, SynB(1), pVEC, hCT-derived peptides, SAP, or histones. Additionally, preferred cationic or polycationic proteins or peptides may be selected from the following proteins or peptides having the following total formula: (Arg)ₗ;(Lys)ₘ;(His)ₙ;(Orn)ₒ;(Xaa)ₓ, wherein I + m + n +o + x = 8-15, and I, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred cationic peptides in this context are e.g. Arg₇, Arg₈, Arg₉, H₃R₉, R₉H₃, H₃R₉H₃, YSSR₉SSY, (RKH)₄, Y(RKH)₂R, etc. Further preferred cationic or polycationic compounds, which can be used as transfection agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminοacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

The at least one mRNA of the inventive vaccine encoding at least one antigen may also be complexed with a polymeric carrier formed by disulfide-crosslinked cationic components. The term "cationic component" typically refers to a charged molecule, which is positively charged (cation) at a pH value of about 1 to 9, preferably of a pH value of or below 9, of or below 8, of or below 7, most preferably at physiological pH values, e.g. about 7.3 to 7.4. Accordingly, a cationic peptide, protein or polymer according to the present invention is positively charged under physiological conditions, particularly under physiological salt conditions of the cell *in vivo.* The definition "cationic" may also refer to "polycationic" components.

In this context the cationic components, which form basis for the polymeric carrier of the inventive vaccine by disulfide-crosslinkage, are typically selected from any suitable cationic or polycationic peptide, protein or polymer suitable for this purpose, particular any cationic or polycationic peptide, protein or polymer capable to complex a nucleic acid as defined according to the present invention, and thereby preferably condensing the nucleic acid. The cationic or polycationic peptide, protein or polymer, is preferably a linear molecule, however, branched cationic or polycationic peptides, proteins or polymers may also be used.

Each cationic or polycationic protein, peptide or polymer of the polymeric carrier, which may be used to complex the at least one mRNA of the inventive vaccine contains at least one -SH moiety, most preferably at least one cysteine residue or any further chemical group exhibiting an -SH moiety, capable to form a disulfide linkage upon condensation with at least one further cationic or polycationic protein, peptide or polymer as cationic component of the polymeric carrier as mentioned herein.

Each cationic or polycationic protein, peptide or polymer or any further component of the polymeric carrier, which may be used to complex the at least one mRNA of the inventive vaccine is preferably linked to its neighbouring component(s) (cationic proteins, peptides, polymers or other components) via disulfide-crosslinking. Preferably, the disulfide-crosslinking is a (reversible) disulfide bond (-S-S-) between at least one cationic or polycationic protein, peptide or polymer and at least one further cationic or polycationic protein, peptide or polymer or other component of the polymeric carrier. The disulfide-crosslinking is typically formed by condensation of -SH-moieties of the components of the polymeric carrier particularly of the cationic components. Such an -SH-moiety may be part of the structure of the cationic or polycationic protein, peptide or polymer or any further component of the polymeric carrier prior to disulfide-crosslinking or may be added prior to disulfide-crosslinking by a modification as defined below. In this context, the sulphurs adjacent to one component of the polymeric carrier, necessary for providing a disulfide bond, may be provided by the component itself, e.g. by a -SH moiety as defined herein or may be provided by modifying the component accordingly to exhibit a -SH moiety. These -SH-moieties are typically provided by each of the components, e.g. via a cysteine or any further (modified) amino acid of the component, which carries a -SH moiety. In the case that the cationic component or any further component of the polymeric carrier is a peptide or protein it is preferred that the -SH moiety is provided by at least one cysteine residue. Alternatively, the component of the polymeric carrier may be modified accordingly with a -SH moiety, preferably via a chemical reaction with a compound carrying a -SH moiety, such that each of the components of the polymeric carrier carries at least one such -SH moiety. Such a compound carrying a -SH moiety may be e.g. an (additional) cysteine or any further (modified) amino acid or compound of the component of the polymeric carrier, which carries a -SH moiety. Such a compound may also be any non-amino compound or moiety, which contains or allows to introduce a -SH moiety into the component as defined herein. Such non-amino compounds may be attached to the component of the polymeric carrier according to the present invention via chemical reactions or binding of compounds, e.g. by binding of a 3-thio propionic acid or 2-iminothiolane (Traut's reagent), by amide formation (e.g. carboxylic acids, sulphonic acids, amines, etc.), by Michael addition (e.g maleinimide moieties, α,β unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. In some cases the -SH moiety may be masked by protecting groups during chemical attachment to the component. Such protecting groups are known in the art and may be removed after chemical coupling. In each case, the -SH moiety, e.g. of a cysteine or of any further (modified) amino acid or compound, may be present at the terminal ends or internally at any position of the component of the polymeric carrier. As defined herein, each of the components of the polymeric carrier typically exhibits at least one -SH-moiety, but may also contain two, three, four, five, or even more -SH-moieties. Additionally to binding of cationic components a -SH moiety may be used to attach further components of the polymeric carrier of the inventive vaccine as defined herein, particularly an amino acid component, e.g. antigen epitopes, antigens, antibodies, cell penetrating peptides (e.g. TAT), ligands, etc.

As defined above, the polymeric carrier, which may be used to complex the at least one mRNA of the inventive vaccine may be formed by disulfide-crosslinked cationic (or polycationic) components.

According to one first alternative, at least one cationic (or polycationic) component of the polymeric carrier, which may be used to complex the at least one mRNA of the inventive vaccine may be selected from cationic or polycationic peptides or proteins. Such cationic or polycationic peptides or proteins preferably exhibit a length of about 3 to 100 amino acids, preferably a length of about 3 to 50 amino acids, more preferably a length of about 3 to 25 amino acids, e.g. a length of about 3 to 10, 5 to 15, 10 to 20 or 15 to 25 amino acids. Alternatively or additionally, such cationic or polycationic peptides or proteins may exhibit a molecular weight of about 0.01 kDa to about 100 kDa, including a molecular weight of about 0.5 kDa to about 100 kDa, preferably of about 10 kDa to about 50 kDa, even more preferably of about 10 kDa to about 30 kDa.

In the specific case that the cationic component of the polymeric carrier, which may be used to complex the at least one mRNA of the inventive vaccine comprises a cationic or polycationic peptide or protein, the cationic properties of the cationic or polycationic peptide or protein or of the entire polymeric carrier, if the polymeric carrier is entirely composed of cationic or polycationic peptides or proteins, may be determined upon its content of cationic amino acids. Preferably, the content of cationic amino acids in the cationic or polycationic peptide or protein and/or the polymeric carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 15% to 75%, even more preferably in the range of about 20% to 50%, e.g. 20, 30, 40 or 50%, or in a range formed by any two of the afore mentioned values, provided, that the content of all amino acids, e.g. cationic, lipophilic, hydrophilic, aromatic and further amino acids, in the cationic or polycationic peptide or protein, or in the entire polymeric carrier, if the polymeric carrier is entirely composed of cationic or polycationic peptides or proteins, is 100%. Preferably, such cationic or polycationic peptides or proteins of the polymeric carrier, which comprise or are additionally modified to comprise at least one -SH moiety, are selected from, without being restricted thereto, cationic peptides or proteins such as protamine, nucleoline, spermine or spermidine, oligo- or poly-L-lysine (PLL), basic polypeptides, oligo or poly-arginine, cell penetrating peptides (CPPs), chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*), pAntp, plsl, etc., antimicrobial-derived CPPs e.g. Buforin-2, Bac715-24, SynB, SynB(1), pVEC, hCT-derived peptides, SAP, MAP, PpTG20, Loligomere, FGF, Lactoferrin, histones, VP22 derived or analog peptides, Pestivirus Erns, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

Alternatively or additionally, such cationic or polycationic peptides or proteins of the polymeric carrier, which comprise or are additionally modified to comprise at least one -SH moiety, are selected from, without being restricted thereto, following cationic peptides having the following sum formula (I):

{(Arg)ₗ;(Lys)ₘ;(His)ₙ;(Orn)ₒ;(Xaa)ₓ};

wherein I + m + n +o + x = 3-100, and I, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred. Even more preferred peptides of this formula are oligoarginines such as e.g. Arg₇, Arg₈, Arg₉, Arg₁₂, His₃Arg₉, Arg₉His₃, His₃Arg₉His₃, His₆Arg₉His₆, His₃Arg₄His₃, His₆Arg₄His₆, TyrSer₂Arg₉Ser₂Tyr, (ArgLysHis)₄, Tyr(ArgLysHis)₂Arg, etc.

According to a particular preferred embodiment, such cationic or polycationic peptides or proteins of the polymeric carrier having the empirical sum formula (I) as shown above, may, without being restricted thereto, comprise at least one of the following subgroup of formulae:
Arg₇, Arg₈, Arg₉, Arg₁₀, Arg₁₁, Arg₁₂, Arg₁₃, Arg₁₄, Arg₁₅₋₃₀;
Lys₇, Lys₈, Lys₉, Lys₁₀, Lys₁₁, Lys₁₂, Lys₁₃, Lys₁₄, Lys₁₅₋₃₀;
His₇, His₈, His₉, His₁₀, His₁₁, His₁₂, His₁₃, His₁₄, His₁₅₋₃₀;
Orn₇, Orn₈, Orn₉, Orn₁₀, Orn₁₁, Orn₁₂, Orn₁₃, Orn₁₄, Orn₁₅₋₃₀;

According to a further particularly preferred embodiment, cationic or polycationic peptides or proteins of the polymeric carrier, having the empirical sum formula (I) as shown above and which comprise or are additionally modified to comprise at least one -SH moiety, may be preferably selected from, without being restricted thereto, at least one of the following subgroup of formulae. The following formulae (as with empirical formula (I)) do not specify any amino acid order, but are intended to reflect empirical formulae by exclusively specifying the (number of) amino acids as components of the respective peptide. Accordingly, as an example, empirical formula Arg₍₇₋₂₉₎Lys₁ is intended to mean that peptides falling under this formula contain 7 to 19 Arg residues and 1 Lys residue of whatsoever order. If the peptides contain 7 Arg residues and 1 Lys residue, all variants having 7 Arg residues and 1 Lys residue are encompassed. The Lys residue may therefore be positioned anywhere in the e.g. 8 amino acid long sequence composed of 7 Arg and 1 Lys residues. The subgroup preferably comprises:
Arg(₁₄₋₂₉₎Lys, Arg₍₄₋₂₉₎His₁, Arg₍₄₋₂₉Orn₁, Lys₍₄₋₂₉)His₁, Lys₍₄₋₂₉₎Orn₁, His₍₄₋₂₉₎Orn₁,
Arg₍₃₋₂₈₎Lys₂, Arg₍₃₋₂₈₎His₂, Arg₍₃₋₂₈₎Orn₂, Lys₍₃₋₂₈₎His₂, Lys₍₃₋₂₈₎Orn₂, His₍₃₋₂₈₎Orn₂,
Arg₍₂₋₂₇₎Lys₃, Arg₍₂₋₂₇₎His₃, Arg₍₂₋₂₇₎Orn₃, Lys₍₂₋₂₇₎His₃, Lys₍₂₋₂₇₎Orn₃, His₍₂₋₂₇₎Orn₃,
Arg₍₁₋₂₆₎Lys₄, Arg₍₁₋₂₆₎His₄, Arg₍₁₋₂₆₎Orn₄, Lys₍₁₋₂₆₎His₄, Lys₍₁₋₂₆₎Orn₄, His₍₁₋₂₆₎Orn₄,

Arg(3-28)Lys1His1, Arg(3-28)Lys1Orn1, Arg(3-28)His1Orn1, Arg1Lys(3-28)His1, Arg1Lys(3-28)Orn1, Lys(3-
28)His1Orn1, Arg1Lys1His(3-28), Arg1His(3-28)Orn1, Lys1His(3-28)Orn1;

Arg(2-27)Lys2His1, Arg(2-27)Lys1His2, Arg(2-27)Lys2Orn1, Arg(2-27)Lys1Orn2, Arg(2-27)His2Orn1, Arg(2-
27)His1Orn2, Arg2Lys(2-27)His1, Arg1Lys(2-27)His2, Arg2Lys(2-27)Orn1, Arg1Lys(2-27)Orn2, Lys(2-
27)His2Orn1, Lys(2-27)His1Orn2, Arg2Lys1His(2-27), Arg1Lys2His(2-27), Arg2His(2-27)Orn1, Arg1His(2-
27)Orn2, Lys2His(2-27)Orn1, Lys1His(2-27)Orn2;

Arg(1-26)Lys3His1, Arg(1-26)Lys2His2, Arg(1-26)Lys1His3, Arg(1-26)Lys3Orn1, Arg(1-26)Lys2Orn2, Arg(1-
26)Lys1Orn3, Arg(1-26)His3Orn1, Arg(1-26)His2Orn2, Arg(1-26)His1Orn3, Arg3Lys(1-26)His1, Arg2Lys(1-
26)His2, Arg1Lys(1-26)His3, Arg3Lys(1-26)Orn1, Arg2Lys(1-26)Orn2, Arg1Lys(1-26)Orn3, Lys(1-26)His3Orn1,
Lys(1-26)His2Orn2, Lys(1-26)His1Orn3, Arg3Lys1His(1-26), Arg2Lys2His(1-26), Arg1Lys3His(1-26),
Arg3His(1-26)Orn1, Arg2His(1-26)Orn2, Arg1His(1-26)Orn3, Lys3His(1-26)Orn1, Lys2His(1-26)Orn2,
Lys1His(1-26)Orn3;

Arg₍₂₋₂₇₎Lys₁, His₁Orn₁, Arg₁Lys₍₂₋₂₇₎His₁Orn₁, Arg₁Lys₁His₍₂₋₂₇₎Orn₁, Arg₁Lys₁His₁Orn₍₂₋₂₇₎;

Arg(1-26)Lys2His1Orn1,    Arg(1-26)Lys1His2Orn1,    Arg(1-26)Lys1His1Orn2,    Arg2Lys(1-26)His1Orn1,
Arg1Lys(1-26)His2Orn1,    Arg1Lys(1-26)His1Orn2,    Arg2Lys1His(1-26)Orn1,    Arg1Lys2His(1-26)Orn1,
Arg1Lys1His(1-26)Orn2, Arg2Lys1His1Orn(1-26), Arg1Lys2His1Orn(1-26), Arg1Lys1His2Orn(1-26);

According to a further particular preferred embodiment, cationic or polycationic peptides or proteins of the polymeric carrier, having the empirical sum formula (I) as shown above and which comprise or are additionally modified to comprise at least one -SH moiety, may be, without being restricted thereto, selected from the subgroup consisting of generic formulas Arg₇ (also termed as R₇), Arg₉ (also termed R₉), Arg₁₂ (also termed as R₁₂).

According to a one further particular preferred embodiment, the cationic or polycationic peptide or protein of the polymeric carrier, when defined according to formula {(Arg)₁;(Lys)ₘ;(His)ₙ;(Orn)ₒ;(Xaa)ₓ} (formula (I)) as shown above and which comprises or is additionally modified to comprise at least one -SH moiety, may be, without being restricted thereto, selected from subformula (Ia):

{(Arg)ₗ;(Lys)ₘ;(His)ₙ;(Orn)ₒ;(Xaa')ₓ(Cys)_{y}} formula (Ia)

wherein (Arg)ₗ;(Lys)ₘ;(His)ₙ;(Orn)ₒ; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide.

This embodiment may apply to situations, wherein the cationic or polycationic peptide or protein of the polymeric carrier, e.g. when defined according to empirical formula (Arg)ₗ;(Lys)ₘ;(His)ₙ;(Orn)ₒ;(Xaa)ₓ (formula (I)) as shown above, comprises or has been modified with at least one cysteine as -SH moiety in the above meaning such that the cationic or polycationic peptide as cationic component carries at least one cysteine, which is capable to form a disulfide bond with other components of the polymeric carrier.

According to another particular preferred embodiment, the cationic or polycationic peptide or protein of the polymeric carrier, when defined according to formula {(Arg)ₗ;(Lys)ₘ;(His)ₙ;(Orn)ₒ;(Xaa)ₓ} (formula (I)) as shown above, may be, without being restricted thereto, selected from subformula (Ib):

Cys¹ {(Arg)ₗ;(Lys)ₘ;(His)ₙ;(Orn)ₒ;(Xaa)ₓ} Cys² (formula (Ib))

wherein empirical formula {(Arg)ₗ;(Lys)ₘ;(His)ₙ;(Orn)ₒ;(Xaa)ₓ} (formula (I)) is as defined herein and forms a core of an amino acid sequence according to (semiempirical) formula (I) and wherein Cys¹ and Cys² are Cysteines proximal to, or terminal to (Arg)ₗ;(Lys)ₘ;(His)ₙ;(Orn)ₒ;(Xaa)ₓ. Exemplary examples may comprise any of the above sequences flanked by two Cys and following sequences:
CysArg₇Cys Cys-Arg-Arg-Arg-Arg-Arg-Arg-Arg-Cys (SEQ ID NO. 1)
CysArg₈Cys Cys-Arg-Arg-Arg-Arg-Arg-Arg-Arg-Arg-Cys (SEQ ID NO. 2)
CysArg₉Cys: Cys-Arg-Arg-Arg-Arg-Arg-Arg-Arg-Arg-Arg-Cys (SEQ ID NO. 3)
CysArg₁₀Cys Cys-Arg-Arg-Arg-Arg-Arg-Arg-Arg-Arg-Arg-Arg-Cys (SEQ ID NO. 4)
CysArg₁₁Cys Cys-Arg-Arg-Arg-Arg-Arg-Arg-Arg-Arg-Arg-Arg-Arg-Cys (SEQ ID NO. 5)
CysArg₁₂Cys: Cys-Arg-Arg-Arg-Arg-Arg-Arg-Arg-Arg-Arg-Arg-Arg-Arg-Cys (SEQ ID NO. 6)

CysArg13Cys: Cys-Arg-Arg-Arg-Arg-Arg-Arg-Arg-Arg-Arg-Arg-Arg-Arg-Cys (SEQ ID NO.
7)

CysArg14Cys: Cys-Arg-Arg-Arg-Arg-Arg-Arg-Arg-Arg-Arg-Arg-Arg-Arg-Arg-Arg-Cys (SEQ ID
NO. 8)

CysArg15Cys: Cys-Arg-Arg-Arg-Arg-Arg-Arg-Arg-Arg-Arg-Arg-Arg-Arg-Arg-Arg-Cys
(SEQ ID NO. 9)

CysArg16Cys: Cys-Arg-Arg-Arg-Arg-Arg-Arg-Arg-Arg-Arg-Arg-Arg-Arg-Arg-Arg-Arg-Cys
(SEQ ID NO. 10)

CysArg17Cys: Cys-Arg-Arg-Arg-Arg-Arg-Arg-Arg-Arg-Arg-Arg-Arg-Arg-Arg-Arg-Arg-Arg-
Cys (SEQ ID NO. 11)

CysArg18Cys: Cys-Arg-Arg-Arg-Arg-Arg-Arg-Arg-Arg-Arg-Arg-Arg-Arg-Arg-Arg-Arg-Arg-
Arg-Cys (SEQ ID NO. 12)

CysArg19Cys: Cys-Arg-Arg-Arg-Arg-Arg-Arg-Arg-Arg-Arg-Arg-Arg-Arg-Arg-Arg-Arg-Arg-Arg-
Arg-Cys (SEQ ID NO. 13)

CysArg20Cys: Cys-Arg-Arg-Arg-Arg-Arg-Arg-Arg-Arg-Arg-Arg-Arg-Arg-Arg-Arg-Arg-Arg-
Arg-Arg Cys (SEQ ID NO. 14)

This embodiment may apply to situations, wherein the cationic or polycationic peptide or protein of the polymeric carrier, which may be used to complex the at least one mRNA of the inventive vaccine, e.g. when defined according to empirical formula (Arg)ₗ;(Lys)ₘ;(His)ₙ;(Orn)ₒ;(Xaa)ₓ (formula (I)) as shown above, has been modified with at least two cysteines as -SH moieties in the above meaning such that the cationic or polycationic peptide of the inventive polymeric carrier carries at least two (terminal) cysteines, which are capable to form a disulfide bond with other components of the polymeric carrier.

According to a second alternative, at least one cationic (or polycationic) component of the polymeric carrier may be selected from e.g. any (non-peptidic) cationic or polycationic polymer suitable in this context, provided that this (non-peptidic) cationic or polycationic polymer exhibits or is modified to exhibit at least one -SH-moiety, which provide for a disulfide bond linking the cationic or polycationic polymer with another component of the polymeric carrier as defined herein. Thus, likewise as defined herein, the polymeric carrier may comprise the same or different cationic or polycationic polymers.

In the specific case that the cationic component of the polymeric carrier comprises a (non-peptidic) cationic or polycationic polymer the cationic properties of the (non-peptidic) cationic or polycationic polymer may be determined upon its content of cationic charges when compared to the overall charges of the components of the cationic polymer. Preferably, the content of cationic charges in the cationic polymer at a (physiological) pH as defined herein is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 10% to 90%, more preferably in the range of about 30% to 100%, even preferably in the range of about 50% to 100%, e.g. 50, 60, 70, 80%, 90% or 100%, or in a range formed by any two of the afore mentioned values, provided, that the content of all charges, e.g. positive and negative charges at a (physiological) pH as defined herein, in the entire cationic polymer is 100%.

Preferably, the (non-peptidic) cationic component of the polymeric carrier represents a cationic or polycationic polymer, typically exhibiting a molecular weight of about 0.1 or 0.5 kDa to about 100 kDa, preferably of about 1 kDa to about 75 kDa, more preferably of about 5 kDa to about 50 kDa, even more preferably of about 5 kDa to about 30 kDa, or a molecular weight of about 10 kDa to about 50 kDa, even more preferably of about 10 kDa to about 30 kDa. Additionally, the (non-peptidic) cationic or polycationic polymer typically exhibits at least one -SH-moiety, which is capable to form a disulfide linkage upon condensation with either other cationic components or other components of the polymeric carrier as defined herein.

In the above context, the (non-peptidic) cationic component of the polymeric carrier, which may be used to complex the at least one mRNA of the inventive vaccine may be selected from acrylates, modified acrylates, such as pDMAEMA (poly(dimethylaminoethyl methylacrylate)), chitosanes, aziridines or 2-ethyl-2-oxazoline (forming oligo ethylenimines or modifed oligoethylenimines), polymers obtained by reaction of bisacrylates with amines forming oligo beta aminoesters or poly amido amines, or other polymers like polyesters, polycarbonates, etc. Each molecule of these (non-peptidic) cationic or polycationic polymers typically exhibits at least one -SH-moiety, wherein these at least one -SH-moiety may be introduced into the (non-peptidic) cationic or polycationic polymer by chemical modifications, e.g. using imonothiolan, 3-thio propionic acid or introduction of -SH-moieties containing amino acids, such as cysteine or any further (modified) amino acid. Such -SH-moieties are preferably as already defined above.

In the context of the polymeric carrier, the cationic components, which form basis for the polymeric carrier, which may be used to complex the at least one mRNA of the inventive vaccine by disulfide-crosslinkage, may be the same or different from each other. It is also particularly preferred that the polymeric carrier of the present invention comprises mixtures of cationic peptides, proteins or polymers and optionally further components as defined herein, which are crosslinked by disulfide bonds as described herein.

In this context, the inventive polymeric carrier, which may be used to complex the at least one mRNA of the inventive vaccine allows to combine desired properties of different (short) cationic or polycationic peptides, proteins or polymers or other components. The polymeric carrier, e.g., allows to efficiently compact nucleic acids for the purpose of efficient transfection of nucleic acids, for adjuvant therapy, for the purposes of gene therapy, for gene knockdown or others strategies without loss of activity, particularly exhibiting an efficient transfection of a nucleic acid into different cell lines *in vitro* but particularly transfection *in vivo.* The polymeric carrier is furthermore not toxic to cells, provides for efficient release of its nucleic acid cargo, is stable during lyophilization and is applicable as immunostimulating agent or adjuvant. In this context the components of the inventive polymeric carrier can be varied in such a way that the cytokine pattern induced can be determined.

In particular, the polymeric carrier formed by disulfide-linked cationic components allows considerably to vary its peptide or polymeric content and thus to modulate its biophysical/biochemical properties, particularly the cationic properties of the polymeric carrier, quite easily and fast, e.g. by incorporating as cationic components the same or different cationic peptide(s) or polymer(s) and optionally adding other components into the polymeric carrier. Even though consisting of quite small non-toxic monomer units the polymeric carrier forms a long cationic binding sequence providing a strong condensation of the mRNA as its nucleic acid cargo and complex stability. Under the reducing conditions of the cytosole (e.g. cytosolic GSH), the complex is rapidly degraded into its (cationic) components, which are further degraded (e.g. oligopeptides). This supports deliberation of the nucleic acid cargo in the cytosol. Due to degradation into small oligopeptides or polymers in the cytosol, no toxicity is observed as known for high-molecular oligopeptides or polymers, e.g. from high-molecular polyarginine.

Accordingly, the polymeric carrier, which may be used to complex the at least one mRNA of the inventive vaccine may comprise different (short) cationic or polycationic peptides, proteins or polymers selected from cationic or polycationic peptides, proteins or (non-peptidic) polymers as defined above, optionally together with further components as defined herein.

Additionally, the polymeric carrier, which may be used to complex the at least one mRNA of the inventive vaccine as defined above, more preferably at least one of the different (short) cationic or polycationic peptides or (non-peptidic) polymers forming basis for the polymeric carrier via disulfide-crosslinking, may be, preferably prior to the disulfide-crosslinking, modified with at least one further component. Alternatively, the polymeric carrier as such may be modified with at least one further component. It may also optionally comprise at least one further component, which typically forms the polymeric carrier disulfide together with the other the (short) cationic or polycationic peptides as defined above via disulfide crosslinking.

To allow modification of a cationic or polycationic peptide or a (non-peptidic) polymer as defined above, each of the components of the polymeric carrier may (preferably already prior to disulfide-crosslinking) also contain at least one further functional moiety, which allows attaching such further components as defined herein. Such functional moieties may be selected from functionalities which allow the attachment of further components, e.g. functionalities as defined herein, e.g. by amide formation (e.g. carboxylic acids, sulphonic acids, amines, etc.), by Michael addition (e.g maleinimide moieties, α,β unsatured carbonyls, etc.), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

According to a particularly preferred embodiment, the further component, which may be contained in the polymeric carrier, and which may be used to complex the at least one mRNA of the inventive vaccine or which may be used to modify the different (short) cationic or polycationic peptides or (non-peptidic) polymers forming basis for the polymeric carrier or the biophysical/biochemical properties of the polymeric carrier as defined herein, is an amino acid component (AA). According to the present invention, the amino acid component (AA) comprises a number of amino acids preferably in a range of about 1 to 100, preferably in a range of about 1 to 50, more preferably selected from a number comprising 1, 2, 3, 4, 5, 6, 7, 8, 9, 10, 11, 12, 13, 14 or 15-20, or may be selected from a range formed by any two of the afore mentioned values. In this context the amino acids of amino acid component (AA) can be chosen independently from each other. For example if in the polymeric carrier two or more (AA) components are present they can be the same or can be different from each other.

The amino acid component (AA) may contain or may be flanked (e.g. terminally) by a -SH containing moiety, which allows introducing this component (AA) via a disulfide bond into the polymeric carrier as defined herein. In the specific case that the -SH containing moiety represents a cysteine, the amino acid component (AA) may also be read as -Cys-(AA)-Cys-wherein Cys represents Cysteine and provides for the necessary -SH-moiety for a disulfide bond. The -SH containing moiety may be also introduced into amino acid component (AA) using any of modifications or reactions as shown above for the cationic component or any of its components.

Furthermore, the amino acid component (AA) may be provided with two -SH-moieties (or even more), e.g. in a form represented by formula HS-(AA)-SH to allow binding to two functionalities via disulfide bonds, e.g. if the amino acid component (AA) is used as a linker between two further components (e.g. as a linker between two cationic polymers). In this case, one -SH moiety is preferably protected in a first step using a protecting group as known in the art, leading to an amino acid component (AA) of formula HS-(AA)-S-protecting group. Then, the amino acid component (AA) may be bound to a further component of the polymeric carrier, to form a first disulfide bond via the non-protected -SH moiety. The protected-SH-moiety is then typically deprotected and bound to a further free -SH-moiety of a further component of the polymeric carrier to form a second disulfide bond.

Alternatively, the amino acid component (AA) may be provided with other functionalities as already described above for the other components of the polymeric carrier, which allow binding of the amino acid component (AA) to any of components of the polymeric carrier.

Thus, according to the present invention, the amino acid component (AA) may be bound to further components of the polymeric carrier, which may be used to complex the at least one mRNA of the inventive vaccine with or without using a disulfide linkage. Binding without using a disulfide linkage may be accomplished by any of the reactions described above, preferably by binding the amino acid component (AA) to the other component of the polymeric carrier using an amid-chemistry as defined herein. If desired or necessary, the other terminus of the amino acid component (AA), e.g. the N- or C-terminus, may be used to couple another component, e.g. a ligand L. For this purpose, the other terminus of the amino acid component (AA) preferably comprises or is modified to comprise a further functionality, e.g. an alkyn-species (see above), which may be used to add the other component via e.g. click-chemistry. If the ligand is bound via an acid-labile bond, the bond is preferably cleaved off in the endosome and the polymeric carrier presents amino acid component (AA) at its surface.

The amino acid component (AA) may occur as a further component of the polymeric carrier, which may be used to complex the at least one mRNA of the inventive vaccine as defined above, e.g. as a linker between cationic components e.g. as a linker between one cationic peptide and a further cationic peptide, as a linker between one cationic polymer and a further cationic polymer, as a linker between one cationic peptide and a cationic polymer, all preferably as defined herein, or as an additional component of the polymeric carrier, e.g. by binding the amino acid component (AA) to the polymeric carrier or a component thereof, e.g. via side chains, SH-moieties or via further moieties as defined herein, wherein the amino acid component (AA) is preferably accordingly modified.

According to a further and particularly preferred alternative, the amino acid component (AA), may be used to modify the polymeric carrier, particularly the content of cationic components in the polymeric carrier as defined above.

In this context it is preferable, that the content of cationic components in the polymeric carrier is at least 10%, 20%, or 30%, preferably at least 40%, more preferably at least 50%, 60% or 70%, but also preferably at least 80%, 90%, or even 95%, 96%, 97%, 98%, 99% or 100%, most preferably at least 30%, 40%, 50%, 60%, 70%, 80%, 90%, 95%, 96%, 97%, 98%, 99% or 100%, or may be in the range of about 30% to 100%, more preferably in the range of about 50% to 100%, even preferably in the range of about 70% to 100%, e.g. 70, 80, 90 or 100%, or in a range formed by any two of the afore mentioned values, provided, that the content of all components in the polymeric carrier is 100%.

In the context of the present invention, the amino acid component (AA) may be selected from the following alternatives.

According to a first alternative, the amino acid component (AA) may be an aromatic amino acid component (AA). The incorporation of aromatic amino acids or sequences as amino aromatic acid component (AA) into the polymeric carrier of the present invention enables a different (second) binding of the polymeric carrier to the nucleic acid due to interactions of the aromatic amino acids with the bases of the nucleic acid cargo in contrast to the binding thereof by cationic charged sequences of the polymeric carrier molecule to the phosphate backbone. This interaction may occur e.g. by intercalations or by minor or major groove binding. This kind of interaction is not prone to decompaction by anionic complexing partners (e.g. Heparin, Hyaluronic acids) which are found mainly in the extracellular matrix *in vivo* and is also less susceptible to salt effects.

For this purpose, the amino acids in the aromatic amino acid component (AA) may be selected from either the same or different aromatic amino acids e.g. selected from Trp, Tyr, or Phe. Alternatively, the amino acids (or the entire aromatic amino acid component (AA)) may be selected from following peptide combinations Trp-Tyr, Tyr-Trp, Trp-Trp, Tyr-Tyr, Trp-Tyr-Trp, Tyr-Trp-Tyr, Trp-Trp-Trp, Tyr-Tyr-Tyr, Trp-Tyr-Trp-Tyr, Tyr-Trp-Tyr-Trp, Trp-Trp-Trp-Trp, Phe-Tyr, Tyr-Phe, Phe-Phe, Phe-Tyr-Phe, Tyr-Phe-Tyr, Phe-Phe-Phe, Phe-Tyr-Phe-Tyr, Tyr-Phe-Tyr-Phe, Phe-Phe-Phe-Phe, Phe-Trp, Trp-Phe, Phe-Phe, Phe-Trp-Phe, Trp-Phe-Trp, Phe-Trp-Phe-Trp, Trp-Phe-Trp-Phe, or Tyr-Tyr-Tyr-Tyr, etc. (SEQ ID NOs: 15 - 42). Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the aromatic amino acid component (AA) may contain or may be flanked by a -SH containing moiety, which allows introducing this component via a disulfide bond as a further part of the polymeric carrier as defined above, e.g. as a linker. Such a -SH containing moiety may be any moiety as defined herein suitable to couple one component as defined herein to a further component as defined herein. As an example, such a -SH containing moiety may be a cysteine. Then, e.g. the aromatic amino acid component (AA) may be selected from e.g. peptide combinations Cys-Tyr-Cys, Cys-Trp-Cys, Cys-Trp-Tyr-Cys, Cys-Tyr-Trp-Cys, Cys-Trp-Trp-Cys, Cys-Tyr-Tyr-Cys, Cys-Trp-Tyr-Trp-Cys, Cys-Tyr-Trp-Tyr-Cys, Cys-Trp-Trp-Trp-Cys, Cys-Tyr-Tyr-Tyr-Cys, Cys-Trp-Tyr-Trp-Tyr-Cys, Cys-Tyr-Trp-Tyr-Trp-Cys, Cys-Trp-Trp-Trp-Trp-Cys, Cys-Tyr-Tyr-Tyr-Tyr-Cys, Cys-Phe-Cys, Cys-Phe-Tyr-Cys, Cys-Tyr-Phe-Cys, Cys-Phe-Phe-Cys, Cys-Tyr-Tyr-Cys, Cys-Phe-Tyr-Phe-Cys, Cys-Tyr-Phe-Tyr-Cys, Cys-Phe-Phe-Phe-Cys, Cys-Tyr-Tyr-Tyr-Cys, Cys-Phe-Tyr-Phe-Tyr-Cys, Cys-Tyr-Phe-Tyr-Phe-Cys, or Cys-Phe-Phe-Phe-Phe-Cys, Cys-Phe-Trp-Cys, Cys-Trp-Phe-Cys, Cys-Phe-Phe-Cys, Cys-Phe-Trp-Phe-Cys, Cys-Trp-Phe-Trp-Cys, Cys-Phe-Trp-Phe-Trp-Cys, Cys-Trp-Phe-Trp-Phe-Cys, etc. Each Cys above may also be replaced by any modified peptide or chemical compound carrying a free -SH-moiety as defined herein. (SEQ ID NOs: 43-75). Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the aromatic amino acid component (AA) may contain or represent at least one proline, which may serve as a structure breaker of longer sequences of Trp, Tyr and Phe in the aromatic amino acid component (AA), preferably two, three or more prolines.

According to a second alternative, the amino acid component (AA) may be a hydrophilic (and preferably non charged polar) amino acid component (AA). The incorporation of hydrophilic (and preferably non charged polar) amino acids or sequences as amino hydrophilic (and preferably non charged polar) acid component (AA) into the polymeric carrier of the present invention enables a more flexible binding to the nucleic acid cargo. This leads to a more effective compaction of the nucleic acid cargo and hence to a better protection against nucleases and unwanted decompaction. It also allows provision of a (long) polymeric carrier which exhibits a reduced cationic charge over the entire carrier and in this context to better adjusted binding properties, if desired or necessary.

For this purpose, the amino acids in the hydrophilic (and preferably non charged polar) amino acid component (AA) may be selected from either the same or different hydrophilic (and preferably non charged polar) amino acids e.g. selected from Thr, Ser, Asn or Gln. Alternatively, the amino acids (or the entire hydrophilic (and preferably non charged polar) amino acid component (AA)) may be selected from following peptide combinations Ser-Thr, Thr-Ser, Ser-Ser, Thr-Thr, Ser-Thr-Ser, Thr-Ser-Thr, Ser-Ser-Ser, Thr-Thr-Thr, Ser-Thr-Ser-Thr, Thr-Ser-Thr-Ser, Ser-Ser-Ser-Ser, Thr-Thr-Thr-Thr, Gln-Asn, Asn-Gln, Gln-Gln, Asn-Asn, Gln-Asn-Gln, Asn-Gln-Asn, Gln-Gln-Gln, Asn-Asn-Asn, Gln-Asn-Gln-Asn, Asn-Gln-Asn-Gln, Gln-Gln-Gln-Gln, Asn-Asn-Asn-Asn, Ser-Asn, Asn-Ser, Ser-Ser, Asn-Asn, Ser-Asn-Ser, Asn-Ser-Asn, Ser-Ser-Ser, Asn-Asn-Asn, Ser-Asn-Ser-Asn, Asn-Ser-Asn-Ser, Ser-Ser-Ser-Ser, or Asn-Asn-Asn-Asn, etc. (SEQ ID NOs: 76 - 111). Such peptide combinations may be repeated e.g. 1, 2, 3, 4, 5, 6, 7, 8, 9, 10, 11, 12, 13, 14, 15 or even more times. These peptide combinations may also be combined with each other as suitable.

Additionally, the hydrophilic (and preferably non-charged polar) amino acid component (AA) may contain or may be flanked by a -SH containing moiety, which allows introducing this component via a disulfide bond as a further part of the polymeric carrier as defined above, e.g. as a linker. Such a -SH containing moiety may be any moiety as defined herein suitable to couple one component as defined herein to a further component as defined herein. As an example, such a -SH containing moiety may be a cysteine. Then, e.g. the hydrophilic (and preferably non-charged polar) amino acid component (AA) may be selected from e.g. peptide combinations Cys-Thr-Cys, Cys-Ser-Cys, Cys-Ser-Thr-Cys, Cys-Thr-Ser-Cys, Cys-Ser-Ser-Cys, Cys-Thr-Thr-Cys, Cys-Ser-Thr-Ser-Cys, Cys-Thf-Ser-Thr-Cys, Cys-Ser-Ser-Ser-Cys, Cys-Thr-Thr-Thr-Cys, Cys-Ser-Thr-Ser-Thr-Cys, Cys-Thr-Ser-Thr-Ser-Cys, Cys-Ser-Ser-Ser-Ser-Cys, Cys-Thr-Thr-Thr-Thr-Cys, Cys-Asn-Cys, Cys-Gln-Cys, Cys-Gln-Asn-Cys, Cys-Asn-Gln-Cys, Cys-Gln-Gln-Cys, Cys-Asn-Asn-Cys, Cys-Gln-Asn-Gln-Cys, Cys-Asn-Gln-Asn-Cys, Cys-Gln-Gln-Gln-Cys, Cys-Asn-Asn-Asn-Cys, Cys-Gln-Asn-Gln-Asn-Cys, Cys-Asn-Gln-Asn-Gln-Cys, Cys-Gln-Gln-Gln-Gln-Cys, Cys-Asn-Asn-Asn-Asn-Cys, Cys-Asn-Cys, Cys-Ser-Cys, Cys-Ser-Asn-Cys, Cys-Asn-Ser-Cys, Cys-Ser-Ser-Cys, Cys-Asn-Asn-Cys, Cys-Ser-Asn-Ser-Cys, Cys-Asn-Ser-Asn-Cys, Cys-Ser-Ser-Ser-Cys, Cys-Asn-Asn-Asn-Cys, Cys-Ser-Asn-Ser-Asn-Cys, Cys-Asn-Ser-Asn-Ser-Cys, Cys-Ser-Ser-Ser-Ser-Cys, or Cys-Asn-Asn-Asn-Asn-Cys, etc. Each Cys above may also be replaced by any modified peptide or chemical compound carrying a free -SH-moiety as defined herein. (SEQ ID NOs: 112 - 153) Such peptide combinations may be repeated e.g. 1, 2, 3, 4, 5, 6, 7, 8, 9, 10, 12, 13, 14, 15 or even more times. These peptide combinations may also be combined with each other as suitable.

Additionally, the hydrophilic (and preferably non-charged polar) amino acid component (AA) may contain at least one proline, which may serve as a structure breaker of longer sequences of Ser, Thr and Asn in the hydrophilic (and preferably non charged polar) amino acid component (AA), preferably two, three or more prolines.

According to a third alternative, the amino acid component (AA) may be a lipophilic amino acid component (AA). The incorporation of lipophilic amino acids or sequences as amino lipophilic acid component (AA) into the polymeric carrier of the present invention enables a stronger compaction of the nucleic acid cargo and/or the polymeric carrier and its nucleic acid cargo when forming a complex. This is particularly due to interactions of one or more polymer strands of the polymeric carrier, particularly of lipophilic sections of lipophilic amino acid component (AA) and the nucleic acid cargo. This interaction will preferably add an additional stability to the complex between the polymeric carrier and its nucleic acid cargo. This stabilization may somehow be compared to a sort of non covalent crosslinking between different polymerstrands. Especially in aqueous environment this interaction is typically strong and provides a significant effect.

For this purpose, the amino acids in the lipophilic amino acid component (AA) may be selected from either the same or different lipophilic amino acids e.g. selected from Leu, Val, Ile, Ala, Met. Alternatively, the amino acid AA (or the entire lipophilic amino acid component (AA)) may be selected from following peptide combinations Leu-Val, Val-Leu, Leu-Leu, Val-Val, Leu-Val-Leu, Val-Leu-Val, Leu-Leu-Leu, Val-Val-Val, Leu-Val-Leu-Val, Val-Leu-Val-Leu, Leu-Leu-Leu-Leu, Val-Val-Val-Val, Ile-Ala, Ala-Ile, Ile-Ile, Ala-Ala, Ile-Ala-Ile, Ala-Ile-Ala, Ile-Ile-Ile, Ala-Ala-Ala, Ile-Ala-Ile-Ala, Ala-Ile-Ala-Ile, Ile-Ile-Ile-Ile, Ala-Ala-Ala-Ala, Met-Ala, Ala-Met, Met-Met, Ala-Ala, Met-Ala-Met, Ala-Met-Ala, Met-Met-Met, Ala-Ala-Ala, Met-Ala-Met-Ala, Ala-Met-Ala-Met, or Met-Met-Met-Met etc. (SEQ ID NOs: 154 - 188) Such peptide combinations may be repeated e.g. 1, 2, 3, 4, 5, 6, 7, 8, 9, 10, 12, 13, 14, 15 or even more times. These peptide combinations may also be combined with each other as suitable.

Additionally, the lipophilic amino acid component (AA) may contain or may be flanked by a -SH containing moiety, which allows introducing this component via a disulfide bond as a further part of the polymeric carrier above, e.g. as a linker. Such a -SH containing moiety may be any moiety as defined herein suitable to couple one component as defined herein to a further component as defined herein. As an example, such a -SH containing moiety may be a cysteine. Then, e.g. the lipophilic amino acid component (AA) may be selected from e.g. peptide combinations Cys-Val-Cys, Cys-Leu-Cys, Cys-Leu-Val-Cys, Cys-Val-Leu-Cys, Cys-Leu-Leu-Cys, Cys-Val-Val-Cys, Cys-Leu-Val-Leu-Cys, Cys-Val-Leu-Val-Cys, Cys-Leu-Leu-Leu-Cys, Cys-Val-Val-Val-Cys, Cys-Leu-Val-Leu-Val-Cys, Cys-Val-Leu-Val-Leu-Cys, Cys-Leu-Leu-Leu-Leu-Cys, Cys-Val-Val-Val-Val-Cys, Cys-Ala-Cys, Cys-Ile-Cys, Cys-Ile-Ala-Cys, Cys-Ala-Ile-Cys, Cys-Ile-Ile-Cys, Cys-Ala-Ala-Cys, Cys-Ile-Ala-Ile-Cys, Cys-Ala-Ile-Ala-Cys, Cys-Ile-Ile-Ile-Cys, Cys-Ala-Ala-Ala-Cys, Cys-Ile-Ala-Ile-Ala-Cys, Cys-Ala-Ile-Ala-Ile-Cys, Cys-Ile-Ile-Ile-Ile-Cys, or Cys-Ala-Ala-Ala-Ala-Cys, Cys-Met-Cys, Cys-Met-Ala-Cys, Cys-Ala-Met-Cys, Cys-Met-Met-Cys, Cys-Ala-Ala-Cys, Cys-Met-Ala-Met-Cys, Cys-Ala-Met-Ala-Cys, Cys-Met-Met-Met-Cys, Cys-Ala-Ala-Ala-Cys, Cys-Met-Ala-Met-Ala-Cys, Cys-Ala-Met-Ala-Met-Cys, Cys-Met-Met-Met-Met-Cys, or Cys-Ala-Ala-Ala-Ala-Cys, etc. Each Cys above may also be replaced by any modified peptide or chemical compound carrying a free -SH-moiety as defined herein. (SEQ ID NOs: 189 - 229) Such peptide combinations may be repeated e.g. 1, 2, 3, 4, 5, 6, 7, 8, 9, 10, 12, 13, 14, 15 or even more times. These peptide combinations may also be combined with each other as suitable.

Additionally, the lipophilic amino acid component (AA) may contain at least one proline, which may serve as a structure breaker of longer sequences of Leu, Val, Ile, Ala and Met in the lipophilic amino acid component (AA), preferably two, three or more prolines.

Finally, according to a fourth alternative, the amino acid component (AA) may be a weak basic amino acid component (AA). The incorporation of weak basic amino acids or sequences as weak basic amino acid component (AA) into the polymeric carrier of the present invention may serve as a proton sponge and facilitates endosomal escape (also called endosomal release) (proton sponge effect). Incorporation of such a weak basic amino acid component (AA) preferably enhances transfection efficiency.

For this purpose, the amino acids in the weak basic amino acid component (AA) may be selected from either the same or different weak amino acids e.g. selected from histidine or aspartate (aspartic acid). Alternatively, the weak basic amino acids (or the entire weak basic amino acid component (AA)) may be selected from following peptide combinations Asp-His, His-Asp, Asp-Asp, His-His, Asp-His-Asp, His-Asp-His, Asp-Asp-Asp, His-His-His, Asp-His-Asp-His, His-Asp-His-Asp, Asp-Asp-Asp-Asp, or His-His-His-His, etc. (SEQ ID NOs: 230 - 241) Such peptide combinations may be repeated e.g. 1, 2, 3, 4, 5, 6, 7, 8, 9, 10, 12, 13, 14, 15 or even more times. These peptide combinations may also be combined with each other as suitable.

Additionally, the weak basic amino acid component (AA) may contain or may be flanked by a -SH containing moiety, which allows introducing this component via a disulfide bond as a further part of the polymeric carrier as defined above, e.g. as a linker. Such a -SH containing moiety may be any moiety as defined herein suitable to couple one component as defined herein to a further component as defined herein. As an example, such a -SH containing moiety may be a cysteine. Then, e.g. the weak basic amino acid component (AA) may be selected from e.g. peptide combinations Cys-His-Cys, Cys-Asp-Cys, Cys-Asp-His-Cys, Cys-His-Asp-Cys, Cys-Asp-Asp-Cys, Cys-His-His-Cys, Cys-Asp-His-Asp-Cys, Cys-His-Asp-His-Cys, Cys-Asp-Asp-Asp-Cys, Cys-His-His-His-Cys, Cys-Asp-His-Asp-His-Cys, Cys-His-Asp-His-Asp-Cys, Cys-Asp-Asp-Asp-Asp-Cys, or Cys-His-His-His-His-Cys, etc. Each Cys above may also be replaced by any modified peptide or chemical compound carrying a free -SH-moiety as defined herein. (SEQ ID NOs: 242 - 255) Such peptide combinations may be repeated e.g. 1, 2, 3, 4, 5, 6, 7, 8, 9, 10, 12, 13, 14, 15 or even more times. These peptide combinations may also be combined with each other as suitable.

Additionally, the weak basic amino acid component (AA) may contain at least one proline, which may serve as a structure breaker of longer sequences of histidine or aspartate (aspartic acid) in the weak basic amino acid component (AA), preferably two, three or more prolines.

According to a fifth alternative, the amino acid component (AA) may be a signal peptide or signal sequence, a localisation signal or sequence, a nuclear localisation signal or sequence (NLS), an antibody, a cell penetrating peptide, (e.g. TAT), etc. Preferably such an amino acid component (AA) is bound to the polymeric carrier or to another component of the polymeric carrier via a (reversible) disulfide bond. In this context the signal peptide or signal sequence, a localisation signal or sequence, a nuclear localisation signal or sequence (NLS), an antibody, a cell penetrating peptide, (e.g. TAT), etc.; additionally comprises at least one-SH-moiety. In this context a signal peptide, a localisation signal or sequence or a nuclear localisation signal or sequence (NLS), may be used to direct the inventive polymeric carrier cargo complex to specific target cells (e.g. hepatocytes or antigen-presenting cells) and preferably allows a translocalisation of the polymeric carrier to a specific target, e.g. into the cell, into the nucleus, into the endosomal compartment, sequences for the mitochondrial matrix, localisation sequences for the plasma membrane, localisation sequences for the Golgi apparatus, the nucleus, the cytoplasm and the cytoskeleton, etc. Such signal peptide, a localisation signal or sequence or a nuclear localisation signal may be used for the transport of any of the herein defined nucleic acids, preferably an RNA or a DNA, more preferably an shRNA or a pDNA, e.g. into the nucleus. Without being limited thereto, such a signal peptide, a localisation signal or sequence or a nuclear localisation signal may comprise, e.g., localisation sequences for the endoplasmic reticulum. Particular localisation signals or sequences or a nuclear localisation signals may include e.g. KDEL (SEQ ID NO: 256), DDEL (SEQ ID NO: 257), DEEL (SEQ ID NO: 258), QEDL (SEQ ID NO: 259), RDEL (SEQ ID NO: 260), and GQNLSTSN (SEQ ID NO: 261), nuclear localisation sequences, including PKKKRKV (SEQ ID NO: 262), PQKKIKS (SEQ ID NO: 263), QPKKP (SEQ ID NO: 264), RKKR (SEQ ID NO: 265), RKKRRQRRRAHQ (SEQ ID NO: 266), RQARRNRRRRWRERQR (SEQ ID NO: 267), MPLTRRRPAASQALAPPTP (SEQ ID NO: 268), GAALTILV (SEQ ID NO: 269), and GAALTLLG (SEQ ID NO: 270), localisation sequences for the endosomal compartment, including MDDQRDLISNNEQLP (SEQ ID NO: 271), localisation sequences for the mitochondrial matrix, including MLFNLRXXLNNAAFRHGHNFMVRNFRCGQPLX (SEQ ID NO: 272), localisation sequences for the plasma membrane: GCVCSSNP (SEQ ID NO: 273), GQTVTTPL (SEQ ID NO: 274), GQELSQHE (SEQ ID NO: 275), GNSPSYNP (SEQ ID NO: 276), GVSGSKGQ (SEQ ID NO: 277), GQTlTTPL (SEQ ID NO: 278), GQTLTTPL (SEQ ID NO: 279), GQIFSRSA (SEQ ID NO: 280), GQIHGLSP (SEQ ID NO: 281), GARASVLS (SEQ ID NO: 282), and GCTLSAEE (SEQ ID NO: 283), localisation sequences for the endoplasmic reticulum and the nucleus, including GAQVSSQK (SEQ ID NO: 284), and GAQLSRNT (SEQ ID NO: 285), localisation sequences for the Golgi apparatus, the nucleus, the cytoplasm and the cytoskeleton, including GNAAAAKK (SEQ ID NO: 286), localisation sequences for the cytoplasm and cytoskeleton, including GNEASYPL (SEQ ID NO: 287), localisation sequences for the plasma membrane and cytoskeleton, including GSSKSKPK (SEQ ID NO: 288), etc. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulins as defined herein, signal sequences of the invariant chain of immunoglobulins or antibodies as defined herein, signal sequences of Lamp1, Tapasin, Erp57, Calreticulin, Calnexin, and further membrane associated proteins or of proteins associated with the endoplasmic reticulum (ER) or the endosomal-lysosomal compartment. Particularly preferably, signal sequences of MHC class I molecule HLA-A*0201 may be used according to the present invention. Such an additional component may be bound e.g. to a cationic polymer or to any other component of the polymeric carrier as defined herein. Preferably this signal peptide, localisation signal or sequence or nuclear localisation signal or sequence (NLS), is bound to the polymeric carrier or to another component of the polymeric carrier via a (reversible) disulfide bond. For this purpose the (AA) component additionally comprises at least one -SH moiety as defined herein. The binding to any of components of the polymeric carrier may also be accomplished using an acid-labile bond, preferably via a side chain of any of components of the polymeric carrier, which allows to detach or release the additional component at lower pH-values, e.g. at physiological pH-values as defined herein.

Additionally, according to another alternative, the amino acid component (AA) may be a functional peptide or protein, which may modulate the functionality of the polymeric carrier accordingly. Such functional peptides or proteins as the amino acid component (AA) preferably comprise any peptides or proteins as defined herein, e.g. as defined below as therapeutically active proteins. According to one alternative, such further functional peptides or proteins may comprise so called cell penetrating peptides (CPPs) or cationic peptides for transportation. Particularly preferred are CPPs, which induce a pH-mediated conformational change in the endosome and lead to an improved release of the polymeric carrier (in complex with a nucleic acid) from the endosome by insertion into the lipid layer of the liposome. These cell penetrating peptides (CPPs) or cationic peptides for transportation, may include, without being limited thereto protamine, nucleoline, spermine or spermidine, oligo- or poly-L-lysine (PLL), basic polypeptides, oligo or poly-arginine, chimeric CPPs, such as Transportan, or MPG peptides, HIV-binding peptides, Tat, HIV-1 Tat (HIV), Tat-derived peptides, members of the penetratin family, e.g. Penetratin, Antennapedia-derived peptides (particularly from *Drosophila antennapedia*), pAntp, plsl, etc., antimicrobial-derived CPPs e.g. Buforin-2, Bac715-24, SynB, SynB(1), pVEC, hCT-derived peptides, SAP, MAP, PpTG20, Loligomere, FGF, Lactoferrin, histones, VP22 derived or analog peptides, Pestivirus Erns, HSV, VP22 (Herpes simplex), MAP, KALA or protein transduction domains (PTDs, PpT620, proline-rich peptides, arginine-rich peptides, lysine-rich peptides, Pep-1, L-oligomers, Calcitonin peptide(s), etc. Such an amino acid component (AA) may also be bound to any component of the polymeric carrier as defined herein. Preferably it is bound to the polymeric carrier or to another component of the polymeric carrier via a (reversible) disulfide bond. For the above purpose, the amino acid component (AA) preferably comprises at least one -SH moiety as defined herein. The binding to any of components of the polymeric carrier may also be accomplished using an SH-moiety or an acid-labile bond, preferably via a side chain of any of components of the polymeric carrier which allows to detach or release the additional component at lower pH-values, e.g. at physiological pH-values as defined herein.

According to a last alternative, the amino acid component (AA) may consist of any peptide or protein which can execute any favourable function in the cell. Particularly preferred are peptides or proteins selected from therapeutically active proteins or peptides, from antigens, e.g. tumour antigens, pathogenic antigens (animal antigens, viral antigens, protozoan antigens, bacterial antigens, allergic antigens), autoimmune antigens, or further antigens, from allergens, from antibodies, from immunostimulatory proteins or peptides, from antigen-specific T cell receptors, or from any other protein or peptide suitable for a specific (therapeutic) application as defined below for coding nucleic acids. Particularly preferred are peptide epitopes from antigens as defined herein.

The polymeric carrier, which may be used to complex the at least one mRNA of the inventive vaccine may comprise at least one of the above mentioned cationic or polycationic peptides, proteins or polymers or further components, e.g. (AA), wherein any of the above alternatives may be combined with each other, and may be formed by polymerizing same in a polymerization condensation reaction via their -SH-moieties.

According to another aspect, the polymeric carrier, which may be used to complex the at least one mRNA of the inventive vaccine or single components thereof, e.g. of the above mentioned cationic or polycationic peptides, proteins or polymers or further components, e.g. (AA), may be further modified with a ligand, preferably a carbohydrate, more preferably a sugar, even more preferably mannose. Preferably this ligand is bound to the polymeric carrier or to a component of the polymeric carrier via a (reversible) disulfide bond or via Michael addition. In the case that the ligand is bound by a disulfide bond the ligand additionally comprises at least one -SH-moiety. These ligands may be used to direct the inventive polymeric carrier cargo complex to specific target cells (e.g. hepatocytes or antigen-presenting cells). In this context mannose is particularly preferred as ligand in the case that dendritic cells are the target especially for vaccination or adjuvant purposes.

According to one specific aspect, the entire inventive polymeric carrier may be formed by a polymerization condensation (of at least one) of the above mentioned cationic or polycationic peptides, proteins or polymers or further components, e.g. (AA), via their -SH-moieties in a first step and complexing the nucleic acid to such a polymeric carrier in a second step. The polymeric carrier may thus contain a number of at least one or even more of the same or different of the above defined cationic or polycationic peptides, proteins or polymers or further components, e.g. (AA), the number preferably determined by the above range.

According to one alternative specific aspect, the inventive polymeric carrier, which may be used to complex the at least one mRNA of the inventive vaccine is formed by carrying out the polymerization condensation of at least one of the above mentioned cationic or polycationic peptides, proteins or polymers or further components, e.g. (AA), via their -SH-moieties simultaneously to complexing the at least one mRNA encoding the at least one antigen to the (in situ prepared) polymeric carrier. Likewise, the polymeric carrier may thus also here contain a number of at least one or even more of the same or different of the above defined cationic or polycationic peptides, proteins or polymers or further components, e.g. (AA), the number preferably determined by the above range.

According to a further alternative aspect, the inventive polymeric carrier may be selected from a polymeric carrier molecule according to generic formula (VI):

L-P¹-S-[S-P²-S]ₙ-S-P³-L

wherein,
- P¹ and P³: are different or identical to each other and represent a linear or branched hydrophilic polymer chain, each P¹ and P³ exhibiting at least one -SH-moiety, capable to form a disulfide linkage upon condensation with component P², or alternatively with (AA), (AA)ₓ, or [(AA)ₓ]_{z} if such components are used as a linker between P¹ and P² or P³ and P²) and/or with further components (e.g. (AA), (AA)ₓ, [(AA)ₓ]_{z} or L), the linear or branched hydrophilic polymer chain selected independent from each other from polyethylene glycol (PEG), poly-*N*-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
- P²: is a cationic or polycationic peptide or protein, e.g. as defined herein, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20; or
is a cationic or polycationic polymer, e.g. as defined herein, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa;
each P² exhibiting at least two -SH-moieties, capable to form a disulfide linkage upon condensation with further components P² or component(s) P¹ and/or P³ or alternatively with further components (e.g. (AA), (AA)ₓ, or [(AA)ₓ]_{z});
- -S-S-: is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P¹ and P², P² and P², or P² and P³, or optionally of further components as defined herein (e.g. L, (AA), (AA)ₓ, [(AA)ₓ]_{z}, etc); The -SH-moiety may be part of the structure of these components or added by a modification as defined below;

- L: is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc), small molecules (e.g. carbohydrates like mannose or galctose or synthetic ligands), small molecule agonists, inhibitors or antagonists of receptors (e.g. RGD peptidomimetic analogues), or any further protein as defined herein, etc.;
- n: is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

As defined above, ligands (L), may be optionally used in the inventive polymeric carrier molecule according to generic formula (VI), e.g. for direction of the inventive carrier polymer and its entire "cargo" (the adjuvant component and/or the antigen of the inventive composition or vaccine composition) into specific cells. They may be selected independent from the other from RGD, Transferrin, Folate, a signal peptide or signal sequence, a localization signal or sequence, a nuclear localization signal or sequence (NLS), an antibody, a cell penetrating peptide (CPP), (e.g. TAT, KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues) or any such molecule as further defined below, etc. Particularly preferred are cell penetrating peptides (CPPs), which induce a pH-mediated conformational change in the endosome and lead to an improved release of the inventive polymeric carrier (in complex with a nucleic acid) from the endosome by insertion into the lipid layer of the liposome. Such called CPPs or cationic peptides for transportation, may include, without being limited thereto protamine, nucleoline, spermine or spermidine, poly-L-lysine (PLL), basic polypeptides, poly-arginine, chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*), pAntp, plsl, etc., antimicrobial-derived CPPs e.g. Buforin-2, Bac715-24, SynB, SynB(1), pVEC, hCT-derived peptides, SAP, MAP, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, Pestivirus Erns, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc. Particularly preferred in this context is mannose as ligand to target antigen presenting cells which carries on their cell membrane mannose receptors. In a further preferred aspect of the first embodiment of the present invention galactose as optional ligand can be used to target hepatocytes. Such ligands may be attached to component P¹ and/or P³ by reversible disulfide bonds as defined below or by any other possible chemical attachement, e.g. by amide formation (e.g. carboxylic acids, sulphonic acids, amines, etc), by Michael addition (e.g. maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

In the context of formula (VI) of the present invention components P¹ and P³ represent a linear or branched hydrophilic polymer chain, containing at least one -SH-moiety, each P¹ and P³ independently selected from each other, e.g. from polyethylene glycol (PEG), poly-*N*-(2-hydroxypropyl)methacrylamide, poly-2-(methacryloyloxy)ethyl phosphorylcholines, poly(hydroxyalkyl L-asparagine) or poly(hydroxyalkyl L-glutamine). P¹ and P³ may be identical or different to each other. Preferably, each of hydrophilic polymers P¹ and P³ exhibits a molecular weight of about 1 kDa to about 100 kDa, preferably of about 1 kDa to about 75 kDa, more preferably of about 5 kDa to about 50 kDa, even more preferably of about 5 kDa to about 25 kDa. Additionally, each of hydrophilic polymers P¹ and P³ typically exhibits at least one -SH-moiety, wherein the at least one -SH-moiety is capable to form a disulfide linkage upon reaction with component P² or with component (AA) or (AA)ₓ, if used as linker between P¹ and P² or P³ and P² as defined below and optionally with a further component, e.g. L and/or (AA) or (AA)ₓ, e.g. if two or more -SH-moieties are contained. The following subformulae "P¹-S-S-P²" and "P²-S-S-P³" within generic formula (VI) above (the brackets are omitted for better readability), wherein any of S, P¹ and P³ are as defined herein, typically represent a situation, wherein one-SH-moiety of hydrophilic polymers P¹ and P³ was condensed with one -SH-moiety of component P² of generic formula (VI) above, wherein both sulphurs of these -SH-moieties form a disulfide bond -S-S- as defined herein in formula (VI). These -SH-moieties are typically provided by each of the hydrophilic polymers P¹ and P³, e.g. via an internal cysteine or any further (modified) amino acid or compound which carries a -SH moiety. Accordingly, the subformulae "P¹-S-S-P²" and "P²-S-S-P³" may also be written as "P¹-Cys-Cys-P²" and "P²-Cys-Cys-P³", if the -SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers P¹ and P³ may be modified with a -SH moiety, preferably via a chemical reaction with a compound carrying a -SH moiety, such that each of the hydrophilic polymers P¹ and P³ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers P¹ and P³ as defined herein. Such non-amino compounds may be attached to the hydrophilic polymers P¹ and P³ of formula (VI) of the polymeric carrier according to the present invention via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH-moiety, e.g. of a cysteine or of any further (modified) amino acid or compound, may be present at the terminal ends or internally at any position of hydrophilic polymers P¹ and P³. As defined herein, each of hydrophilic polymers P¹ and P³ typically exhibits at least one -SH-moiety preferably at one terminal end, but may also contain two or even more -SH-moieties, which may be used to additionally attach further components as defined herein, preferably further functional peptides or proteins e.g. a ligand, an amino acid component (AA) or (AA)ₓ, antibodies, cell penetrating peptides or enhancer peptides (e.g. TAT, KALA), etc.

According to one preferred alternative, such further functional peptides or proteins may comprise so called cell penetrating peptides (CPPs) or cationic peptides for transportation. Particularly preferred are CPPs, which induce a pH-mediated conformational change in the endosome and lead to an improved release of the inventive polymeric carrier (in complex with a nucleic acid) from the endosome by insertion into the lipid layer of the liposome. Such called cell penetrating peptides (CPPs) or cationic peptides for transportation, may include, without being limited thereto protamine, nucleoline, spermine or spermidine, poly-L-lysine (PLL), basic polypeptides, poly-arginine, chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from *Drosophila antennapedia*), pAntp, plsl, etc., antimicrobial-derived CPPs e.g. Buforin-2, Bac715-24, SynB, SynB(1), pVEC, hCT-derived peptides, SAP, MAP, PpTG20, Proline-rich peptides, Loligomers, Arginine-rich peptides, Calcitonin-peptides, FGF, Lactoferrin, , poly-L-Lysine, poly-Arginine, histones, VP22 derived or analog peptides, Pestivirus Erns, HSV, VP22 (Herpes simplex), MAP, KALA or protein transduction domains (PTDs, PpT620, prolin-rich peptides, arginine-rich peptides, lysine-rich peptides, Pep-1, L-oligomers, Calcitonin peptide(s), etc.

According to a further preferred aspect of the first embodiment of the present invention, each of hydrophilic polymers P¹ and P³ of formula (VI) of the polymeric carrier used according to the present invention may also contain at least one further functional moiety, which allows attaching further components as defined herein, e.g. a ligand as defined above, or functionalities which allow the attachment of further components, e.g. by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. Further functional moieties may comprise an amino acid component (AA) as defined herein or (AA)ₓ., wherein (AA) is preferably an amino component as defined above. In the above context, x is preferably an integer and may be selected from a range of about 1 to 100, preferably from a range of about 1 to 50, more preferably 1 to 30, and even more preferably selected from a number comprising 1, 2, 3, 4, 5, 6, 7, 8, 9, 10, 11, 12, 13, 14 or 15-30, e.g. from a range of about 1 to 30, from a range of about 1 to 15, or from a number comprising 1, 2, 3, 4, 5, 6, 7, 8, 9, 10, 11, 12, 13, 14 or 15, or may be selected from a range formed by any two of the afore mentioned values. Most preferably, x is 1. Such an amino acid component (AA) or (AA)ₓ may be contained in every part of the inventive polymeric carrier according to formula (VI) above and therefore may be attached to all components of the inventive polymeric carrier according to formula (VI). It is particularly preferred that amino acid component (AA) or (AA)ₓ is present as a ligand or part of the repetitive component [S-P²-S]ₙ within formula (VI) of the inventive polymeric carrier.

In the context of the entire formula (VI) of the inventive polymeric carrier may be preferably defined as follows:

L-P¹-S-[Cys-P²-Cys]ₙ-S-P³-L

wherein L, P¹, P², P³ and n are as defined herein, S is sulphur and each Cys provides for one -SH-moiety for the disulfide bond.

According to a particular aspect, the inventive polymeric carrier according to formula (VI) as defined above, may comprise at least one amino acid component (AA) or (AA)ₓ, as defined above. Such an amino acid component (AA) or (AA)ₓ may be contained in every part of the inventive polymeric carrier according to formula (VI) above and therefore may be attached to all components of the inventive polymeric carrier according to formula (VI). It is particularly preferred that amino acid component (AA) or (AA)ₓ is present as a ligand or part of the repetitive component [S-P²-S]ₙ within formula (VI) of the inventive polymeric carrier. The amino acid component (AA) or (AA)ₓ preferably contains or is flanked (e.g. terminally) by at least one -SH containing moiety, which allows introducing this component (AA) or (AA)ₓ via a disulfide bond into the polymeric carrier according to formula (VI) as defined herein. Such a -SH-containing moiety may be any -SH containing moiety (or, of course, one sulphur of a disulfide bond), e.g. a cysteine residue. In the specific case that the -SH containing moiety represents a cysteine, the amino acid component (AA)ₓ may also be read as -Cys-(AA)ₓ- or -Cys-(AA)ₓ-Cys- wherein Cys represents Cysteine and provides for the necessary -SH-moiety for a disulfide bond. The -SH containing moiety may be also introduced into the amino acid component (AA)ₓ using any of modifications or reactions as shown above for components P¹, P² or P³. In the specific case that the amino acid component (AA)ₓ is linked to two components of the inventive polymeric carrier according to formula (VI) it is preferred that (AA) or (AA)ₓ contains at least two -SH-moieties, e.g. at least two Cysteines, preferably at its terminal ends. This is particularly preferred if (AA) or (AA)ₓ is part of the repetitive component [S-P²-S]ₙ. Alternatively, the amino acid component (AA) or (AA)ₓ is introduced into the inventive polymeric carrier according to formula (VI) as defined herein via any chemical possible addition reaction. Therefore the amino acid component (AA) or (AA)ₓ contains at least one further functional moiety, which allows attaching same to a further component as defined herein, e.g. component P¹ or P³, P², L, or a further amino acid component (AA) or (AA)ₓ, etc. Such functional moieties may be selected from functionalities which allow the attachment of further components, e.g. functionalities as defined herein, e.g. by amide formation (e.g. carboxylic acids, sulphonic acids, amines, etc), by Michael addition (e.g maleinimide moieties, α,β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components.

The amino acid component (AA) or (AA)ₓ in the polymeric carrier of formula (VI) may also occur as a mixed repetitive amino acid component [(AA)ₓ]_{z}, wherein the number of amino acid components (AA) or (AA)ₓ is further defined by integer z. In this context, z may be selected from a range of about 1 to 30, preferably from a range of about 1 to 15, more preferably 1 to 10 or 1 to 5 and even more preferably selected from a number selected from 1, 2, 3, 4, 5, 6, 7, 8, 9, 10, 11, 12, 13, 14 or 15, or may be selected from a range formed by any two of the afore mentioned values.

According to a specific and particularly preferred alternative, the amino acid component (AA) or (AA)ₓ, preferably written as S-(AA)ₓ-S or [S-(AA)ₓ-S] may be used to modify component P², particularly the content of component S-P²-S in repetitive component [S-P²-S]ₙ of the polymeric carrier of formula (VI) above. This may be represented in the context of the entire polymeric carrier according to formula (VI) e.g. by following formula (VIa):

L-P¹-S-{[S-P²-S]ₐ[S-(AA)ₓ-S]_{b}}S-P³-L,

wherein x, S, L, AA, P¹, P² and P³ are preferably as defined herein. In formula (Via) above, any of the single components [S-P²-5] and [S-(AA)ₓ-S] may occur in any order in the subformula {[S-P²-S]ₐ[S-(AA)ₓ-S]b}. The numbers of single components [S-P²-S] and [S-(AA)ₓ-S] in the subformula {[S-P²-S]ₐ[S-(AA)ₓ-S]_{b}} are determined by integers a and b, wherein a + b = n. n is an integer and is defined as above for formula (VI).

a is an integer, typically selected independent from integer b from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, a is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

b is an integer, typically selected independent from integer a from a range of about 0 to 50 or 1 to 50, preferably from a range of about 0, 1, 2 or 3 to 30, more preferably from a range of about 0, 1, 2, 3, 4, or 5 to 25, or a range of about 0, 1, 2, 3, 4, or 5 to 20, or a range of about 0, 1, 2, 3, 4, or 5 to 15, or a range of about 0, 1, 2, 3, 4, or 5 to 10, including e.g. a range of about 3 to 20, 4 to 20, 5 to 20, or 10 to 20, or a range of about 3 to 15, 4 to 15, 5 to 15, or 10 to 15, or a range of about 6 to 11 or 7 to 10. Most preferably, b is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

According to one preferred aspect, the mRNA of the inventive vaccine encoding at least one antigen as defined above may be formulated together with a cationic or polycationic compound and/or with a polymeric carrier, preferably as defined herein.

According to a further preferred aspect, the mRNA of the inventive vaccine encoding at least one antigen as defined above may be formulated together with an (adjuvant) component. According to a particularly preferred aspect, the mRNA of the inventive vaccine encoding at least one antigen as defined above may be formulated to comprise a) an (adjuvant) component, comprising or consisting of at least one immunostimulatory nucleic acid, complexed with a cationic or polycationic compound and/or with a polymeric carrier, preferably as defined herein, and b) at least one free mRNA, encoding an antigen, preferably as defined herein for the inventive vaccine.

In the above context, a cationic or polycationic compound and/or a polymeric carrier used to complex the at least one immunostimulatory nucleic acid in the adjuvant component, may be selected from a cationic or polycationic compound and/or a polymeric carrier as defined above.

Furthermore, an immunostimulatory nucleic acid as defined above for the adjuvant component may be preferably selected from an mRNA as defined herein for the inventive vaccine, encoding at least one antigen. Alternatively, such an immunostimulatory nucleic acid may be selected from an immunostimulatory nucleic acid, as defined herein, preferably an immunostimulatory RNA (isRNA) as defined herein.

In this context, an immunostimulatory nucleic acid, as used herein, is preferably selected from immunostimulatory nucleic acids which are known to bind to TLR receptors. Such an immunostimulatory nucleic acid can be in the form of a(n) (immunostimulatory) CpG nucleic acid, in particular CpG-RNA or CpG-DNA, which preferably induces an innate immune response. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably, the CpG motifs are unmethylated.

Furthermore, an immunostimulatory nucleic acid, as used herein, is preferably selected from an immunostimulatory RNA (isRNA), which preferably elicits an innate immune response. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a (long) (linear) (single-stranded) non-coding RNA. In this context it is particular preferred that the isRNA carries a triphosphate at its 5'-end which is the case for *in vitro* transcribed RNA. An immunostimulatory RNA may also occur as a short RNA oligonucleotide as defined herein. An immunostimulatory RNA as used herein may furthermore be selected from any class of RNA molecules, found in nature or being prepared synthetically, and which can induce an innate immune response and may support an adaptive immune response induced by an antigen. In this context, an immune response may occur in various ways. A substantial factor for a suitable (adaptive) immune response is the stimulation of different T cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T cells. Th2 cells, on the other hand, promote the humoral immune response by stimulation of B-cells for conversion into plasma cells and by formation of antibodies (e.g. against antigens). The Th1/Th2 ratio is therefore of great importance in the induction and maintenance of an adaptive immune response. In connection with the present invention, the Th1/Th2 ratio of the (adaptive) immune response is preferably shifted in the direction towards the cellular response (Th1 response) and a cellular immune response is thereby induced. According to one example, the innate immune system which may support an adaptive immune response may be activated by ligands of Toll-like receptors (TLRs). TLRs are a family of highly conserved pattern recognition receptor (PRR) polypeptides that recognize pathogen-associated molecular patterns (PAMPs) and play a a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogs thereof (CpG DNA) are ligands for TLR9 (Hemmi H et al. (2000) Nature 408:740-5; Bauer S et al. (2001) Proc Natl. Acad. Sci. USA 98, 9237-42). Furthermore, it has been reported that ligands for certain TLRs include certain nucleic acid molecules and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-I, MDA-5, etc.

Preferably, an immunostimulatory nucleic acid, preferably an immunostimulatory RNA (isRNA), as used herein, may comprise any RNA sequence known to be immunostimulatory, including, without being limited thereto, RNA sequences representing and/or encoding ligands of TLRs, preferably selected from human family members TLR1 - TLR10 or murine family members TLR1 - TLR13, more preferably selected from (human) family members TLR1 - TLR10, even more preferably from TLR7 and TLR8, ligands for intracellular receptors for RNA (such as RIG-I or MDA-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Furthermore, (classes of) immunostimulatory RNA molecules, used as a further compound of the inventive vaccine, may include any other RNA capable of eliciting an immune response. Without being limited thereto, such an immunostimulatory RNA may include ribosomal RNA (rRNA), transfer RNA (tRNA), messenger RNA (mRNA), and viral RNA (vRNA). Such an immunostimulatory RNA may comprise a length of 1000 to 5000, of 500 to 5000, of 5 to 5000, or of 5 to 1000, 5 to 500, 5 to 250, of 5 to 100, of 5 to 50 or of 5 to 30 nucleotides.

According to a particularly preferred embodiment, an immunostimulatory nucleic acid sequence, particularly an isRNA, as used herein, may consist of or comprise a nucleic acid of formula (I) or (II):

GₗXₘGₙ, (formula (I))

wherein:
- G: is guanosine, uracil or an analogue of guanosine or uracil;
- X: is guanosine, uracil, adenosine, thymidine, cytosine or an analogue of the above-mentioned nucleotides;
- l: is an integer from 1 to 40,
wherein
when l = 1 G is guanosine or an analogue thereof,
when l > 1 at least 50% of the nucleotides are guanosine or an analogue thereof;
- m: is an integer and is at least 3;
wherein
when m = 3 X is uracil or an analogue thereof,
when m > 3 at least 3 successive uracils or analogues of uracil occur;
- n: is an integer from 1 to 40,
wherein
when n = 1 G is guanosine or an analogue thereof,
when n > 1 at least 50% of the nucleotides are guanosine or an analogue thereof.

CₗXₘCₙ, (formula (II)) wherein:

- C: is cytosine, uracil or an analogue of cytosine or uracil;
- X: is guanosine, uracil, adenosine, thymidine, cytosine or an analogue of the above-mentioned nucleotides;
- l: is an integer from 1 to 40, wherein
when l = 1 C is cytosine or an analogue thereof,
when l > 1 at least 50% of the nucleotides are cytosine or an analogue thereof;
- m: is an integer and is at least 3;
wherein
when m = 3 X is uracil or an analogue thereof,
when m > 3 at least 3 successive uracils or analogues of uracil occur;
- n: is an integer from 1 to 40,
wherein
when n = 1 C is cytosine or an analogue thereof,
when n > 1 at least 50% of the nucleotides are cytosine or an analogue thereof.

The nucleic acids of formula (I) or (II), which may be used as an immunostimulatory nucleic acid sequence, particularly an isRNA, may be relatively short nucleic acid molecules with a typical length of approximately from 5 to 100 (but may also be longer than 100 nucleotides for specific embodiments, e.g. up to 200 nucleotides), from 5 to 90 or from 5 to 80 nucleotides, preferably a length of approximately from 5 to 70, more preferably a length of approximately from 8 to 60 and, more preferably a length of approximately from 15 to 60 nucleotides, more preferably from 20 to 60, most preferably from 30 to 60 nucleotides. If the nucleic acid of formula (I) or (II) has a maximum length of e.g. 100 nucleotides, m will typically be <=98. The number of nucleotides G in the nucleic acid of formula (I) is determined by l or n. I and n, independently of one another, are each an integer from 1 to 40, wherein when l or n = 1 G is guanosine or an analogue thereof, and when l or n > 1 at least 50% of the nucleotides are guanosine or an analogue thereof. For example, without implying any limitation, when l or n = 4 Gₜ or Gₙ can be, for example, a GUGU, GGUU, UGUG, UUGG, GUUG, GGGU, GGUG, GUGG, UGGG or GGGG, etc.; when l or n = 5 Gₗ or Gₙ can be, for example, a GGGUU, GGUGU, GUGGU, UGGGU, UGGUG, UGUGG, UUGGG, GUGUG, GGGGU, GGGUG, GGUGG, GUGGG, UGGGG, or GGGGG, etc.; etc. A nucleotide adjacent to Xₘ in the nucleic acid of formula (I) according to the invention is preferably not a uracil. Similarly, the number of nucleotides C in the nucleic acid of formula (II) according to the invention is determined by l or n. l and n, independently of one another, are each an integer from 1 to 40, wherein when l or n = 1 C is cytosine or an analogue thereof, and when l or n > 1 at least 50% of the nucleotides are cytosine or an analogue thereof. For example, without implying any limitation, when l or n = 4, C₁ or Cₙ can be, for example, a CUCU. CCUU, UCUC, UUCC, CUUC, CCCU, CCUC, CUCC, UCCC or CCCC, etc.; when l or n = 5 C₁ or Cₙ can be, for example, a CCCUU, CCUCU, CUCCU, UCCCU, UCCUC, UCUCC, UUCCC, CUCUC, CCCCU, CCCUC, CCUCC, CUCCC, UCCCC, or CCCCC, etc.; etc. A nucleotide adjacent to Xₘ in the nucleic acid of formula (II) according to the invention is preferably not a uracil. Preferably, for formula (I), when l or n > 1, at least 60%, 70%, 80%, 90% or even 100% of the nucleotides are guanosine or an analogue thereof, as defined above. The remaining nucleotides to 100% (when guanosine constitutes less than 100% of the nucleotides) in the flanking sequences Gₗ and/or Gₙ are uracil or an analogue thereof, as defined hereinbefore. Also preferably, I and n, independently of one another, are each an integer from 2 to 30, more preferably an integer from 2 to 20 and yet more preferably an integer from 2 to 15. The lower limit of l or n can be varied if necessary and is at least 1, preferably at least 2, more preferably at least 3, 4, 5, 6, 7, 8, 9 or 10. This definition applies correspondingly to formula (II).

According to a particularly preferred embodiment, a nucleic acid according to any of formulas (I) or (II) above, which may be used as an immunostimulatory nucleic acid sequence, particularly an isRNA, may be selected from a sequence consisting or comprising any of the following sequences: 
- GGUUUUUUUUUUUUUUUGGG (SEQ ID NO: 289);
- GGGGGUUUUUUUUUUGGGGG (SEQ ID NO: 290);
- GGGGGUUUUUUUUUUUUUUUUUUUUUUUUUUUUUUGGGGG (SEQ ID NO:
291);
- GUGUGUGUGUUUUUUUUUUUUUUUUUUUUGUGUGUGUGU (SEQ ID NO: 292);
- GGUUGGUUGGUUUUUUUUUUUUUUUUUUUUGGUUGGUUGGUU (SEQ ID NO: 293);
- GGGGGGGGGUUGGGGGGGGG (SEQ ID NO: 294);
- GGGGGGGGGUUUGGGGGGGG (SEQ ID NO: 295);
- GGGGGGGUUUUUGGGGGGGG (SEQ ID NO: 296);
- GGGGGGGUUUUUUUGGGGGG (SEQ ID NO: 297);
- GGGGGGUUUUUUUUGGGGGG (SEQ ID NO: 298);
- GGGGGGUUUUUUUUUGGGGG (SEQ ID NO: 299);
- GGGGGGUUUUUUUUUUGGGG (SEQ ID NO: 300);
- GGGGGUUUUUUUUUUUGGGG (SEQ ID NO: 301);
- GGGGGUUUUUUUUUUUUGGG (SEQ ID NO: 302);
- GGGGUUUUUUUUUUUUUGGG (SEQ ID NO: 303);
- GGGGUUUUUUUUUUUUUUGG (SEQ ID NO: 304);
- GGUUUUUUUUUUUUUUUUGG (SEQ ID NO: 305);
- GUUUUUUUUUUUUUUUUUUG (SEQ ID NO: 306);
- GGGGGGGGGUUUGGGGGGGGG (SEQ ID NO: 307);
 
- GGGGGGGGGUUUGGGGGGGG (SEQ ID NO: 308);
- GGGGGGGGUUUUUGGGGGGG (SEQ ID NO: 309);
- GGGGGGGUUUUUUGGGGGGG (SEQ ID NO: 310);
- GGGGGGGUUUUUUUGGGGGG (SEQ ID NO: 311);
- GGGGGGUUUUUUUUGGGGGG (SEQ ID NO: 312);
- GGGGGGUUUUUUUUUGGGGG (SEQ ID NO: 313);
- GGGGGUUUUUUUUUUGGGGG (SEQ ID NO: 314);
- GGGGGUUUUUUUUUUUGGGG (SEQ ID NO: 315);
- GGGGUUUUUUUUUUUUGGGG (SEQ ID NO: 316);
- GGGGUUUUUUUUUUUUUGGG (SEQ ID NO: 317);
- GGGUUUUUUUUUUUUUUGGG (SEQ ID NO: 318);
- GGUUUUUUUUUUUUUUUUGG (SEQ ID NO: 319);
- GGGGGGGGGGUUGGGGGGGGGG (SEQ ID NO: 320);
- GGGGGGGGGUUUGGGGGGGGGG (SEQ ID NO: 321);
- GGGGGGGGGUUUUGGGGGGGGG (SEQ ID NO: 322);
- GGGGGGGGGUUUUUGGGGGGGG (SEQ ID NO: 323);
- GGGGGGGGUUUUUUGGGGGGGG (SEQ ID NO: 324);
- GGGGGGGGUUUUUUUGGGGGGG (SEQ ID NO: 325);
- GGGGGGGUUUUUUUUGGGGGGG (SEQ ID NO: 326);
- GGGGGGGUUUUUUUUUGGGGGG (SEQ ID NO: 327);
- GGGGGGUUUUUUUUUUGGGGGG (SEQ ID NO: 328);
- GGGGGGUUUUUUUUUUUGGGGG (SEQ ID NO: 329);
- GGGGGUUUUUUUUUUUUGGGGG (SEQ ID NO: 330);
- GGGGUUUUUUUUUUUUUUGGGG (SEQ ID NO: 331);
- GGUUUUUUUUUUUUUUUUUUGGG (SEQ ID NO: 332);
- GUUUUUUUUUUUUUUUUUUUUUUUUUUUUG (SEQ ID NO: 333);
- GGUUUUUUUUUUUUUUUUUUUUUUUUUUUUGG (SEQ ID NO: 334);
- GGGUUUUUUUUUUUUUUUUUUUUUUUUUUUUGGG (SEQ ID NO: 335);
- GGGGUUUUUUUUUUUUUUUUUUUUUUUUUUUUGGGG (SEQ ID NO: 336);
- GGGGGUUUUUUUUUUUUUUUUUUUUUUUUUUUUGGGGG (SEQ ID NO: 337);
- GGGGGGUUUUUUUUUUUUUUUUUUUUUUUUUUUUGGGGGG (SEQ ID NO:
338);
- GGGGGGGUUUUUUUUUUUUUUUUUUUUUUUUUUUUGGGGGGG (SEQ ID
NO: 339);
- GGGGGGGGUUUUUUUUUUUUUUUUUUUUUUUUUUUUGGGGGGGG (SEQ ID
NO: 340);
- GGGGGGGGGUUUUUUUUUUUUUUUUUUUUUUUUUUUUGGGGGGGGG (SEQ
ID NO: 341);
- GGUUGG (SEQ ID NO: 342);
- GGUUUGG (SEQ ID NO: 343);
- GGUUUUGG (SEQ ID NO: 344);
- GGUUUUUGG (SEQ ID NO: 345);
- GGUUUUUUGG (SEQ ID NO: 346);
- GGUUUUUUUGG (SEQ ID NO: 347);
- GGUUUUUUUUGG (SEQ ID NO: 348);
- GGUUUUUUUUUGG (SEQ ID NO: 349);
- GGUUUUUUUUUUGG (SEQ ID NO: 350);
- GGUUUUUUUUUUUGG (SEQ ID NO: 351);
- GGUUUUUUUUUUUUGG (SEQ ID NO: 352);
- GGUUUUUUUUUUUUUGG (SEQ ID NO: 353);
 
- GGUUUUUUUUUUUUUUUGG (SEQ ID NO: 354);
- GGGUUUGGG (SEQ ID NO: 355);
- GGGUUUUGGG (SEQ ID NO: 356);
- GGGUUUUUGGG (SEQ ID NO: 357);
- GGGUUUUUUGGG (SEQ ID NO: 358);
- GGGUUUUUUUGGG (SEQ ID NO: 359);
- GGGUUUUUUUUGGG (SEQ ID NO: 360);
- GGGUUUUUUUUUGGG (SEQ ID NO: 361);
- GGGUUUUUUUUUUGGG (SEQ ID NO: 362);
- GGGUUUUUUUUUUUGGG (SEQ ID NO: 363);
- GGGUUUUUUUUUUUUGGG (SEQ ID NO: 364);
- GGGUUUUUUUUUUUUUGGG (SEQ ID NO: 365);
- GGGUUUUUUUUUUUUUUUGGGUUUUUUUUUUUUUUUGG-
GUUUUUUUUUUUUUUUGGG
(SEQ ID NO: 366);
- GGGUUUUUUUUUUUUUUUUGGGGGUUUUUUUUUUUUUUUUGGG (SEQ ID
NO: 367);
- GGGUUGGGUUGGGUUGGGUUGGGUUGGGUUGGGUUGGGUUGG-
GUUGGG (SEQ ID NO: 368);
- GGUUUUUUUUUUUUUUUUGGG (short GU-rich, SEQ ID NO: 369)

or

- CCCUUUUUUUUUUUUUUUCCCUUUUUUUUUUUUUUUCCC-
UUUUUUUUUUUUUUUCCC
(SEQ ID NO: 370)
- CCCUUCCCUUCCCUUCCCUUCCCUUCCCUUCCCUUCCCUUCCCUUCCC
(SEQ ID NO: 371)
- CCCUUUUUUUUUUUUUUUUCCCCCUUUUUUUUUUUUUUUUCCC (SEQ ID NO:
372)
 or from a sequence having at least 60%, 70%, 80%, 90%, or even 95% sequence identity with any of these sequences.

According to a further particularly preferred embodiment, an immunostimulatory nucleic acid sequence, particularly an isRNA, as used herein, may consist of or comprise a nucleic acid of formula (III) or (IV):

(NᵤGₗXₘGₙNᵥ)ₐ, (formula (III)

wherein:
G is guanosine (guanine), uridine (uracil) or an analogue of guanosine (guanine) or uridine (uracil), preferably guanosine (guanine) or an analogue thereof;

- X: is guanosine (guanine), uridine (uracil), adenosine (adenine), thymidine (thymine), cytidine (cytosine), or an analogue of these nucleotides (nucleosides), preferably uridine (uracil) or an analogue thereof;
- N: is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);
- a: is an integer from 1 to 20, preferably from 1 to 15, most preferably from 1 to 10;
- I: is an integer from 1 to 40,
wherein
when l = 1, G is guanosine (guanine) or an analogue thereof,
when l > 1, at least 50% of these nucleotides (nucleosides) are guanosine (guanine)
or an analogue thereof;
- m: is an integer and is at least 3;
wherein
when m = 3, X is uridine (uracil) or an analogue thereof, and
when m > 3, at least 3 successive uridines (uracils) or analogues of uridine (uracil) occur;
- n: is an integer from 1 to 40,
wherein
when n = 1, G is guanosine (guanine) or an analogue thereof,
when n > 1, at least 50% of these nucleotides (nucleosides) are guanosine (guanine)
or an analogue thereof;
- u,v: may be independently from each other an integer from 0 to 50, preferably wherein when u = 0, v ≥ 1, or
when v = 0, u ≥ 1;
wherein the nucleic acid molecule of formula (III) has a length of at least 50 nucleotides, preferably of at least 100 nucleotides, more preferably of at least 150 nucleotides, even more preferably of at least 200 nucleotides and most preferably of at least 250 nucleotides.

(NᵤCₗXₘCₙNᵥ)ₐ (formula (IV))

wherein:
- C: is cytidine (cytosine), uridine (uracil) or an analogue of cytidine (cytosine) or uridine (uracil), preferably cytidine (cytosine) or an analogue thereof;
- X: is guanosine (guanine), uridine (uracil), adenosine (adenine), thymidine (thymine), cytidine (cytosine) or an analogue of the above-mentioned nucleotides (nucleosides), preferably uridine (uracil) or an analogue thereof;
- N: is each a nucleic acid sequence having independent from each other a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides);
- a: is an integer from 1 to 20, preferably from 1 to 15, most preferably from 1 to 10;
- l: is an integer from 1 to 40,
wherein
when I = 1, C is cytidine (cytosine) or an analogue thereof,
when l > 1, at least 50% of these nucleotides (nucleosides) are cytidine (cytosine) or an analogue thereof;
- m: is an integer and is at least 3;
wherein
when m = 3, X is uridine (uracil) or an analogue thereof,
when m > 3, at least 3 successive uridines (uracils) or analogues of uridine (uracil) occur;
- n: is an integer from 1 to 40,
wherein
when n = 1, C is cytidine (cytosine) or an analogue thereof,
when n > 1, at least 50% of these nucleotides (nucleosides) are cytidine (cytosine) or an analogue thereof.
- u, v: may be independently from each other an integer from 0 to 50, preferably wherein when u = 0, v ≥ 1, or
when v = 0, u ≥ 1;
wherein the nucleic acid molecule of formula (IV) according to the invention has a length of at least 50 nucleotides, preferably of at least 100 nucleotides, more preferably of at least 150 nucleotides, even more preferably of at least 200 nucleotides and most preferably of at least 250 nucleotides.

Any of the definitions given above in formulae (I) and (II), e.g. for elements N (i.e. Nᵤ and Nᵥ) and X (Xₘ), particularly the core structure as defined above, as well as for integers a, I, m, n, u and v, similarly apply to elements of formula (III) and (IV) correspondingly. The definition of bordering elements Nᵤ and Nᵥ in formula (IV) is identical to the definitions given above for Nᵤ and Nᵥ in formula (IV).

According to a very particularly preferred embodiment, the inventive nucleic acid molecule according to formula (IV), which may be used as an immunostimulatory nucleic acid sequence, particularly an isRNA, may be selected from e.g. any of the following sequences:

UAGCGAAGCUCUUGGACCUAGGUUUUUUUUUUUUUUUGGGUGCGUUCCUA-
GAAGUACACG (SEQ ID NO: 373)

UAGCGAAGCUCUUGGACCUAGGUUUUUUUUUUUUUUGGGUGCGUUCCUA-
GAAGUACACG AUCGCUUCGA GAACCUGGAUCCAAAAAAAAAAAAAACCCACG-
CAAGGAUCUUCAUGUGC (SEQ ID NO: 374)

GGGAGAAAGCUCAAGCUUGGAGCAAUGCCCGCACAUUGAGGAAACCGAGUUG-
CAUAUCUCAGAGUAUUGGCCCCCGUGUAGGUUAUUCUUGACAGACAGUG-
GAGCUUAUUCACUCCCAGGAUCCGAGUCGCAUACUACGGUACUGGUGACAGAC-
CUAGGUCGUCAGUUGACCAGUCCGCCACUAGACGUGAGUCCGUCAAAGCAGUUA-
GAUGUUACACUCUAUUAGAUC (SEQ ID NO: 375)

GGGAGAAAGCUCAAGCUUGGAGCAAUGCCCGCACAUUGAGGAAACCGAGUUG-
CAUAUCUCAGAGUAUUGGCCCCCGUGUAGGUUAUUCUUGACAGACAGUG-
GAGCUUAUUCACUCCCAGGAUCCGAGUCGCAUACUACGGUACUGGUGACAGAC-
CUAGGUCGUCAGUUGACCAGUCCGCCACUAGACGUGAGUCCGUCAAAGCAGUUA-
GAUGUUACACUCUAUUAGAUCUCGGAUUACAGCUGGAAGGAGCAGGAGUAGUG-
UUCUUGCUCUAAGUACCGAGUGUGCCAAUACCCGAUCAGCUUAUUAACGAAC-
GGCUCCUCCUCUUAGACUGCAGCGUAAGUGCGGAAUCUGGGGAUCAAAUUACU-
GACUGCCUGGAUUACCCUCGGACAUAUAACCUUGUAGCAC-
GCUGUUGCUGUAUAGGUGACCAACGCCCACUCGAGUAGACCAGCUCU-
CUUAGUCCGGACAAUGAUAGGAGGCGCGGUCAAUCUACUUCUGGCUAGUU-
AAGAAUAGGCUGCACCGACCUCUAUAAGUAGCGUGUCCUCUAG (SEQ ID NO: 376)

GGGAGAAAGCUCAAGCUUGGAGCAAUGCCCGCACAUUGAGGAAACCGAGUUG-
CAUAUCUCAGAGUAUUGGCCCCCGUGUAGGUUAUUCUUGACAGACAGUG-
GAGCUUAUUCACUCCCAGGAUCCGAGUCGCAUACUACGGUACUGGUGACAGAC-
CUAGGUCGUCAGUUGACCAGUCCGCCACUAGACGUGAGUCCGUCAAAGCAGUUA-
GAUGUUACACUCUAUUAGAUCUCGGAUUACAGCUGGAAGGAGCAGGAGUAGUG-

UUCUUGCUCUAAGUACCGAGUGUGCCCAAUACCCGAUCAGCUUAUUAACGAAC-
GGCUCCUCCUCUUAGACUGCAGCGUAAGUGCGGAAUCUGGGGAUCAAAUUACU-
GACUGCCUGGAUUACCCUCGGACAUAUAACCUUGUAGCAC-
GCUGUUGCUGUAUAGGUGACCAACGCCCACUCGAGUAGACCAGCUCU-
CUUAGUCCGGACAAUGAUAGGAGGCGCGGUCAAUCUACUUCUGGCUAGUU-
AAGAAUAGGCUGCACCGACCUCUAUAAGUAGCGUGUCCUCUAGAGCUAC-
GCAGGUUCGCAAUAAAAGCGUUGAUUAGUGUGCAUAGAACAGACCU-
CUUAUUCGGUGAAACGCCAGAAUGCUAAAUUCCAAUAACUCUUCCCAAAC-
GCGUACGGCCGAAGACGCGCUUAUCUUGUGUACGUUCUCGCACAUGGAA-
GAAUCAGCGGGCAUGGUGGUAGGCAAUAGGGGAGCUGGGUAG-
CAGCGAAAAGGGCCCCUGCGCACGUAGCUUCGCUGUUCGUCUGAAACAACCCGG-
CAUCCGUUGUAGCGAUCCCGUUAUCAGUGUUAUUCUUGUGCGCACUAAGAU-
UCAUGGUGUAGUCGACAAUAACAGCGUCUUGGCAGAUUCUGGUCACGUG-
CCCUAUGCCCGGGCUUGUGCCUCUCAGGUGCACAGCGAUACUUAAA-
GCCUUCAAGGUACUCGACGUGGGUACCGAUUCGUGACACUUCCUAAGAU-
UAUUCCACUGUGUUAGCCCCGCACCGCCGACCUAAACUGGUCCAAUGUAUAC-
GCAUUCGCUGAGCGGAUCGAUAAUAAAGCUUGAAUU (SEQ ID NO: 377)

GGGAGAAAGCUCAAGCUUAUCCAAGUAGGCUGGUCACCUGUACAAC-
GUAGCCGGUAUUUUUUUUUUUUUUUUUUUUGACCGUCUCAAGGUCCAA-
GUUAGUCUGCCUAUAAAGGUGCGGAUCCACAGCUGAUGAAAGACUUGUG-
CGGUACGGUUAAUCUCCCUUUUUUUUUUUUUUUUUUUUAGU-
AAAUGCGUCUACUGAAUCCAGCGAUGAUGCUGGCCCAGAUC (SEQ ID NO: 378)

GGGAGAAAGCUCAAGCUUAUCCAAGUAGGCUGGUCACCUGUACAAC-
GUAGCCGGUAUUUUUUUUUUUUUUUUUUUUUGACCGUCUCAAGGUCCAA-
GUUAGUCUGCCUAUAAAGGUGCGGAUCCACAGCUGAUGAAAGACUUGUG-
CGGUACGGUUAAUCUCCCCUUUUUUUUUUUUUUUUUUUUUUAGU-
AAAUGCGUCUACUGAAUCCAGCGAUGAUGCUGGCCCAGAUCUUCGACCACAA-
GUGCAUAUAGUAGUCAU-
CGAGGGUCGCCUUUUUUUUUUUUUUUUUUUUUUUUUGGCCCAGUUCUGAGACU-
UCGCUAGAGACUACAGUUACAGCUGCAGUAGUAACCACUGCGGCUAUUGCAG-
GAAAUCCCGUUCAGGUUUUUUUUUUUUUUUUUUUUUUUUCCGCUCACUAUGAU-
UAAGAACCAGGUGGAGUGUCACUGCUCUCGAGGUCUCACGA-
GAGCGCUCGAUACAGUCCUUGGAA-
GAAUCUUUUUUUUUUUUUUUUUUUUUUUGUGCGACGAUCACAGAGAACU-
UCUAUUCAUGCAGGUCUGCUCUA (R 722 SEQ ID NO: 379)

GGGAGAAAGCUCAAGCUUAUCCAAGUAGGCUGGUCACCUGUACAAC-
GUAGCCGGUAUUUUUUUUUUUUUUUUUUUUUUUGACCGUCUCAAGGUCCAA-
GUUAGUCUGCCUAUAAAGGUGCGGAUCCACAGCUGAUGAAAGACUUGUG-
CGGUACGGUUAAUCUCCCUUUUUUUUUUUUUUUUUUUUUUUUAGU-
AAAUGCGUCUACUGAAUCCAGCGAUGAUGCUGGCCCAGAUCUUCGACCACAA-
GUGCAUAUAGUAGUCAU-
CGAGGGUCGCCUUUUUUUUUUUUUUUUUUUUUUUUUUGGCCCAGUUCUGAGACU-
UCGCUAGAGACUACAGUUACAGCUGCAGUAGUAACCACUGCGGCUAUUGCAG-
GAAAUCCCGUUCAGGUUUUUUUUUUUUUUUUUUUUUUUUUCCGCUCACUAUGAU-
UAAGAACCAGGUGGAGUGUCACUGCUCUCGAGGUCUCACGA-
GAGCGCUCGAUACAGUCCUUGGAA-
GAAUCUUUUUUUUUUUUUUUUUUUUUUUUGUGCGACGAUCACAGAGAACU-

UCUAUUCAUGCAGGUCUGCUCUAGAACGAACUGACCUGACGCCUGAACU-
UAUGAGCGUGCGUAUUUUUUUUUUUUUUUUUUUUUCCUCCCAACAAAU-
GUCGAUCAAUAGCUGGGCUGUUGGAGACGCGUCAGCAAAUGCCGUG-
GCUCCAUAGGACGUGUAGACUUCUAUUUUUUUUUUUUUUUUUUUUCCCGG-
GACCACAAAUAAUAUUCUUGCUUGGUUGGGCGCAAGGGCCCCGUAUCAGGUCAU-
AAACGGGUACAUGUUG-
CACAGGCUCCUUUUUUUUUUUUUUUUUUUUUUUCGCUGAGUUAUUCCGGUCUC
AAAAGACGGCAGACGUCAGUCGACAACACGGUCUAAAGCAGUG-
CUACAAUCUGCCGUGUUCGUGUUUUUUUUUUUUUUUUUUUUUGUGAACCUACAC-
GGCGUGCACUGUAGUUCGCAAU-
UCAUAGGGUACCGGCUCAGAGUUAUGCCUUGGUUGAAACUGCCCAGCAUACU-
UUUUUUUUUUUUUUUUUUUUCAUAUUCCCAUGCU-
AAGCAAGGGAUGCCGCGAGUCAUGUUAAGCUUGAAUU (SEQ ID NO: 380)

According to another very particularly preferred embodiment, the nucleic acid molecule according to formula (V) may be selected from e.g. any of the following sequences: 
UAGCGAAGCUCUUGGACCUACCUUUUUUUUUUUUUUCCCUGCGUUCCUA-
GAAGUACACG (SEQ ID NO: 381)
 or 
UAGCGAAGCUCUUGGACCUACCUUUUUUUUUUUUUUUCCCUGCGUUCCUA GAA-
GUACACGAUCGCUUCGAGAACCUGGAUGGAAAAAAAAAAAAAAAGGGAC-
GCAAGGAUCUUCAUGUGC (SEQ ID NO: 382)
 or from a sequence having at least 60%, 70%, 80%, 90%, or even 95% sequence identity with any of these sequences.

Finally, the so called "(adjuvant) component", which may be used to together with the mRNA in the inventive vaccine, is preferably prepared according to a first step by complexing the at least one (m)RNA of the (adjuvant) component with a cationic or polycationic compound and/or with a polymeric carrier, preferably as defined herein, in a specific ratio to form a stable complex. In this context, it is highly preferable, that no free cationic or polycationic compound or polymeric carrier or only a neglectably small amount thereof remains in the (adjuvant) component after complexing the (m)RNA. Accordingly, the ratio of the (m)RNA and the cationic or polycationic compound and/or the polymeric carrier in the (adjuvant) component is typically selected in a range that the (m)RNA is entirely complexed and no free cationic or polycationic compound or polymeric carrier or only a neglectably small amount thereof remains in the composition. Preferably the ratio of the (adjuvant) component, i.e. the ratio of the (m)RNA to the cationic or polycationic compound and/or the polymeric carrier, preferably as defined herein, is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w). Alternatively, the ratio of the (m)RNA to the cationic or polycationic compound and/or the polymeric carrier, preferably as defined herein, in the (adjuvant) component, may also be calculated on the basis of the nitrogen/phosphate ratio (N/P-ratio) of the entire complex of the (adjuvant) component. In the context of the present invention, an N/P-ratio is preferably in the range of about 0.1-10, preferably in a range of about 0.3-4 and most preferably in a range of about 0.5-2 or 0.7-2 regarding the ratio of (m)RNA: cationic or polycationic compound and/or polymeric carrier, preferably as defined herein, in the complex, and most preferably in the range of about 0.7-1.5, preferably provided the cationic or polycationic compound in the complex is a cationic or polycationic cationic or polycationic protein or peptide and/or the polymeric carrier is as defined above. Such ratios, particularly weight and/or N/P ratios may also be applied to ratios of the at least one mRNA encoding at least one antigen as defined herein to a cationic or polycationic polymer or a polymeric carrier as defined herein used to complex the at least one mRNA.

According to a further preferred aspect, the mRNA of the inventive vaccine encoding at least one antigen as defined above may be formulated together with an (adjuvant) component as defined above, wherein the inventive vaccine may comprise a) an (adjuvant) component, comprising or consisting of at least one (m)RNA, complexed with a cationic or polycationic compound and/or with a polymeric carrier, preferably as defined herein, and b) at least one free mRNA, encoding an antigen, preferably as defined herein. This formulation is preferably as defined above. Furthermore, the entire formulation of a) and b) may be additionally packaged with a carrier molecule to allow combined packaging the (adjuvant) component and the antigen. Such a carrier molecule may be selected from any polymer suitable for packaging and preferably transporting the entire formulation of a) and b) into cells, tissue, etc., of a patient as defined herein, e.g. from a cationic or polycationic polymer as defined herein or from any further polymer suitable for this purpose, e.g. a polymeric carrier as defined above.

The ratio of all components of the entire inventive vaccine composition, as defined above, preferably, an adjuvant component comprising or consisting of at least one immunostimulatory nucleic acid sequence, complexed with a cationic or polycationic compound, the at least one mRNA encoding at least one antigen, and/or a carrier molecule, formulated in the inventive vaccine, may be calculated on the basis of the nitrogen/phosphate ratio (N/P-ratio) of all these components. In the context of the present invention, an N/P-ratio is preferably in the range of about 0.01-4, 0.01-2, 0.1-2 or 0.1-1.5 regarding the ratio of nucleic acids: cationic or polycationic peptide contained in the inventive vaccine, and most preferably in the range of about 0.1-1. Such an N/P ratio is preferably designed to provide good transfection properties *in vivo* and transport into and through cell membranes. Preferably, for this purpose, cationic or polycationic compound and/or polymeric carriers as used herein, are based on peptide sequences.

In a further preferred aspect of the present invention the inventive vaccine may comprise a pharmaceutically acceptable carrier and/or vehicle. In the context of the present invention, a pharmaceutically acceptable carrier typically includes the liquid or non-liquid basis of a composition comprising the components of the inventive vaccine. If the composition is provided in liquid form, the carrier will typically be pyrogen-free water; isotonic saline or buffered (aqueous) solutions, e.g. phosphate, citrate etc. buffered solutions. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds, which are suitable for administration to a patient to be treated, may be used as well for the inventive vaccine. The term "compatible" as used here means that these constituents of the inventive vaccine are capable of being mixed with the components of the inventive vaccine in such a manner that no interaction occurs which would substantially reduce the pharmaceutical effectiveness of the inventive vaccine under typical use conditions.

According to a specific aspect, the inventive vaccine may comprise an adjuvant. In this context, an adjuvant may be understood as any compound, which is suitable to initiate or increase an immune response of the innate immune system, i.e. a non-specific immune response. With other words, when administered, the vaccine preferably elicits an innate immune response due to the adjuvant, optionally contained therein. Preferably, such an adjuvant may be selected from an adjuvant known to a skilled person and suitable for the present case, i.e. supporting the induction of an innate immune response in a mammal, e.g. an adjuvant protein as defined above or an adjuvant as defined in the following.

According to one aspect such an adjuvant may be selected from an (adjuvant) component as defined above.

According to one further aspect such an adjuvant may be selected from any adjuvant known to a skilled person and suitable for the present case, i.e. supporting the induction of an innate immune response in a mammal and/or suitable for depot and delivery of the components of the inventive vaccine. Preferred as adjuvants suitable for depot and delivery are cationic or polycationic compounds as defined above. Likewise, the adjuvant may be selected from the group consisting of, without being limited thereto, cationic or polycationic compounds as defined above, from chitosan, TDM, MDP, muramyl dipeptide, pluronics, alum solution, aluminium hydroxide, ADJUMERTM (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINETM (propanediamine); BAY R1005TM ((N-(2-deoxy-2-L-leucylaminob- D-glucopyranosyl)-N-octadecyl-dodecanoyl-amide hydroacetate); CALCITRIOLTM (1-alpha,25-dihydroxy-vitamin D3); calcium phosphate gel; CAPTM (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D35 glutamine (GMDP), ii) di-methyldioctadecylammonium chloride (DDA), iii) zinc-L-proline salt complex (ZnPro-8); GM-CSF); GMDP (N-acetylglucosaminyl-(b1-4)-N-acetylmuramyl-L47 alanyl-D-isoglutamine); imiquimod (1-(2-methypropyl)-1H-imidazo[4,5-c]quinoline-4-amine); ImmTher™ (N-acetylglucosaminyl-N-acetylmuramyl-L-Ala-D-isoGlu-L-Ala-glycerol di-palmitate); DRVs (immunoliposomes prepared from dehydration-rehydration vesicles); interferongamma; interleukin-1beta; interleukin-2; interleukin-7; interleukin-12; ISCOMSTM; ISCOPREP 7.0.3. TM; liposomes; LOXORIBINETM (7-allyl-8-oxoguanosine); LT 5 oral adjuvant (*E.coli* labile enterotoxin-protoxin); microspheres and microparticles of any composition; MF59TM; (squalenewater emulsion); MONTANIDE ISA 51TM (purified incomplete Freund's adjuvant); MONTANIDE ISA 720TM (metabolisable oil adjuvant); MPLTM (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDETM (Nac-Mur-L-Ala-D-Gln-OCH3); MURA-PALMITINETM and DMURAPALMITINETM (Nac-Mur-L-Thr-D-isoGln-sn-glyceroldipalmitoyl); NAGO (neuraminidase- galactose oxidase); nanospheres or nanoparticles of any composition; NISVs (non-ionic surfactant vesicles); PLEURANTM (-glucan); PLGA, PGA and PLA (homo- and co-polymers of lactic acid and glycolic acid; microspheres/nanospheres); PLURONIC L121TM; PMMA (polymethylmethacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULONTM (QS-21); Quil-A (Quil-A saponin); S-28463 (4-amino-otec-dimethyl-2-ethoxymethyl-1H-imidazo[4,5-c]quinoline-1-ethanol); SAF-1TM ("Syntex adjuvant formulation"); Sendai proteoliposomes and Sendaicontaining lipid matrices; Span-85 (sorbitan trioleate); Specol (emulsion of Marcol 52, Span 85 and Tween 85); squalene or Robane^{®} (2,6,10,15,19,23-hexamethyltetracosan and 2,b,10,15,19,23-hexamethyl-2,6,10,14,18,22-tetracosahexane); stearyltyrosine (octadecyltyrosine hydrochloride); Theramid^{®} (N-acetylglucosaminyl-N-acetylmuramyl-L-Ala-D-isoGlu-L-Aladipalmitoxypropylamide); Theronyl-MDP (TermurtideTM or [thr 1]-MDP; N-acetylmuramyl-Lthreonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including Q521, Quil A, Iscomatrix, ISCOM; adjuvants suitable for costimulation including Tomatine, biopolymers, including PLG, PMM, Inulin, microbe derived adjuvants, including Romurtide, DETOX, MPL, CWS, Mannose, CpG nucleic acid sequences, CpG7909, ligands of human TLR 1-10, ligands of murine TLR 1-13, ISS-1018, 35 IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Particularly preferred, an adjuvant may be selected from adjuvants, which support induction of a Th1-immune response or maturation of naïve T-cells, such as GM-CSF, IL-12, IFNg, any RNA as defined herein, preferably an immunostimulatory RNA, CpG DNA, etc.

The inventive vaccine may additionally contain a further immunotherapeutic agent selected from immunoglobulins, preferably IgGs, monoclonal or polyclonal antibodies, polyclonal serum or sera, etc. Preferably, such a further immunotherapeutic agent may be provided as a peptide/protein or may be encoded by a nucleic acid, preferably by a DNA or an RNA, more preferably an mRNA. Such an immunostimulatory agent allows providing passive vaccination additional to active vaccination triggered by the mRNA encoded antigen of the inventive composition or vaccine composition.

The inventive vaccine can additionally contain one or more auxiliary substances in order to increase its immunogenicity or immunostimulatory capacity, if desired. A synergistic action of the inventive vaccine and of an auxiliary substance, which may be optionally contained in the vaccine or may be formulated with the inhibitor, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

The inventive vaccine can also additionally contain any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13, a ligand of a NOD-like receptor, or a ligand of a RIG-I like receptor.

In this context the inventive vaccine may also additionally contain an immunostimulatory nucleic acid, preferably an immunostimulatory RNA (isRNA), as defined above.

The inventive vaccine as defined according to the first embodiment of the present invention may furthermore comprise further additives or additional compounds. Further additives which may be included in the inventive vaccine are emulsifiers, such as, for example, Tween^{®}; wetting agents, such as, for example, sodium lauryl sulfate; colouring agents; taste-imparting agents, pharmaceutical carriers; tablet-forming agents; stabilizers; antioxidants; preservatives.

One further additive, which may be contained in the inventive vaccine, may be an anti-bacterial agent. In this context, any anti-bacterial agents known to one of skill in the art may be used in combination with the components of the inventive vaccine as defined herein. Non-limiting examples of anti-bacterial agents include Amikacin, Amoxicillin, Amoxicillin-clavulanic acid, Amphothericin-B, Ampicillin, Ampicllin-sulbactam, Apramycin, Azithromycin, Aztreonam, Bacitracin, Benzylpenicillin, Caspofungin, Cefaclor, Cefadroxil, Cefalexin, Cefalothin, Cefazolin, Cefdinir, Cefepime, Cefixime, Cefmenoxime, Cefoperazone, Cefoperazone-sulbactam, Cefotaxime, Cefoxitin, Cefbirome, Cefpodoxime, Cefpodoxime-clavulanic acid, Cefpodoxime-sulbactam, Cefbrozil, Cefquinome, Ceftazidime, Ceftibutin, Ceftiofur, Ceftobiprole, Ceftriaxon, Cefuroxime, Chloramphenicole, Florfenicole, Ciprofloxacin, Clarithromycin, Clinafloxacin, Clindamycin, Cloxacillin, Colistin, Cotrimoxazol (Trimthoprim/sulphamethoxazole), Dalbavancin, Dalfopristin/Quinopristin, Daptomycin, Dibekacin, Dicloxacillin, Doripenem, Doxycycline, Enrofloxacin, Ertapenem, Erythromycin, Flucloxacillin, Fluconazol, Flucytosin, Fosfomycin, Fusidic acid, Garenoxacin, Gatifloxacin, Gemifloxacin, Gentamicin, Imipenem, Itraconazole, Kanamycin, Ketoconazole, Levofloxacin, Lincomycin, Linezolid, Loracarbef, Mecillnam (amdinocillin), Meropenem, Metronidazole, Meziocillin, Mezlocillin- sulbactam, Minocycline, Moxifloxacin, Mupirocin, Nalidixic acid, Neomycin, Netilmicin, Nitrofurantoin, Norfloxacin, Ofloxacin, Oxacillin, Pefloxacin, Penicillin V, Piperacillin, Piperacillin-sulbactam, Piperacillin-tazobactam, Rifampicin, Roxythromycin, Sparfloxacin, Spectinomycin, Spiramycin, Streptomycin, Sulbactam, Sulfamethoxazole, Teicoplanin, Telavancin, Telithromycin, Temocillin, Tetracyklin, Ticarcillin, Ticarcillin-clavulanic acid, Tigecycline, Tobramycin, Trimethoprim, Trovafloxacin, Tylosin, Vancomycin, Virginiamycin, and Voriconazole.

Another additive, which may be contained in the inventive vaccine, may be an anti-viral agents, preferably, but are not limited to, nucleoside analogs (e.g., zidovudine, acyclovir, gangcyclovir, vidarabine, idoxuridine, trifluridine, and ribavirin), foscarnet, amantadine, peramivir, rimantadine, saquinavir, indinavir, ritonavir, alpha-interferons and other interferons, AZT, t-705, zanamivir (Relenza^{®}), and oseltamivir (Tamiflu^{®}). Other anti-viral agents include influenza virus vaccines, e.g., Fluarix^{®} (Glaxo SmithKline), FluMist^{®} (MedImmune Vaccines), Fluvirin^{®} (Chiron Corporation), Flulaval^{®} (GlaxoSmithKline), Afluria^{®} (CSL Bio-therapies Inc.), Agriflu^{®} (Novartis) or Fluzone^{®} (Aventis Pasteur).

The inventive vaccine typically comprises a "safe and effective amount" of the components of the inventive vaccine as defined herein. As used herein, a "safe and effective amount" preferably means an amount of the components, preferably of the at least one mRNA, that is sufficient to significantly induce a positive modification of a disease or disorder as defined herein. At the same time, however, a "safe and effective amount" is small enough to avoid serious side-effects and to permit a sensible relationship between advantage and risk. The determination of these limits typically lies within the scope of sensible medical judgment. As defined according to the first embodiment, the inventive vaccine comprising at least one mRNA encoding at least one antigen may be used in the prophylaxis and treatment of a disease in an elderly patient preferably exhibiting an age of at least 50 years, more preferably of at least 55 years, 60 years, 65 years, 70 years, or older. In this context, an elderly patient is preferably a mammal, more preferably a human, even more preferably a human adult, likewise more preferably an elderly (adult) human patient exhibiting an age of at least 50 years, 55 years, 60 years, 65 years, 70 years, or older. The elderly patient may be male or female.

As furthermore defined in the first embodiment of the present invention, the treatment comprises vaccination of the patient and eliciting an immune response in said patient. In this context, vaccination typically occurs via administration of the inventive vaccine. Administration may occur parenterally, orally, nasally, pulmonary, by inhalation (e.g. via an aerosol or spray), topically, rectally, buccally, vaginally, or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. Preferably, the inventive vaccine may be administered intradermally to reach APCs in the dermis. Likewise preferably, the inventive vaccine as defined herein may be administered orally in any orally acceptable dosage form including, but not limited to, capsules, tablets, aqueous suspensions or solutions. Likewise preferably, the inventive vaccine may be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the inventive vaccine may be formulated in a suitable ointment, containing the inventive vaccine and optionally further components as defined herein suspended or dissolved in one or more carriers. Pulmonary administration can also be employed, e.g., by use of an inhaler or nebulizer, and formulation with an aerosolizing agent for use as a spray.

The inventive vaccine may be used in combination with other therapies, preferably with a therapy for a disease as defined herein, or further therapies. As used herein, the term "in combination," in the context of the administration of two or more therapies to an elderly patient as defined herein, refers to the use of more than one therapy, preferably two therapies or even more. The use of the term "in combination" does not restrict the order in which therapies are administered to an elderly patient as defined herein. For example, a first therapy (e.g., a first prophylactic or therapeutic agent) can be administered at any time prior to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to an elderly patient as defined herein. In some aspects, the one or more other therapies are conventional tumour therapies, surgery, chemotherapies, immunotherapies, gene therapies, pain treatments, anti-fever medications, therapies that alleviate or assist with breathing, other (active or passive) vaccinations/immunizations, antiviral therapies, antibacterial therapies, antifungal therapies, anti-parasite therapies, anti-allergic therapies etc.

In certain aspects, the therapies are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart. In specific aspects, two or more therapies are administered within the same patient visit.

Exemplary doses for mRNAs encoding at least one antigen as defined herein may range, without being limited thereto, from about 10 ng to 1 g, 100 ng to 100 mg, 1 µg to 10 µg, or 30-300 µg mRNA per patient. Preferably, the inventive vaccine is formulated accordingly to comprise one dose, two doses, three or even more doses.

According to a specific aspect, the inventive vaccine may be administered to an elderly patient as a single dose. In certain aspects, the inventive vaccine may be administered to an elderly patient as a single dose followed by a second dose later and optionally even a third, fourth (or more) dose subsequent thereto etc. In accordance with this aspect, booster inoculations with the inventive vaccine may be administered to an elderly patient at specific time intervals, preferably as defined below, following the second (or third, fourth, etc.) inoculation. In certain aspects, such booster inoculations with the inventive vaccine may utilize an additional compound or component as defined for the inventive vaccine as defined herein. In some aspects, the administration of the same inventive vaccine and/or booster administrations may be repeated and such administrations may be separated by at least 1 day, 2 days, 3 days, 4 days, 5 days, 10 days, 15 days, 30 days, 45 days, 2 months, 75 days, e.g. 1 to 5 days, 1 to 10 days, 5 to 15 days, 10 to 20 days, 15 to 25 days, 20 to 30 days, 25 to 35 days, 30 to 50 days, 40 to 60 days, 50 to 70 days, 1 to 75 days, or 1 month, 2 months, 3 months, 4 months, 5 months, or at least 6, 7, 8, 9, 10, 11, 12 months, 18 months, 24 months, 30 months, 36 months, 1 year, 2 years, 3 years, 5 years, 10 years, 15 years, 20 years, 30 years, 40 years, 50 years, 60 years, or even more. In certain aspects, the inventive vaccine may be administered to a subject as a single dose once per year.

In particular aspects, the inventive vaccine may be administered to an elderly patient in the fall or winter, i.e., prior to or during the influenza season in each hemisphere. In one aspect, an elderly patient is administered his/her first dose early in the season, e.g., late September or early October, so that the second dose (if necessary) can be given prior to the peak of the influenza season.

In particular aspects, the inventive vaccine may be administered at least once, preferably twice or more to an elderly patient prior to a treatment of a disease as defined herein, preferably at least 1 day, 2 days, 3 days, 4 days, 5 days, 10 days, 15 days, 30 days, 45 days, 2 months, 75 days, e.g. 1 to 5 days, 1 to 10 days, 5 to 15 days, 10 to 20 days, 15 to 25 days, 20 to 30 days, 25 to 35 days, 30 to 50 days, 40 to 60 days, 50 to 70 days, 1 to 75 days, or 1 month, 2 months, 3 months, 4 months, 5 months, or at least 6, 7, 8, 9, 10, 11, or 12 months prior to a treatment of a disease as defined herein. A second or further dose may then be administered directly prior to treatment, concurrent with or subsequent to treatment. Furthermore, a disease as defined according to the first embodiment of the present invention is any disease selected from infectious diseases, preferably (viral, bacterial or protozoological) infectious diseases, autoimmune diseases, or allergies or allergic diseases or cancer or tumour diseases.

Such diseases include cancer or tumour diseases, preferably selected from melanomas, malignant melanomas, colon carcinomas, lymphomas, sarcomas, blastomas, renal carcinomas, gastrointestinal tumours, gliomas, prostate tumours, bladder cancer, rectal tumours, stomach cancer, oesophageal cancer, pancreatic cancer, liver cancer, mammary carcinomas (= breast cancer), uterine cancer, cervical cancer, acute myeloid leukaemia (AML), acute lymphoid leukaemia (ALL), chronic myeloid leukaemia (CML), chronic lymphocytic leukaemia (CLL), hepatomas, various virus-induced tumours such as, for example, papilloma virus-induced carcinomas (e.g. cervical carcinoma = cervical cancer), adenocarcinomas, herpes virus-induced tumours (e.g. Burkitt's lymphoma, EBV-induced B-cell lymphoma), heptatitis B-induced tumours (hepatocell carcinomas), HTLV-1- and HTLV-2-induced lymphomas, acoustic neuroma, lung carcinomas (= lung cancer = bronchial carcinoma), small-cell lung carcinomas, pharyngeal cancer, anal carcinoma, glioblastoma, rectal carcinoma, astrocytoma, brain tumours, retinoblastoma, basalioma, brain metastases, medulloblastomas, vaginal cancer, pancreatic cancer, testicular cancer, Hodgkin's syndrome, meningiomas, Schneeberger disease, hypophysis tumour, Mycosis fungoides, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumours, oligodendroglioma, vulval cancer, intestinal cancer, colon carcinoma, oesophageal carcinoma (= oesophageal cancer), wart involvement, tumours of the small intestine, craniopharyngeomas, ovarian carcinoma, genital tumours, ovarian cancer (= ovarian carcinoma), pancreatic carcinoma (= pancreatic cancer), endometrial carcinoma, liver metastases, penile cancer, tongue cancer, gall bladder cancer, leukaemia, plasmocytoma, lid tumour, prostate cancer (= prostate tumours), etc.

According to one further specific aspect, diseases as defined herein comprise infectious diseases, preferably (viral, bacterial or protozoological) infectious diseases. Such infectious diseases, preferably viral, bacterial or protozoological infectious diseases, are typically selected from viral infectious diseases such as influenza, preferably influenza A, influenza B, influenza C or thogotovirus, more preferably influenza A comprising e.g. haemagglutinin subtypes H1, H2, H3, H4, H5, H6, H7, H8, H9, H10, H11, H12, H13, H14 or H15, and/or neuraminidase subtypes N1, N2, N3, N4, N5, N6, N7, N8 or N9, or preferably influenza-A subtypes H1N1, H1N2, H2N2, H2N3, H3N1, H3N2, H3N3, H5N1, H5N2, H7N7 or H9N2, etc., or any further combination, malaria, severe acute respiratory syndrome (SARS), yellow fever, AIDS, Lyme borreliosis, Leishmaniasis, anthrax, meningitis, Condyloma acuminata, hollow warts, Dengue fever, three-day fever, Ebola virus, cold, early summer meningoencephalitis (FSME), shingles, hepatitis, herpes simplex type I, herpes simplex type II, Herpes zoster, human herpes virus 8 (HHV-8; Kaposi-Sarcoma-herpesvirus (KSHV)); Human Papilloma virus infection, Japanese encephalitis, Arenavirus-associated diseases (Lassa fever infection), Marburg virus, measles, foot-and-mouth disease, mononucleosis infectiosa (Pfeiffer's glandular fever), mumps, Norwalk virus infection, smallpox, polio (childhood lameness), pseudo-croup, Erythema infectiosum (fifth disease), rabies, warts, West Nile fever, chickenpox, cytomegalic virus (CMV), bacterial infectious diseases such as miscarriage (prostate inflammation), anthrax, appendicitis, borreliosis, botulism, Camphylobacter, Chlamydia trachomatis (inflammation of the urethra, conjunctivitis), cholera, diphtheria, donavanosis, epiglottitis, typhus fever, gas gangrene, gonorrhoea, rabbit fever, Heliobacter pylori, whooping cough, climatic bubo, osteomyelitis, Legionnaire's disease, leprosy, listeriosis, pneumonia, meningitis, bacterial meningitis, anthrax, otitis media, Mycoplasma hominis, neonatal sepsis (Chorioamnionitis), noma, paratyphus, plague, Reiter's syndrome, Rocky Mountain spotted fever, Salmonella paratyphus, Salmonella typhus, scarlet fever, syphilis, tetanus, tripper, tsutsugamushi disease, tuberculosis, typhus, vaginitis (colpitis), soft chancre, and infectious diseases caused by parasites, protozoa or fungi, such as amoebiasis, bilharziosis, Chagas disease, Echinococcus, fish tapeworm, fish poisoning (Ciguatera), fox tapeworm, athlete's foot, canine tapeworm, candidosis, yeast fungus spots, scabies, cutaneous Leishmaniosis, lambliasis (giardiasis), lice, malaria, microscopy, onchocercosis (river blindness), fungal diseases, bovine tapeworm, schistosomiasis, porcine tapeworm, toxoplasmosis, trichomoniasis, trypanosomiasis (sleeping sickness), visceral Leishmaniosis, nappy/diaper dermatitis or miniature tapeworm.

According to another specific aspect, diseases as defined herein comprise autoimmune diseases as defined in the following. Autoimmune diseases can be broadly divided into systemic and organ-specific or localised autoimmune disorders, depending on the principal clinico-pathologic features of each disease. Autoimmune diseases may be divided into the categories of systemic syndromes, including systemic lupus erythematosus (SLE), Sjögren's syndrome, Scleroderma, Rheumatoid Arthritis and polymyositis or local syndromes which may be endocrinologic (type I diabetes (Diabetes mellitus Type 1), Hashimoto's thyroiditis, Addison's disease etc.), dermatologic (pemphigus vulgaris), haematologic (autoimmune haemolytic anaemia), neural (multiple sclerosis) or can involve virtually any circumscribed mass of body tissue. The autoimmune diseases to be treated may be selected from the group consisting of type I autoimmune diseases or type II autoimmune diseases or type III autoimmune diseases or type IV autoimmune diseases, such as, for example, multiple sclerosis (MS), rheumatoid arthritis, diabetes, type I diabetes (Diabetes mellitus Type 1), chronic polyarthritis, Basedow's disease, autoimmune forms of chronic hepatitis, colitis ulcerosa, type I allergy diseases, type II allergy diseases, type III allergy diseases, type IV allergy diseases, fibromyalgia, hair loss, Bechterew's disease, Crohn's disease, Myasthenia gravis, neurodermitis, Polymyalgia rheumatica, progressive systemic sclerosis (PSS), Reiter's syndrome, rheumatic arthritis, psoriasis, vasculitis, etc, or type II diabetes. While the exact mode as to why the immune system induces an immune reaction against autoantigens has not been elucidated so far, there are several findings with regard to the etiology. Accordingly, the autoreaction may be due to a T cell bypass. A normal immune system requires the activation of B cells by T cells before the former can produce antibodies in large quantities. This requirement of a T cell can be by-passed in rare instances, such as infection by organisms producing super-antigens, which are capable of initiating polyclonal activation of B cells, or even of T cells, by directly binding to the ß-subunit of T cell receptors in a non-specific fashion. Another explanation deduces autoimmune diseases from a "Molecular Mimicry": an exogenous antigen may share structural similarities with certain host antigens; thus, any antibody produced against this antigen (which mimics the self-antigens) can also, in theory, bind to the host antigens and amplify the immune response. Autoimmune diseases based on molecular mimicry are known to a skilled person for various viral and bacterial antigens. The most striking form of molecular mimicry is observed in Group A beta-haemolytic streptococci, which shares antigens with human myocardium, and is responsible for the cardiac manifestations of rheumatic fever.

Additionally, according to one further specific aspect, diseases as defined herein comprise allergies or allergic diseases, i.e. diseases related to allergies. Allergy is a condition that typically involves an abnormal, acquired immunological hypersensitivity to certain foreign antigens or allergens, such as the allergy antigens as defined herein. Such allergy antigens or allergens may be selected from allergy antigens as defined herein antigens derived from different sources, e.g. from animals, plants, fungi, bacteria, etc. Allergens in this context include e.g. danders, grasses, pollens, molds, drugs, or numerous environmental triggers, etc. Allergies normally result in a local or systemic inflammatory response to these antigens or allergens and lead to immunity in the body against these allergens. Without being bound to theory, several different disease mechanisms are supposed to be involved in the development of allergies. According to a classification scheme by P. Gell and R. Coombs the word "allergy" was restricted to type I hypersensitivities, which are caused by the classical IgE mechanism. Type I hypersensitivity is characterised by excessive activation of mast cells and basophils by IgE, resulting in a systemic inflammatory response that can result in symptoms as benign as a runny nose, to life-threatening anaphylactic shock and death. Well known types of allergies include, without being limited thereto, asthma, allergic asthma (leading to swelling of the nasal mucosa), allergic conjunctivitis (leading to redness and itching of the conjunctiva), allergic rhinitis ("hay fever"), anaphylaxis, angiodema, atopy, atopic dermatitis (eczema), urticaria (hives), eosinophilia, respiratory, allergies to insect stings, skin allergies (leading to and including various rashes, such as eczema, hives (urticaria) and (contact) dermatitis), food allergies, allergies to medicine, etc.;etc. Treatment of such allergic disorders or diseases may occur preferably by desensitizing the immune reaction which triggers a specific immune response. Such a desensitizing may be carried out by administering an effective amount of the allergen or allergic antigen encoded by the nucleic acid as defined herein, preferably, when formulated as a pharmaceutical composition, to induce a slight immune reaction. The amount of the allergen or allergic antigen may then be raised step by step in subsequent administrations until the immune system of the patient to be treated tolerates a specific amount of allergen or allergic antigen.

Diseases in the context of the present invention may also include type II hypersensitivity reactions (cytotoxic, antibody-dependent), including e.g. autoimmune hemolytic anemia, thrombocytopenia, erythroblastosis fetalis, Goodpasture's syndrome, Graves' disease, Myasthenia Gravis, etc.; type III hypersensitivity reactions (immune complex disease), , including e.g. serum sickness, Arthus reaction, Systemic lupus erythematosus (SLE), etc.; type IV hypersensitivity reactions (delayed-type hypersensitivity (DTH), cell-mediated immune memory response, antibody-independent), including e.g. contact dermatitis, Mantoux test, chronic transplant rejection, multiple sclerosis, etc.; and type V hypersensitivity reactions (receptor mediated autoimmune disease), including e.g. Graves' disease, Myasthenia Gravis, etc.;

In a further preferred embodiment, the inventive vaccine may be formulated as a kit, preferably as a kit of part. Accordingly, the present invention also provides kits, particularly kits of parts, comprising the components of the inventive vaccine either alone or in combination with further ingredients as defined above, and optionally technical instructions with information on the administration and dosage of the inventive vaccine. The components of the inventive vaccine either alone or in combination with further ingredients as defined above may be contained in the kit in either one part of the kit or in different parts of the kit, e.g. each at least one mRNA encoding at least one antigen as defined above in one part of the kit, and preferably further components admixed to the each at least one mRNA encoding at least one antigen or separately in a further part of the kit. Such kits, preferably kits of parts, may be applied, e.g., for any of the above mentioned applications or uses.

In the present invention, if not otherwise indicated, different features of alternatives and embodiments may be combined with each other, where suitable. Furthermore, the term "comprising" shall not be construed as meaning "consisting of", if not specifically mentioned. However, in the context of the present invention, term "comprising" may be substituted with the term "consisting of", where suitable.

All publications, patents and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

### Figures:

The following Figures are intended to illustrate the invention further. They are not intended to limit the subject matter of the invention thereto.

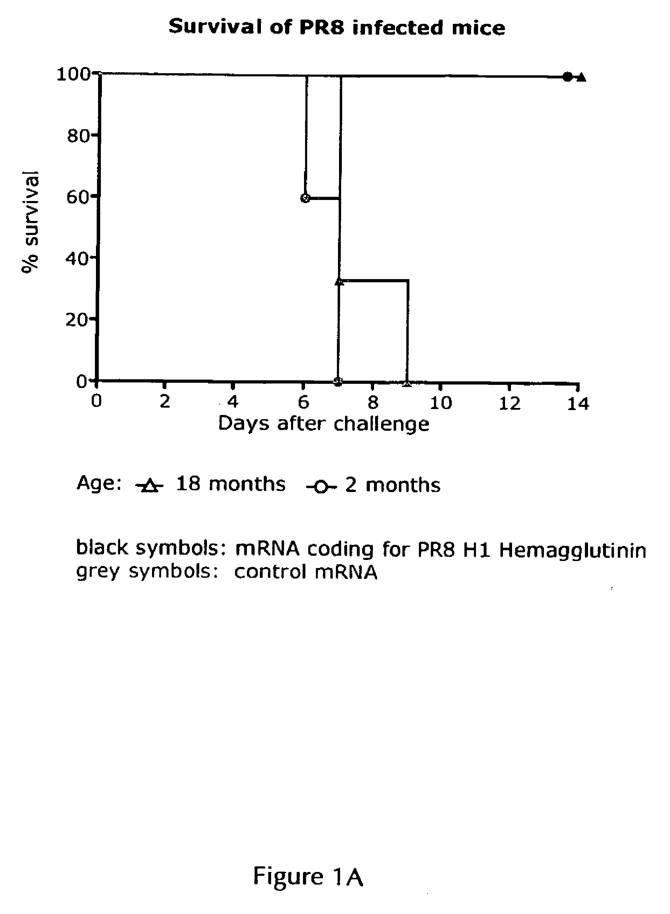

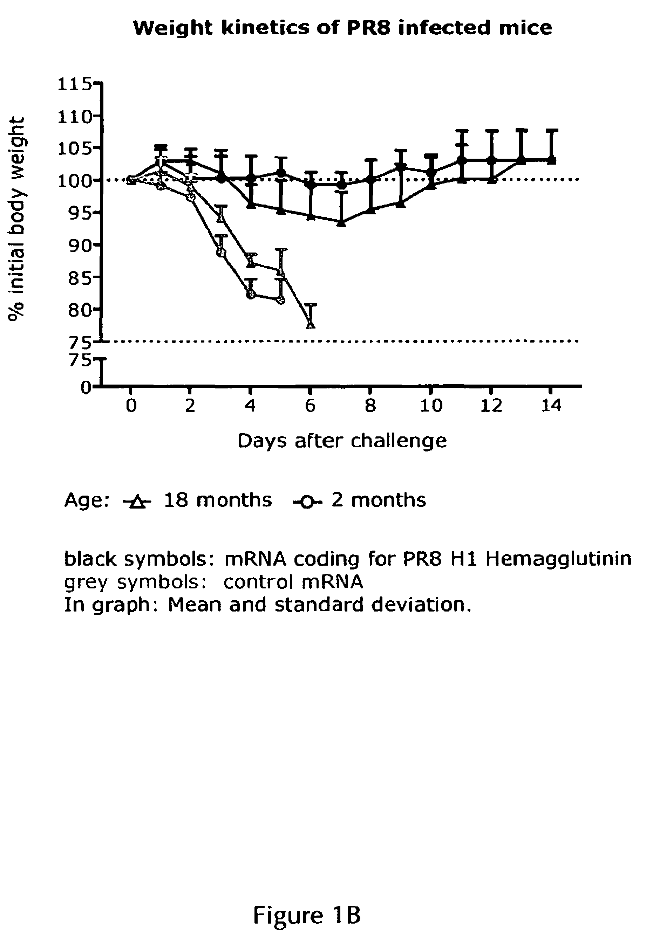

- **Figures 1 A, B:**: show the result of the vaccination of 18 months or 8 weeks old mice. The mice were vaccinated twice intradermally with 80 µg mRNA coding for PR8 H1 HA (Hemagglutinin of influenza virus A/Puerto Rico/8/1934) or with mRNA coding for *Gallus gallus* ovalbumine as a control (control mRNA). Injections were done with an interval of 7 days. 5 weeks after the last vaccination the mice were challenged with a 10fold lethal dose of PR8 virus (10 LD50). The weight of the mice was controlled over 2 weeks and the mice were killed when they have lost more than 25% of their original weight. Figure 1 A shows the overall survival of the mice. Figure 1B shows the weight of the mice.

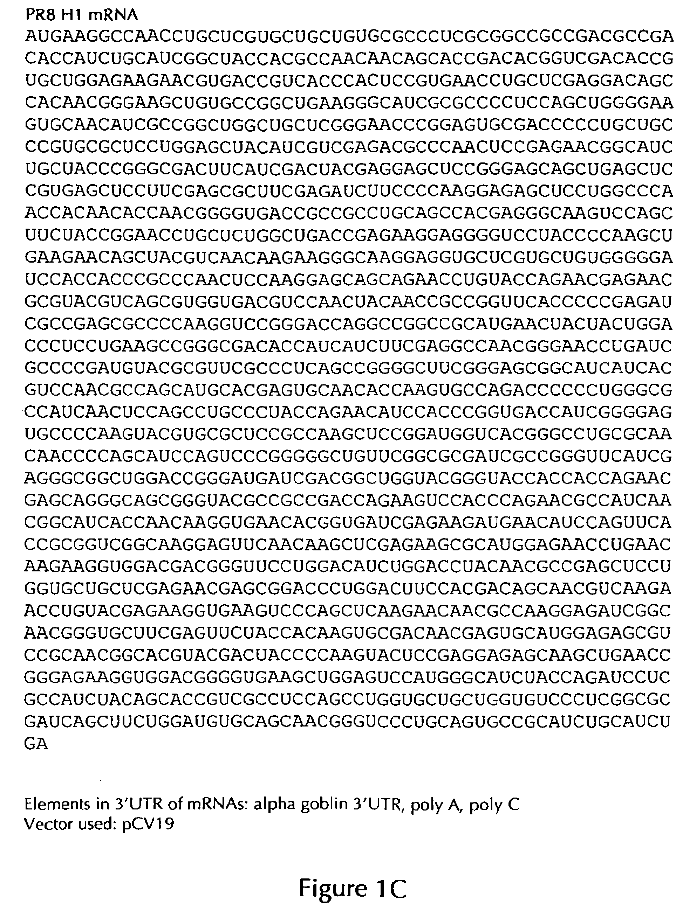

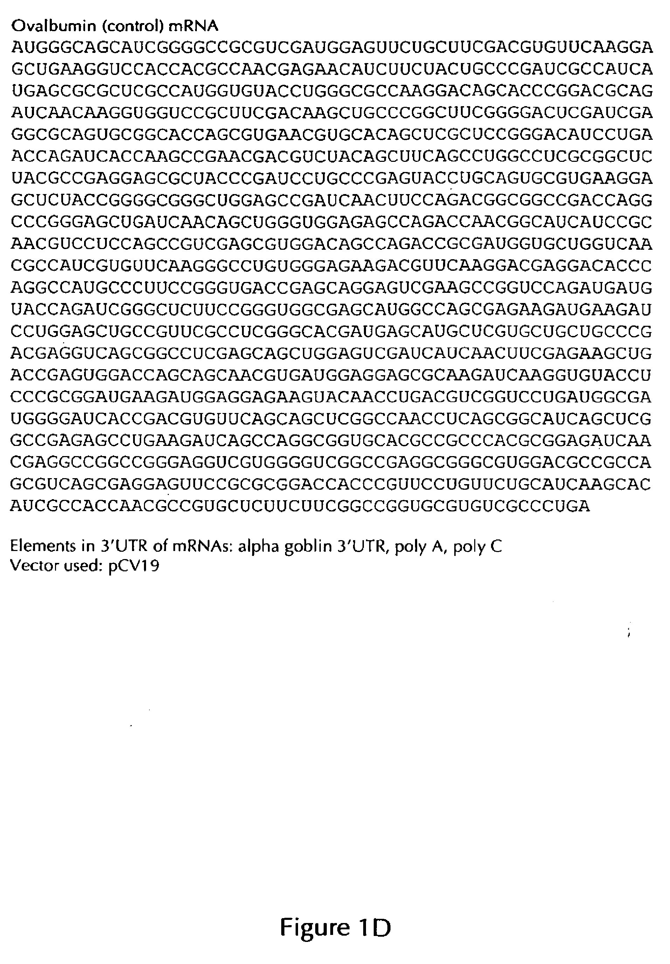

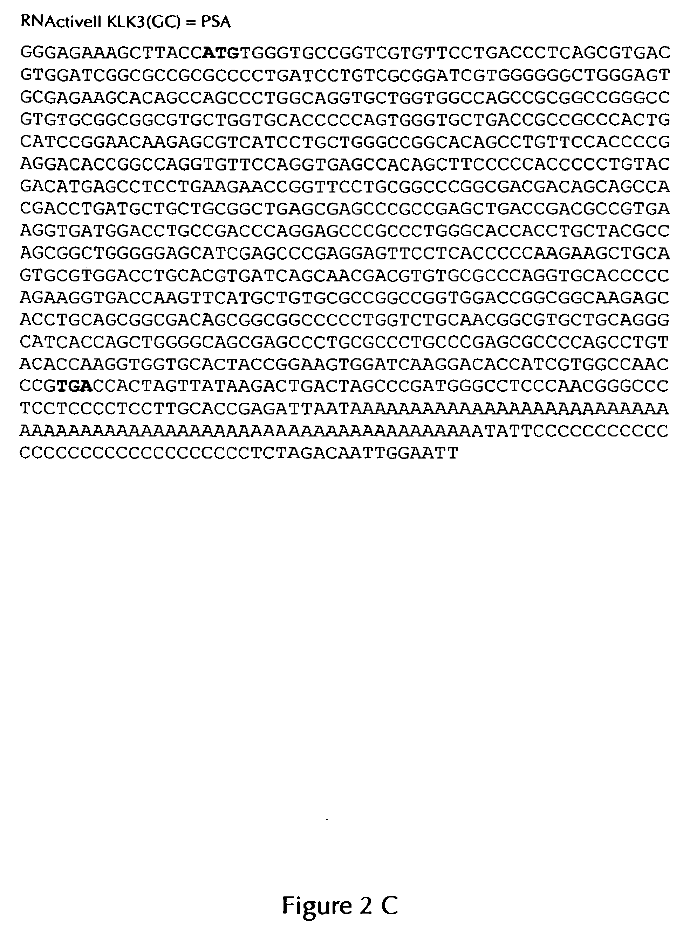

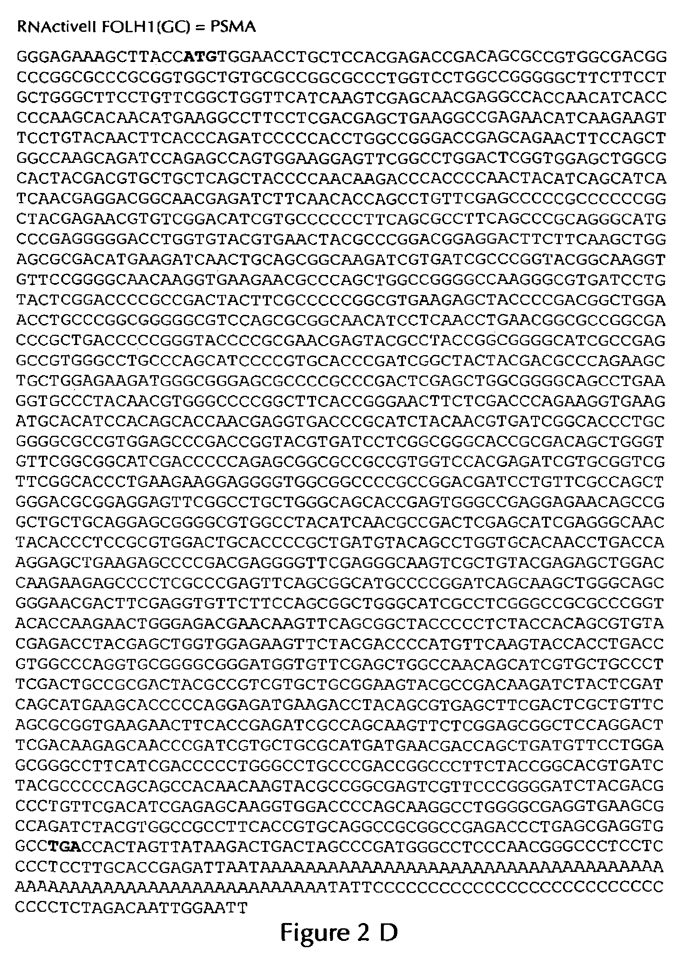

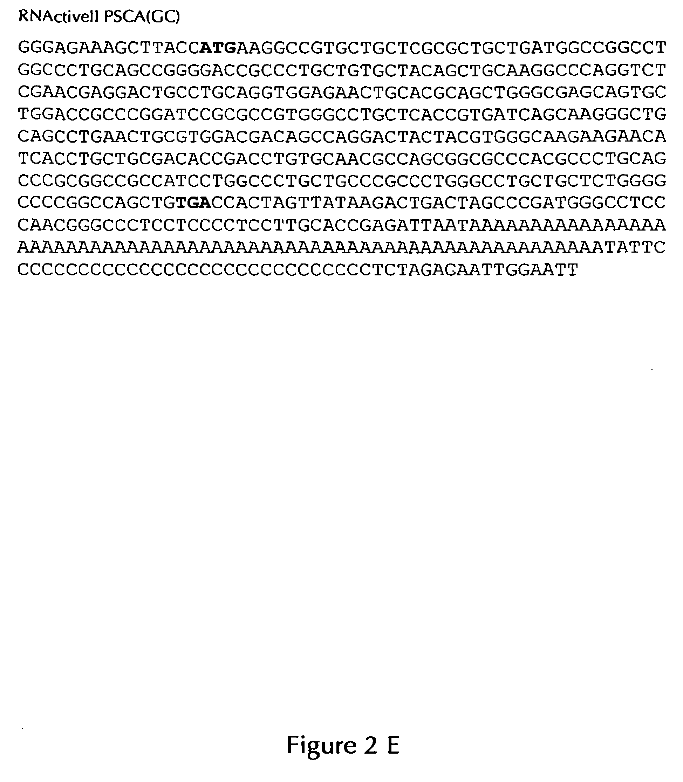

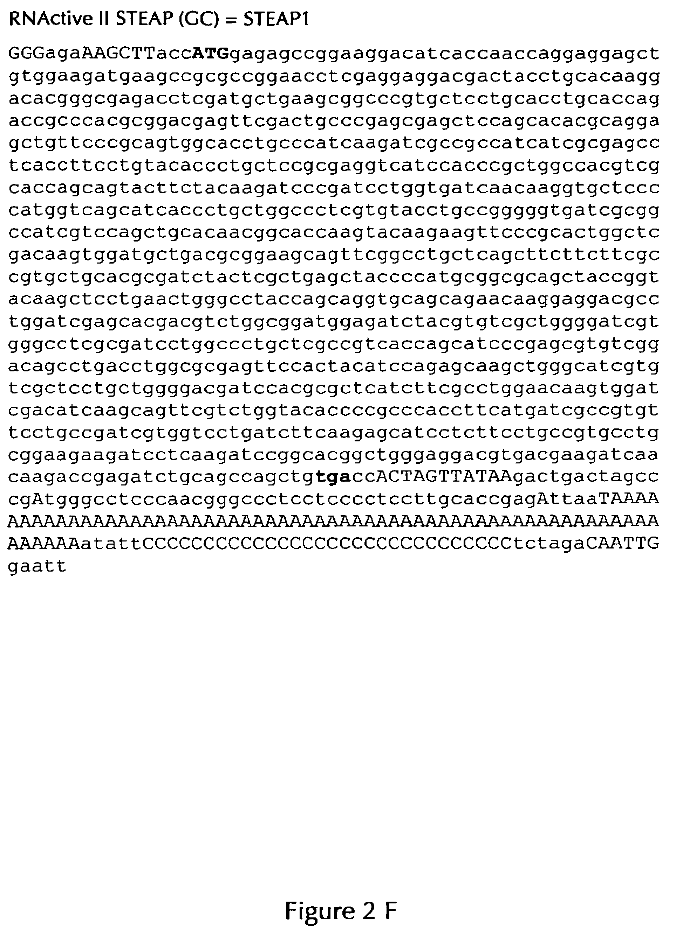

- **Figures 1 C, D:**: show the coding sequence of the mRNAs used for vaccination of 18 months or 8 weeks old mice (see Figures 1A, B) coding for PR8 H1 HA (Hemagglutinin of influenza virus A/Puerto Rico/8/1934) (SEQ ID NO: 384) (Figure 2C) or for *Gallus gallus* ovalbumine as a control (control mRNA) (SEQ ID NO: 385) (Figure 2D)

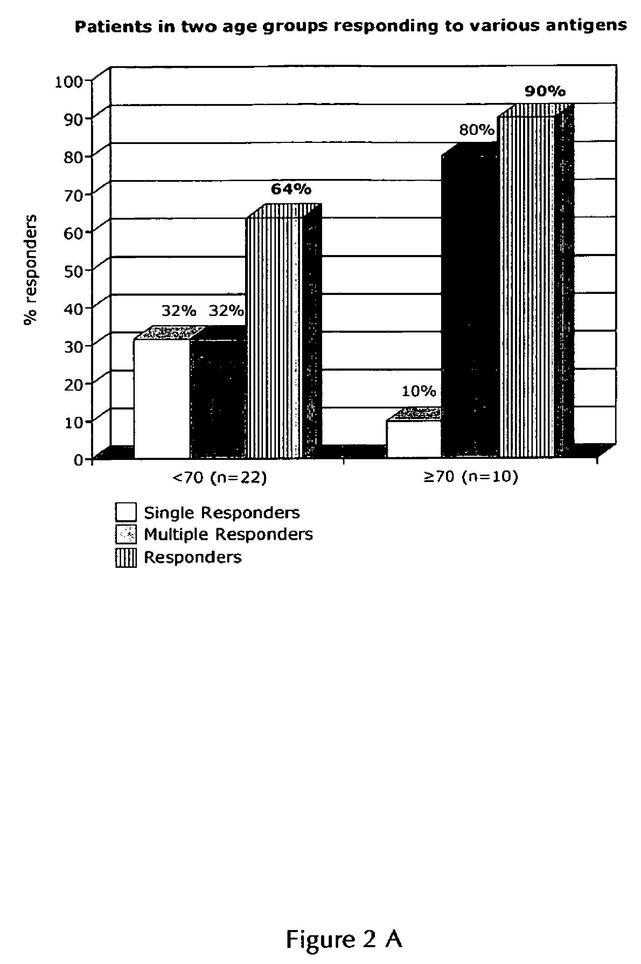

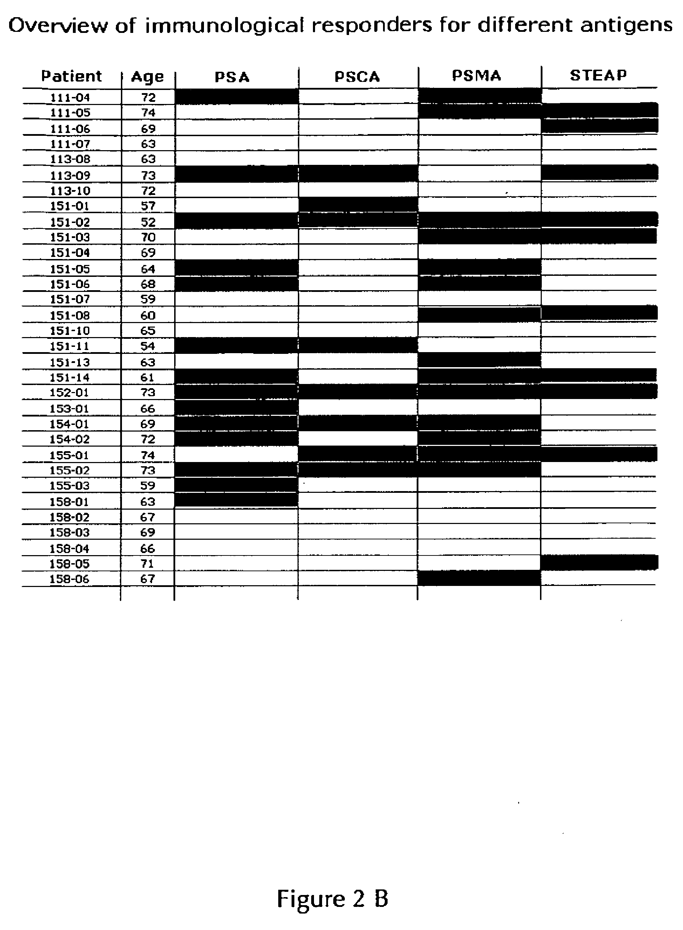

- **Figures 2 A, B:**: show the results of the vaccination of 32 patients with an age between 52 and 74 with histologically confirmed diagnosis of adenocarcinoma of the prostate. These patients were vaccinated intradermally 5 times with a total of 1280 µg mRNA per treatment coding for the tumour antigens PSA, PSCA, PSMA, and STEAP-1. Injections were done in study weeks 1, 3, 7, 15, and 23.2 weeks after the 3^{rd}, 4^{th}, and 5^{th} vaccination blood samples of the patients were collected and analysed for the presence of an antigen specific immune response against the tumour antigens PSA, PSCA, PSMA and STEAP-1. As can be seen, patients older than 70 shows at least the same efficiency in generation of an antigen specific immune response as patients younger than 70. In Figure 2B antigens against which a specific immune response was detected by ELISPOT, Tetramer staining, Intracellular Cytokine Staining (ICS) or ELISA are indicated for each patient included in the study.
- **Figures 2 C-F:**: show the coding sequence of the mRNAs used for vaccination of 32 patients with an age between 52 and 74 with histologically confirmed diagnosis (see Figures 2A, B). The mRNA sequences code for the tumour antigens PSA, PSMA, PSCA, STEAP-1 (SEQ ID NOs: 386, 387, 388 and 389).

### Examples

The following examples are intended to illustrate the invention further. They are not intended to limit the subject matter of the invention thereto.

### Example 1 - Preparation of mRNA constructs

For the present examples DNA sequences, encoding PR8 H1 HA (Haemagglutinin of A/Puerto Rico/8/1934) (SEQ ID NO: 384), and Gallus gallus ovalbumine, respectively, as a control (control mRNA) (SEQ ID NO: 385), were prepared and used for subsequent *in vitro* transcription reactions.

According to a first preparation, the DNA sequence termed PR8 H1 HA (Haemagglutinin of A/Puerto Rico/8/1934) (SEQ ID NO: 384) (see Figure 1C) was prepared by modifying the wildtype Haemagglutinin encoding DNA sequence by introducing a GC-optimized sequence for a better codon usage and stabilization. In SEQ ID NO: 384 (see Figure 1C) the sequence of the corresponding mRNA is shown. The sequence was furthermore introduced into a pCV19 vector and modified to comprise stabilizing sequences derived from alpha-globin-3'-UTR (muag (mutated alpha-globin-3'-UTR)), a stretch of 70 x adenosine at the 3'-terminal end (poly-A-tail) and a stretch of 30 x cytosine at the 3'- terminal end (poly-C-tail). The sequence of the final DNA construct was termed "PR8 H1 HA".

According to a second preparation, the DNA sequence termed Gallus gallus ovalbumine, respectively, as a control (control mRNA) (SEQ ID NO: 385) (see Figure ID) was prepared by modifying the wildtype Gallus gallus ovalbumine encoding DNA sequence by introducing a GC-optimized sequence for a better codon usage and stabilization. In SEQ ID NO: 385 (see Figure 1 D) the sequence of the corresponding mRNA is shown. The sequence was furthermore introduced into a pCV19 vector and modified to comprise stabilizing sequences derived from alpha-globin-3'-UTR (muag (mutated alpha-globin-3'-UTR)), a stretch of 70 x adenosine at the 3'-terminal end (poly-A-tail) and a stretch of 30 x cytosine at the 3'- terminal end (poly-C-tail). The sequence of the final DNA construct was termed "Gallus gallus ovalbumine".

Likewise, DNA plasmids coding for the tumour antigens PSA, PSMA, PSCA, STEAP-1 were prepared. In SEQ ID NOs: 386, 387, 388 and 389, the sequence of the corresponding mRNAs are shown (see also Figures 2 C-F).

In a further step, the respective DNA plasmids prepared above were transcribed into mRNA *in vitro* using T7-Polymerase. Subsequently the obtained mRNA was purified using PureMessenger^{®} (CureVac, Tubingen, Germany).

All obtained mRNAs used herein were furthermore complexed with protamine prior to use. The RNA complexation consisted of a mixture of 50% free mRNA and 50% mRNA complexed with protamine at a weight ratio of 2:1. First, mRNA was complexed with protamine by slow addition of protamine-Ringer's lactate solution to mRNA. As soon as the complexes were stably generated, free mRNA was added, stirred shortly and the final concentration of the vaccine was adjusted with Ringer's lactate solution.

### Example 2 - Vaccination of 18 months or 8 weeks old mice

In this experiment 18 months or 8 weeks old mice were vaccinated twice intradermally with 80 µg mRNA coding for PR8 H1 HA (Hemagglutinin of A/Puerto Rico/8/1934; Figure 1 C) or with mRNA coding for Gallus gallus ovalbumine as a control (control mRNA; Figure 1 D). Injections were done with an interval of 7 days. 5 weeks after the last vaccination the mice were challenged with a 10fold lethal dose of PR8 virus (10 LD50). The weight of the mice was controlled over 2 weeks and the mice were killed when they have lost more than 25% of their original weight. The results are shown in Figures 1A and B. Figure 1A shows the overall survival of the mice. Figure 1 B shows the weight of the mice. As can be seen in Figure 1A, mice vaccinated with mRNA coding for PR8 H1 Hemagglutinin exhibited a significantly better survival (all mice survived) against influenza challenge infection with control mRNA only (all mice died about 7 days subsequent to vaccination with control mRNA encoding chicken ovalbumin, when vaccinated with 8 weeks and died about 9 days subsequent to vaccination with control mRNA, when vaccinated with 18 months).

### Example 3 - Vaccination of human prostate carcinoma patients

In this experiment 32 patients with an age between 52 and 74 with histologically confirmed diagnosis of adenocarcinoma of the prostate were vaccinated intradermally 5 times with a total of 1280 µg mRNA per treatment coding for the tumour antigens PSA, PSCA, PSMA, STEAP-1. Injections were done in study weeks 1, 3, 7, 15, and 23. 22 weeks after the 3^{rd}, 4^{th}, and 5^{th} vaccination blood samples of the patients were collected and analysed for the presence of an antigen specific immune response against the tumour antigens PSA, PSCA, PSMA and STEAP-1. The results are shown in Figure 2A and 2B. As can be seen in Figure 2A, patients older than 70 show at least the same efficiency in generation of an antigen specific immune response as patients younger than 70. In Figure 2B antigens against which a specific immune response was detected by ELISPOT, Tetramer staining, Intracellular Cytokine Staining (ICS) or ELISA are indicated for each patient included in the study.

### Items

1. Vaccine comprising at least one mRNA encoding at least one antigen for use in the prophylaxis and/or treatment of a disease in an elderly patient exhibiting an age of at least 50 years, wherein the treatment comprises vaccination of the patient and eliciting an immune response in said patient.
2. Vaccine for use according to item 1, wherein eliciting an immune response in a patient comprises eliciting a Th1 immune response.
3. Vaccine for use according to any of items 1 or 2, wherein the elderly patient is male or female and/or exhibits an age of at least 55 years, 60 years, 65 years, 70 years, or older.
4. Vaccine for use according to any of items 1 to 3, wherein the disease is selected from infectious diseases, viral, bacterial or protozoological infectious diseases, auto-immune diseases, allergies or allergic diseases or cancer or tumour diseases,.
5. Vaccine for use according to any of items 1 to 4, wherein the antigen is selected from protein and peptide antigens, tumour antigens, self-antigens or auto-antigens, auto-immune self-antigens, pathogenic antigens, viral antigens, bacterial antigens, fungal antigens, protozoological antigens, animal antigens, allergy antigens.
6. Vaccine for use according to any of items 1 to 5, wherein the vaccine is to be administered parenterally, orally, nasally, pulmonary, by inhalation, topically, rectally, buccally, vaginally, or via an implanted reservoir.
7. Vaccine for use according to any of items 1 to 6, wherein the at least one mRNA encoding at least one antigen is to be administered in its naked form or is associated with or complexed with a cationic or polycationic compound.
8. Vaccine for use according to any of items 1 to 6, wherein the at least one mRNA encoding at least one antigen is complexed with a polymeric carrier formed by disulfide-crosslinked cationic components selected from
   an oligopeptide having following sum formula (I):

   {(Arg)ₗ;(Lys)ₘ;(His)ₙ;(Orn)ₒ;(Xaa)ₓ}; (formula (I)

   wherein 1 + m + n +o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide of formula (V); and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide of formula (I),
   or from a disulfide-crosslinked cationic component comprising as a repeat unit an oligopeptide having following subformula (Ia):

   {(Arg)ₗ;(Lys)ₘ;(His)ₙ;(Orn)ₒ;(Xaa')ₓ(Cys)_{y}}; formula (Ia)

   wherein (Arg)ₗ;(Lys)ₘ;(His)ₙ;(Orn)ₒ; and x is preferably are as defined above for formula (I), Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20,21-30,31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide.
   or from a disulfide-crosslinked cationic component comprising as a repeat unit an oligopeptide having following sufoformula (Ib):

   Cys¹{(Arg)ₗ(Lys)ₘ;(His)ₙ;(Orn)ₒ;(Xaa)ₓ}Cys²; (formula (Ib))

   wherein component {(Arg)ₗ;(Lys)ₘ;(His)ₙ;(Orn)ₒ;(Xaa)ₓ} (formula (I)) within formula (Ib) is as defined herein and forms a core of subformula (Ib), and wherein Cys¹ and Cys² are Cysteines proximal to, or terminal to (Arg)ₗ;(Lys)ₘ;(His)ₙ;(Orn)ₒ;(Xaa)ₓ.
9. Vaccine for use according to any of items 1 to 6, wherein the at least one mRNA encoding at least one antigen is complexed with a polymeric carrier according to generic formula (VI):

   L-P¹-S-[S-P²-S]ₙ-S-P³-L

   wherein,
   - P¹ and P³: are different or identical to each other and represent a linear or branched hydrophilic polymer chain, each P¹ and P³ exhibiting at least one -SH-moiety, capable to form a disulfide linkage upon condensation with component P², the linear or branched hydrophilic polymer chain selected independent from each other from polyethylene glycol (PEG), poly-*N*-(2-hydroxypropyl)methacrylamide, poly-2(methacryloyloxy)ethyl phosphorylcholines, poly(hydroxyalkyl Lasparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa,
   - P²: is a cationic or polycationic peptide or protein, having a length of about 3 to about 100 amino acids, or is a cationic or polycationic polymer, having a molecular weight of about 0.5 kDa to about 30 kDa, each P² exhibiting at least two -SH-moieties, capable to form a disulfide linkage upon condensation with further components P² or component(s) P¹ and/or P³;
   - -S-S-: is a (reversible) disulfide bond;
   - L: is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide (CPP), TAT, KALA, a ligand of a receptor, cytokines, hormones, growth factors, small molecules, carbohydrates, mannose, galactose, synthetic ligands, small molecule agonists, inhibitors or antagonists of receptors, or RGD peptidomimetic analogues; and
   - n: is an integer, selected from a range of about 1 to 50, preferably in a range of about 1, 2, 3, 4, or 5 to 10 , more preferably in a range of about 1, 2, 3, or 4 to 9.
10. Vaccine for use according to item 9, wherein the at least one mRNA encoding at least one antigen is complexed with a polymeric carrier molecule according to formula (VIa)

   L-P¹-S-{[S-P²-S]ₐ[S-(AA)ₓ-S]_{b}}-S-P³-L,

   wherein S, L, P¹, P² and P³ are preferably as defined above for formula (VI),
   a and b are integers, wherein a + b = n and n is an integer as defined above for formula (VI),
   x is an integer selected from a range of about 1 to 100, and
   (AA) is selected from an aromatic, a hydrophilic, a lipophilic, or a weak basic amino acid or peptide, or is a signal peptide or signal sequence, a localization signal or sequence, a nuclear localization signal or sequence (NLS), an antibody, a cell penetrating peptide (CPP), or is selected from therapeutically active proteins or peptides, from antigens, tumour antigens, pathogenic antigens, animal antigens, viral antigens, protozoan antigens, bacterial antigens, allergic antigens, autoimmune antigens, from allergens, from antibodies, from immunostimulatory proteins or peptides, or from antigen-specific T-cell receptors.
11. Vaccine for use according to any of items 1 to 10, wherein the vaccine is formulated to comprise a) an (adjuvant) component, comprising or consisting of at least one (m)RNA, complexed with a cationic or polycationic compound and/or with a polymeric carrier as defined according to any of items 7 or 8, and b) at least one free mRNA encoding an antigen, as defined according to any of items 1 and 5.
12. Vaccine for use according to item 11, wherein the (m)RNA is an mRNA as defined according to any of items 1 and 5, an immunostimulatory nucleic acid, a CpG nucleic acid, a CpG-RNA, a CpG-DNA, or an immunostimulatory RNA (isRNA).
13. Vaccine for use according to any of item 1 to 12, wherein the vaccine furthermore comprises a pharmaceutically acceptable carrier and/or vehicle.
14. Vaccine for use according to any of items 1 to 13, wherein the vaccine furthermore comprises at least one adjuvant, an auxiliary substance selected from lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent.
15. Kit, comprising a vaccine as defined according to any of items 1 to 15, wherein each mRNA encoding at least one antigen is provided in a different part of the kit.

## Claims

1. Vaccine comprising at least one mRNA encoding at least one antigen for use in the prophylaxis of an infectious disease in an elderly patient exhibiting an age of at least 50 years, wherein
• the treatment comprises vaccination of the patient and eliciting an immune response in said patient;
• the G/C content of the coding region of the at least one mRNA is increased by at least 15% compared to the G/C content of the coding region of the respective wild type coding sequence; and
• the at least one mRNA is associated with a cationic compound.

2. Vaccine for use in the prophylaxis of an infectious disease according to claim 1, wherein eliciting an immune response in a patient comprises eliciting a Th1 immune response.

3. Vaccine for use in the prophylaxis of an infectious disease according to any of claims 1 or 2, wherein the elderly patient is male or female and/or exhibits an age of at least 55 years, 60 years, 65 years, 70 years, or older.

4. Vaccine for use in the prophylaxis of an infectious disease according to any of claims 1 to 3, wherein the infectious disease is selected from viral infectious diseases, bacterial infectious diseases or protozoological infectious diseases.

5. Vaccine for use in the prophylaxis of an infectious disease according to any of claims 1 to 4, wherein the antigen is selected from protein and peptide antigens, pathogenic antigens, viral antigens, bacterial antigens, fungal antigens, protozoological antigens, and animal antigens.

6. Vaccine for use in the prophylaxis of an infectious disease according to any of claims 1 to 5, wherein the cationic compound is selected from the group consisting of cationic proteins or peptides, cationic polysaccharides, cationic polymers, and cationic lipids.

7. Vaccine for use in the prophylaxis of an infectious disease according to any of claims 1 to 6, wherein the cationic compound is a cationic lipid.


SEQUENCE LISTING

<110>  CureVac AG

<120>  Vaccination in elderly patients

<130>  CU01P100WO1EPT1

<160>  389

<170>  PatentIn version 3.5

<210>  1
<211>  9
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  1

Cys Arg Arg Arg Arg Arg Arg Arg Cys
1               5

<210>  2
<211>  10
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  2

Cys Arg Arg Arg Arg Arg Arg Arg Arg Cys
1               5                   10

<210>  3
<211>  11
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  3

Cys Arg Arg Arg Arg Arg Arg Arg Arg Arg Cys
1               5                   10

<210>  4
<211>  12
<212>  PRT
<213>  Artificial Sequence

<220>

<223>  Description of Sequence: cationic or polycationic peptide of
      formula (Ib): Cys {(Arg)l;(Lys)m;(His)n;(Orn)o;(Xaa)x} Cys

<400>  4

Cys Arg Arg Arg Arg Arg Arg Arg Arg Arg Arg Cys
1               5                   10


<210>  5
<211>  13
<212>  PRT
<213>  Artificial Sequence

<220>
<223>  Description of Sequence: cationic or polycationic peptide of
      formula (Ib): Cys {(Arg)l;(Lys)m;(His)n;(Orn)o;(Xaa)x} Cys

<400>  5

Cys Arg Arg Arg Arg Arg Arg Arg Arg Arg Arg Arg Cys
1               5                   10


<210>  6
<211>  14
<212>  PRT
<213>  Artificial Sequence

<220>
<223>  Description of Sequence: cationic or polycationic peptide of
      formula (Ib): Cys {(Arg)l;(Lys)m;(His)n;(Orn)o;(Xaa)x} Cys

<400>  6

Cys Arg Arg Arg Arg Arg Arg Arg Arg Arg Arg Arg Arg Cys
1               5                   10


<210>  7
<211>  15
<212>  PRT
<213>  Artificial Sequence

<220>
<223>  Description of Sequence: cationic or polycationic peptide of
      formula (Ib): Cys {(Arg)l;(Lys)m;(His)n;(Orn)o;(Xaa)x} Cys

<400>  7

Cys Arg Arg Arg Arg Arg Arg Arg Arg Arg Arg Arg Arg Arg Cys
1               5                   10                  15


<210>  8
<211>  16
<212>  PRT
<213>  Artificial Sequence

<220>
<223>  Description of Sequence: cationic or polycationic peptide of
      formula (Ib): Cys {(Arg)l;(Lys)m;(His)n;(Orn)o;(Xaa)x} Cys

<400>  8

Cys Arg Arg Arg Arg Arg Arg Arg Arg Arg Arg Arg Arg Arg Arg Cys
1               5                   10                  15

<210>  9
<211>  17
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  9

Cys Arg Arg Arg Arg Arg Arg Arg Arg Arg Arg Arg Arg Arg Arg Arg
1               5                   10                  15

Cys

<210>  10
<211>  18
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  10

Cys Arg Arg Arg Arg Arg Arg Arg Arg Arg Arg Arg Arg Arg Arg Arg
1               5                   10                  15

Arg Cys

<210>  11
<211>  19
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  11

Cys Arg Arg Arg Arg Arg Arg Arg Arg Arg Arg Arg Arg Arg Arg Arg
1               5                   10                  15

Arg Arg Cys

<210>  12
<211>  20
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  12

Cys Arg Arg Arg Arg Arg Arg Arg Arg Arg Arg Arg Arg Arg Arg Arg
1               5                   10                  15


Arg Arg Arg Cys
            20


<210>  13
<211>  21
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  13

Cys Arg Arg Arg Arg Arg Arg Arg Arg Arg Arg Arg Arg Arg Arg Arg
1               5                   10                  15


Arg Arg Arg Arg Cys
            20


<210>  14
<211>  22
<212>  PRT
<213>  Artificial Sequence

<220>
<223>  Description of Sequence: cationic or polycationic peptide of
       formula (Ib): Cys {(Arg)l;(Lys)m;(His)n;(Orn)o;(Xaa)x} Cys

<400>  14

Cys Arg Arg Arg Arg Arg Arg Arg Arg Arg Arg Arg Arg Arg Arg Arg
1               5                   10                  15


Arg Arg Arg Arg Arg Cys
            20


<210>  15

<400>  15
000

<210>  16

<400>  16
000

<210>  17

<400>  17
000

<210>  18

<400>  18
000

<210>  19

<400>  19
000

<210>  20

<400>  20
000

<210>  21

<400>  21
000

<210>  22

<400>  22
000

<210>  23
<211>  4
<212>  PRT
<213>  Artificial Sequence

<220>
<223>  Exemplary aromatic amino acid component (AA)

<400>  23

Trp Tyr Trp Tyr
1

<210>  24
<211>  4
<212>  PRT
<213>  Artificial Sequence

<220>
<223>  Exemplary aromatic amino acid component (AA)

<400>  24

Tyr Trp Tyr Trp
1

<210>  25

<211>  4
<212>  PRT
<213>  Artificial Sequence

<220>
<223>  Exemplary aromatic amino acid component (AA)

<400>  25

Trp Trp Trp Trp
1


<210>  26

<400>  26
000

<210>  27

<400>  27
000

<210>  28

<400>  28
000

<210>  29

<400>  29
000

<210>  30

<400>  30
000

<210>  31

<400>  31
000

<210>  32
<211>  4
<212>  PRT
<213>  Artificial Sequence

<220>
<223>  Exemplary aromatic amino acid component (AA)

<400>  32

Phe Tyr Phe Tyr
1


<210>  33
<211>  4
<212>  PRT
<213>  Artificial Sequence

<220>

<223>  Exemplary aromatic amino acid component (AA)

<400>  33

Tyr Phe Tyr Phe
1



<210>  34
<211>  4
<212>  PRT
<213>  Artificial Sequence

<220>
<223>  Exemplary aromatic amino acid component (AA)

<400>  34

Phe Phe Phe Phe
1



<210>  35

<400>  35
000

<210>  36

<400>  36
000

<210>  37

<400>  37
000

<210>  38

<400>  38
000

<210>  39

<400>  39
000

<210>  40
<211>  4
<212>  PRT
<213>  Artificial Sequence

<220>
<223>  Exemplary aromatic amino acid component (AA)

<400>  40

Phe Trp Phe Trp
1



<210>  41
<211>  4

<212>  PRT
<213>  Artificial Sequence

<220>
<223>  Exemplary aromatic amino acid component (AA)

<400>  41

Trp Phe Trp Phe
1


<210>  42
<211>  4
<212>  PRT
<213>  Artificial Sequence

<220>
<223>  Exemplary aromatic amino acid component (AA)

<400>  42

Tyr Tyr Tyr Tyr
1


<210>  43

<400>  43
000

<210>  44

<400>  44
000

<210>  45
<211>  4
<212>  PRT
<213>  Artificial Sequence

<220>
<223>  Exemplary aromatic amino acid component (AA)

<400>  45

Cys Trp Tyr Cys
1


<210>  46
<211>  4
<212>  PRT
<213>  Artificial Sequence

<220>
<223>  Exemplary aromatic amino acid component (AA)

<400>  46

Cys Tyr Trp Cys
1

<210>  47
<211>  4
<212>  PRT
<213>  Artificial Sequence

<220>
<223>  Exemplary aromatic amino acid component (AA)

<400>  47

Cys Trp Trp Cys
1


<210>  48
<211>  4
<212>  PRT
<213>  Artificial Sequence

<220>
<223>  Exemplary aromatic amino acid component (AA)

<400>  48

Cys Tyr Tyr Cys
1


<210>  49
<211>  5
<212>  PRT
<213>  Artificial Sequence

<220>
<223>  Exemplary aromatic amino acid component (AA)

<400>  49

Cys Trp Tyr Trp Cys
1               5


<210>  50
<211>  5
<212>  PRT
<213>  Artificial Sequence

<220>
<223>  Exemplary aromatic amino acid component (AA)

<400>  50

Cys Tyr Trp Tyr Cys
1               5


<210>  51
<211>  5
<212>  PRT
<213>  Artificial Sequence

<220>

<223>  Exemplary aromatic amino acid component (AA)

<400>  51

Cys Trp Trp Trp Cys
1               5


<210>  52
<211>  5
<212>  PRT
<213>  Artificial Sequence

<220>
<223>  Exemplary aromatic amino acid component (AA)

<400>  52

Cys Tyr Tyr Tyr Cys
1               5


<210>  53
<211>  6
<212>  PRT
<213>  Artificial Sequence

<220>
<223>  Exemplary aromatic amino acid component (AA)

<400>  53

Cys Trp Tyr Trp Tyr Cys
1               5


<210>  54
<211>  6
<212>  PRT
<213>  Artificial Sequence

<220>
<223>  Exemplary aromatic amino acid component (AA)

<400>  54

Cys Tyr Trp Tyr Trp Cys
1               5


<210>  55
<211>  6
<212>  PRT
<213>  Artificial Sequence

<220>
<223>  Exemplary aromatic amino acid component (AA)

<400>  55

Cys Trp Trp Trp Trp Cys
1               5

<210>  56
<211>  6
<212>  PRT
<213>  Artificial Sequence

<220>
<223>  Exemplary aromatic amino acid component (AA)

<400>  56

Cys Tyr Tyr Tyr Tyr Cys
1               5


<210>  57

<400>  57
000

<210>  58
<211>  4
<212>  PRT
<213>  Artificial Sequence

<220>
<223>  Exemplary aromatic amino acid component (AA)

<400>  58

Cys Phe Tyr Cys
1


<210>  59
<211>  4
<212>  PRT
<213>  Artificial Sequence

<220>
<223>  Exemplary aromatic amino acid component (AA)

<400>  59

Cys Tyr Phe Cys
1


<210>  60
<211>  4
<212>  PRT
<213>  Artificial Sequence

<220>
<223>  Exemplary aromatic amino acid component (AA)

<400>  60

Cys Phe Phe Cys
1


<210>  61

<211>  4
<212>  PRT
<213>  Artificial Sequence

<220>
<223>  Exemplary aromatic amino acid component (AA)

<400>  61

Cys Tyr Tyr Cys
1


<210>  62
<211>  5
<212>  PRT
<213>  Artificial Sequence

<220>
<223>  Exemplary aromatic amino acid component (AA)

<400>  62

Cys Phe Tyr Phe Cys
1               5


<210>  63
<211>  5
<212>  PRT
<213>  Artificial Sequence

<220>
<223>  Exemplary aromatic amino acid component (AA)

<400>  63

Cys Tyr Phe Tyr Cys
1               5


<210>  64
<211>  5
<212>  PRT
<213>  Artificial Sequence

<220>
<223>  Exemplary aromatic amino acid component (AA)

<400>  64

Cys Phe Phe Phe Cys
1               5


<210>  65
<211>  5
<212>  PRT
<213>  Artificial Sequence

<220>
<223>  Exemplary aromatic amino acid component (AA)

<400>  65

Cys Tyr Tyr Tyr Cys
1               5


<210>  66
<211>  6
<212>  PRT
<213>  Artificial Sequence

<220>
<223>  Exemplary aromatic amino acid component (AA)

<400>  66

Cys Phe Tyr Phe Tyr Cys
1               5


<210>  67
<211>  5
<212>  PRT
<213>  Artificial Sequence

<220>
<223>  Exemplary aromatic amino acid component (AA)

<400>  67

Cys Tyr Phe Tyr Phe
1               5


<210>  68
<211>  6
<212>  PRT
<213>  Artificial Sequence

<220>
<223>  Exemplary aromatic amino acid component (AA)

<400>  68

Cys Phe Phe Phe Phe Cys
1               5


<210>  69
<211>  4
<212>  PRT
<213>  Artificial Sequence

<220>
<223>  Exemplary aromatic amino acid component (AA)

<400>  69

Cys Phe Trp Cys
1


<210>  70

<211>  4
<212>  PRT
<213>  Artificial Sequence

<220>
<223>  Exemplary aromatic amino acid component (AA)

<400>  70

Cys Trp Phe Cys
1


<210>  71
<211>  4
<212>  PRT
<213>  Artificial Sequence

<220>
<223>  Exemplary aromatic amino acid component (AA)

<400>  71

Cys Phe Phe Cys
1


<210>  72
<211>  5
<212>  PRT
<213>  Artificial Sequence

<220>
<223>  Exemplary aromatic amino acid component (AA)

<400>  72

Cys Phe Trp Phe Cys
1               5


<210>  73
<211>  5
<212>  PRT
<213>  Artificial Sequence

<220>
<223>  Exemplary aromatic amino acid component (AA)

<400>  73

Cys Trp Phe Trp Cys
1               5


<210>  74
<211>  6
<212>  PRT
<213>  Artificial Sequence

<220>
<223>  Exemplary aromatic amino acid component (AA)

<400>  74

Cys Phe Trp Phe Trp Cys
1               5


<210>  75
<211>  6
<212>  PRT
<213>  Artificial Sequence

<220>
<223>  Exemplary aromatic amino acid component (AA)

<400>  75

Cys Trp Phe Trp Phe Cys
1               5


<210>  76

<400>  76
000

<210>  77

<400>  77
000

<210>  78

<400>  78
000

<210>  79

<400>  79
000

<210>  80

<400>  80
000

<210>  81

<400>  81
000

<210>  82

<400>  82
000

<210>  83

<400>  83
000

<210>  84
<211>  4
<212>  PRT

<213>  Artificial Sequence

<220>
<223>  Exemplary hydrophilic (and preferably non charged polar) amino
       acid component (AA)

<400>  84

Ser Thr Ser Thr
1


<210>  85
<211>  4
<212>  PRT
<213>  Artificial Sequence

<220>
<223>  Exemplary hydrophilic (and preferably non charged polar) amino
       acid component (AA)

<400>  85

Thr Ser Thr Ser
1


<210>  86
<211>  4
<212>  PRT
<213>  Artificial Sequence

<220>
<223>  Exemplary hydrophilic (and preferably non charged polar) amino
       acid component (AA)

<400>  86

Ser Ser Ser Ser
1


<210>  87
<211>  4
<212>  PRT
<213>  Artificial Sequence

<220>
<223>  Exemplary hydrophilic (and preferably non charged polar) amino
       acid component (AA)

<400>  87

Thr Thr Thr Thr
1


<210>  88

<400>  88
000

<210>  89

<400>  89
000

<210>  90

<400>  90
000

<210>  91

<400>  91
000

<210>  92

<400>  92
000

<210>  93

<400>  93
000

<210>  94

<400>  94
000

<210>  95

<400>  95
000

<210>  96
<211>  4
<212>  PRT
<213>  Artificial Sequence

<220>
<223>  Exemplary hydrophilic (and preferably non charged polar) amino
       acid component (AA)

<400>  96

Gln Asn Gln Asn
1

<210>  97
<211>  4
<212>  PRT
<213>  Artificial Sequence

<220>
<223>  Exemplary hydrophilic (and preferably non charged polar) amino
       acid component (AA)

<400>  97

Asn Gln Asn Gln
1

<210>  98
<211>  4
<212>  PRT
<213>  Artificial Sequence

<220>
<223>  Exemplary hydrophilic (and preferably non charged polar) amino
       acid component (AA)

<400>  98

Gln Gln Gln Gln
1


<210>  99
<211>  4
<212>  PRT
<213>  Artificial Sequence

<220>
<223>  Exemplary hydrophilic (and preferably non charged polar) amino
       acid component (AA)

<400>  99

Asn Asn Asn Asn
1


<210>  100

<400>  100
000

<210>  101

<400>  101
000

<210>  102

<400>  102
000

<210>  103

<400>  103
000

<210>  104

<400>  104
000

<210>  105

<400>  105
000

<210>  106

<400>  106
000

<210>  107

<400>  107
000

<210>  108
<211>  4
<212>  PRT
<213>  Artificial Sequence

<220>
<223>  Exemplary hydrophilic (and preferably non charged polar) amino
       acid component (AA)

<400>  108

Ser Asn Ser Asn
1

<210>  109
<211>  4
<212>  PRT
<213>  Artificial Sequence

<220>
<223>  Exemplary hydrophilic (and preferably non charged polar) amino
       acid component (AA)

<400>  109

Asn Ser Asn Ser
1

<210>  110
<211>  4
<212>  PRT
<213>  Artificial Sequence

<220>
<223>  Exemplary hydrophilic (and preferably non charged polar) amino
       acid component (AA)

<400>  110

Ser Ser Ser Ser
1

<210>  111
<211>  4
<212>  PRT
<213>  Artificial Sequence

<220>
<223>  Exemplary hydrophilic (and preferably non charged polar) amino
       acid component (AA)

<400>  111

Asn Asn Asn Asn
1


<210> 112

<400> 112
000

<210> 113

<400> 113
000

<210> 114
<211> 4
<212> PRT
<213> Artificial Sequence

<220>
<223> Exemplary hydrophilic (and preferably non charged polar) amino
      acid component (AA)

<400> 114

Cys Ser Thr Cys
1


<210> 115
<211> 4
<212> PRT
<213> Artificial Sequence

<220>
<223> Exemplary hydrophilic (and preferably non charged polar) amino
      acid component (AA)

<400> 115

Cys Thr Ser Cys
1


<210> 116
<211> 4
<212> PRT
<213> Artificial Sequence

<220>
<223> Exemplary hydrophilic (and preferably non charged polar) amino
      acid component (AA)

<400> 116

Cys Ser Ser Cys
1


<210> 117
<211> 4
<212> PRT

<213>  Artificial Sequence

<220>
<223>  Exemplary hydrophilic (and preferably non charged polar) amino
       acid component (AA)

<400>  117

Cys Thr Thr Cys
1


<210>  118
<211>  5
<212>  PRT
<213>  Artificial Sequence

<220>
<223>  Exemplary hydrophilic (and preferably non charged polar) amino
       acid component (AA)

<400>  118

Cys Ser Thr Ser Cys
1               5


<210>  119
<211>  5
<212>  PRT
<213>  Artificial Sequence

<220>
<223>  Exemplary hydrophilic (and preferably non charged polar) amino
       acid component (AA)

<400>  119

Cys Thr Ser Thr Cys
1               5


<210>  120
<211>  5
<212>  PRT
<213>  Artificial Sequence

<220>
<223>  Exemplary hydrophilic (and preferably non charged polar) amino
       acid component (AA)

<400>  120

Cys Ser Ser Ser Cys
1               5


<210>  121
<211>  5
<212>  PRT
<213>  Artificial Sequence

<220>

<223>  Exemplary hydrophilic (and preferably non charged polar) amino
       acid component (AA)

<400>  121

Cys Thr Thr Thr Cys
1               5


<210>  122
<211>  6
<212>  PRT
<213>  Artificial Sequence

<220>
<223>  Exemplary hydrophilic (and preferably non charged polar) amino
       acid component (AA)

<400>  122

Cys Ser Thr Ser Thr Cys
1               5


<210>  123
<211>  6
<212>  PRT
<213>  Artificial Sequence

<220>
<223>  Exemplary hydrophilic (and preferably non charged polar) amino
       acid component (AA)

<400>  123

Cys Thr Ser Thr Ser Cys
1               5


<210>  124
<211>  6
<212>  PRT
<213>  Artificial Sequence

<220>
<223>  Exemplary hydrophilic (and preferably non charged polar) amino
       acid component (AA)

<400>  124

Cys Ser Ser Ser Ser Cys
1               5


<210>  125
<211>  6
<212>  PRT
<213>  Artificial Sequence

<220>
<223>  Exemplary hydrophilic (and preferably non charged polar) amino
       acid component (AA)

<400>  125

Cys Thr Thr Thr Thr Cys
1               5


<210>  126

<400>  126
000

<210>  127

<400>  127
000

<210>  128
<211>  4
<212>  PRT
<213>  Artificial Sequence

<220>
<223>  Exemplary hydrophilic (and preferably non charged polar) amino
       acid component (AA)

<400>  128

Cys Gln Asn Cys
1


<210>  129
<211>  4
<212>  PRT
<213>  Artificial Sequence

<220>
<223>  Exemplary hydrophilic (and preferably non charged polar) amino
       acid component (AA)

<400>  129

Cys Asn Gln Cys
1


<210>  130
<211>  4
<212>  PRT
<213>  Artificial Sequence

<220>
<223>  Exemplary hydrophilic (and preferably non charged polar) amino
       acid component (AA)

<400>  130

Cys Gln Gln Cys
1


<210>  131
<211>  4

<212>  PRT
<213>  Artificial Sequence

<220>
<223>  Exemplary hydrophilic (and preferably non charged polar) amino
       acid component (AA)

<400>  131

Cys Asn Asn Cys
1


<210>  132
<211>  5
<212>  PRT
<213>  Artificial Sequence

<220>
<223>  Exemplary hydrophilic (and preferably non charged polar) amino
       acid component (AA)

<400>  132

Cys Gln Asn Gln Cys
1               5


<210>  133
<211>  5
<212>  PRT
<213>  Artificial Sequence

<220>
<223>  Exemplary hydrophilic (and preferably non charged polar) amino
       acid component (AA)

<400>  133

Cys Asn Gln Asn Cys
1               5


<210>  134
<211>  5
<212>  PRT
<213>  Artificial Sequence

<220>
<223>  Exemplary hydrophilic (and preferably non charged polar) amino
       acid component (AA)

<400>  134

Cys Gln Gln Gln Cys
1               5


<210>  135
<211>  5
<212>  PRT
<213>  Artificial Sequence

<220>
<223>  Exemplary hydrophilic (and preferably non charged polar) amino
       acid component (AA)

<400>  135

Cys Asn Asn Asn Cys
1               5


<210>  136
<211>  6
<212>  PRT
<213>  Artificial Sequence

<220>
<223>  Exemplary hydrophilic (and preferably non charged polar) amino
       acid component (AA)

<400>  136

Cys Gln Asn Gln Asn Cys
1               5


<210>  137
<211>  6
<212>  PRT
<213>  Artificial Sequence

<220>
<223>  Exemplary hydrophilic (and preferably non charged polar) amino
       acid component (AA)

<400>  137

Cys Asn Gln Asn Gln Cys
1               5


<210>  138
<211>  6
<212>  PRT
<213>  Artificial Sequence

<220>
<223>  Exemplary hydrophilic (and preferably non charged polar) amino
       acid component (AA)

<400>  138

Cys Gln Gln Gln Gln Cys
1               5


<210>  139
<211>  6
<212>  PRT
<213>  Artificial Sequence

<220>
<223>  Exemplary hydrophilic (and preferably non charged polar) amino
       acid component (AA)

<400>  139

Cys Asn Asn Asn Asn Cys
1               5


<210>  140

<400>  140
000

<210>  141

<400>  141
000

<210>  142
<211>  4
<212>  PRT
<213>  Artificial Sequence

<220>
<223>  Exemplary hydrophilic (and preferably non charged polar) amino
       acid component (AA)

<400>  142

Cys Ser Asn Cys
1


<210>  143
<211>  4
<212>  PRT
<213>  Artificial Sequence

<220>
<223>  Exemplary hydrophilic (and preferably non charged polar) amino
       acid component (AA)

<400>  143

Cys Asn Ser Cys
1


<210>  144
<211>  4
<212>  PRT
<213>  Artificial Sequence

<220>
<223>  Exemplary hydrophilic (and preferably non charged polar) amino
       acid component (AA)

<400>  144

Cys Ser Ser Cys
1


<210>  145

<211>  4
<212>  PRT
<213>  Artificial Sequence

<220>
<223>  Exemplary hydrophilic (and preferably non charged polar) amino
       acid component (AA)

<400>  145

Cys Asn Asn Cys
1


<210>  146
<211>  5
<212>  PRT
<213>  Artificial Sequence

<220>
<223>  Exemplary hydrophilic (and preferably non charged polar) amino
       acid component (AA)

<400>  146

Cys Ser Asn Ser Cys
1               5


<210>  147
<211>  5
<212>  PRT
<213>  Artificial Sequence

<220>
<223>  Exemplary hydrophilic (and preferably non charged polar) amino
       acid component (AA)

<400>  147

Cys Asn Ser Asn Cys
1               5


<210>  148
<211>  5
<212>  PRT
<213>  Artificial Sequence

<220>
<223>  Exemplary hydrophilic (and preferably non charged polar) amino
       acid component (AA)

<400>  148

Cys Ser Ser Ser Cys
1               5


<210>  149
<211>  5
<212>  PRT
<213>  Artificial Sequence

<220>
<223>  Exemplary hydrophilic (and preferably non charged polar) amino
      acid component (AA)

<400>  149

Cys Asn Asn Asn Cys
1               5


<210>  150
<211>  6
<212>  PRT
<213>  Artificial Sequence

<220>
<223>  Exemplary hydrophilic (and preferably non charged polar) amino
      acid component (AA)

<400>  150

Cys Ser Asn Ser Asn Cys
1               5


<210>  151
<211>  6
<212>  PRT
<213>  Artificial Sequence

<220>
<223>  Exemplary hydrophilic (and preferably non charged polar) amino
      acid component (AA)

<400>  151

Cys Asn Ser Asn Ser Cys
1               5


<210>  152
<211>  6
<212>  PRT
<213>  Artificial Sequence

<220>
<223>  Exemplary hydrophilic (and preferably non charged polar) amino
      acid component (AA)

<400>  152

Cys Ser Ser Ser Ser Cys
1               5


<210>  153
<211>  6
<212>  PRT
<213>  Artificial Sequence

<220>
<223>  Exemplary hydrophilic (and preferably non charged polar) amino

acid component (AA)

<400>  153

Cys Asn Asn Asn Asn Cys
1               5


<210>  154

<400>  154
000

<210>  155

<400>  155
000

<210>  156

<400>  156
000

<210>  157

<400>  157
000

<210>  158

<400>  158
000

<210>  159

<400>  159
000

<210>  160

<400>  160
000

<210>  161

<400>  161
000

<210>  162
<211>  4
<212>  PRT
<213>  Artificial Sequence

<220>
<223>  Exemplary lipophilic amino acid component (AA)

<400>  162

Leu Val Leu Val
1


<210>  163

<211>  4
<212>  PRT
<213>  Artificial Sequence

<220>
<223>  Exemplary lipophilic amino acid component (AA)

<400>  163

Val Leu Val Leu
1


<210>  164
<211>  4
<212>  PRT
<213>  Artificial Sequence

<220>
<223>  Exemplary lipophilic amino acid component (AA)

<400>  164

Leu Leu Leu Leu
1


<210>  165
<211>  4
<212>  PRT
<213>  Artificial Sequence

<220>
<223>  Exemplary lipophilic amino acid component (AA)

<400>  165

Val Val Val Val
1


<210>  166

<400>  166
000

<210>  167

<400>  167
000

<210>  168

<400>  168
000

<210>  169

<400>  169
000

<210>  170

<400>  170
000

<210>  171

<400>  171
000

<210>  172

<400>  172
000

<210>  173

<400>  173
000

<210>  174

<400>  174
000

<210>  175
<211>  4
<212>  PRT
<213>  Artificial Sequence

<220>
<223>  Exemplary lipophilic amino acid component (AA)

<400>  175

Ala Ile Ala Ile
1

<210>  176
<211>  4
<212>  PRT
<213>  Artificial Sequence

<220>
<223>  Exemplary lipophilic amino acid component (AA)

<400>  176

Ile Ile Ile Ile
1

<210>  177
<211>  4
<212>  PRT
<213>  Artificial Sequence

<220>
<223>  Exemplary lipophilic amino acid component (AA)

<400>  177

Ala Ala Ala Ala
1

<210>  178

<400>  178
000

<210>  179

<400>  179
000

<210>  180

<400>  180
000

<210>  181

<400>  181
000

<210>  182

<400>  182
000

<210>  183

<400>  183
000

<210>  184

<400>  184
000

<210>  185

<400>  185
000

<210>  186
<211>  4
<212>  PRT
<213>  Artificial Sequence

<220>
<223>  Exemplary lipophilic amino acid component (AA)

<400>  186

Met Ala Met Ala
1

<210>  187
<211>  4
<212>  PRT
<213>  Artificial Sequence

<220>
<223>  Exemplary lipophilic amino acid component (AA)

<400>  187

Ala Met Ala Met
1


<210>  188
<211>  4
<212>  PRT
<213>  Artificial Sequence

<220>
<223>  Exemplary lipophilic amino acid component (AA)

<400>  188

Met Met Met Met
1


<210>  189

<400>  189
000

<210>  190

<400>  190
000

<210>  191
<211>  4
<212>  PRT
<213>  Artificial Sequence

<220>
<223>  Exemplary lipophilic amino acid component (AA)

<400>  191

Cys Leu Val Cys
1


<210>  192
<211>  4
<212>  PRT
<213>  Artificial Sequence

<220>
<223>  Exemplary lipophilic amino acid component (AA)

<400>  192

Cys Val Leu Cys
1


<210>  193
<211>  4
<212>  PRT
<213>  Artificial Sequence

<220>
<223>  Exemplary lipophilic amino acid component (AA)

<400>  193

Cys Leu Leu Cys
1


<210>  194
<211>  4
<212>  PRT
<213>  Artificial Sequence

<220>
<223>  Exemplary lipophilic amino acid component (AA)

<400>  194

Cys Val Val Cys
1


<210>  195
<211>  5
<212>  PRT
<213>  Artificial Sequence

<220>
<223>  Exemplary lipophilic amino acid component (AA)

<400>  195

Cys Leu Val Leu Cys
1               5


<210>  196
<211>  5
<212>  PRT
<213>  Artificial Sequence

<220>
<223>  Exemplary lipophilic amino acid component (AA)

<400>  196

Cys Val Leu Val Cys
1               5


<210>  197
<211>  5
<212>  PRT
<213>  Artificial Sequence

<220>
<223>  Exemplary lipophilic amino acid component (AA)

<400>  197

Cys Leu Leu Leu Cys

1               5


<210>  198
<211>  5
<212>  PRT
<213>  Artificial Sequence

<220>
<223>  Exemplary lipophilic amino acid component (AA)

<400>  198

Cys Val Val Val Cys
1               5


<210>  199
<211>  6
<212>  PRT
<213>  Artificial Sequence

<220>
<223>  Exemplary lipophilic amino acid component (AA)

<400>  199

Cys Leu Val Leu Val Cys
1               5


<210>  200
<211>  6
<212>  PRT
<213>  Artificial Sequence

<220>
<223>  Exemplary lipophilic amino acid component (AA)

<400>  200

Cys Val Leu Val Leu Cys
1               5


<210>  201
<211>  6
<212>  PRT
<213>  Artificial Sequence

<220>
<223>  Exemplary lipophilic amino acid component (AA)

<400>  201

Cys Leu Leu Leu Leu Cys
1               5


<210>  202
<211>  6
<212>  PRT
<213>  Artificial Sequence

<220>
<223>  Exemplary lipophilic amino acid component (AA)

<400>  202

Cys Val Val Val Val Cys
1               5


<210>  203

<400>  203
000

<210>  204

<400>  204
000

<210>  205
<211>  4
<212>  PRT
<213>  Artificial Sequence

<220>
<223>  Exemplary lipophilic amino acid component (AA)

<400>  205

Cys Ile Ala Cys
1


<210>  206
<211>  4
<212>  PRT
<213>  Artificial Sequence

<220>
<223>  Exemplary lipophilic amino acid component (AA)

<400>  206

Cys Ala Ile Cys
1


<210>  207
<211>  4
<212>  PRT
<213>  Artificial Sequence

<220>
<223>  Exemplary lipophilic amino acid component (AA)

<400>  207

Cys Ile Ile Cys
1


<210>  208

<211>  4
<212>  PRT
<213>  Artificial Sequence

<220>
<223>  Exemplary lipophilic amino acid component (AA)

<400>  208

Cys Ala Ala Cys
1


<210>  209
<211>  5
<212>  PRT
<213>  Artificial Sequence

<220>
<223>  Exemplary lipophilic amino acid component (AA)

<400>  209

Cys Ile Ala Ile Cys
1               5


<210>  210
<211>  5
<212>  PRT
<213>  Artificial Sequence

<220>
<223>  Exemplary lipophilic amino acid component (AA)

<400>  210

Cys Ala Ile Ala Cys
1               5


<210>  211
<211>  5
<212>  PRT
<213>  Artificial Sequence

<220>
<223>  Exemplary lipophilic amino acid component (AA)

<400>  211

Cys Ile Ile Ile Cys
1               5


<210>  212
<211>  5
<212>  PRT
<213>  Artificial Sequence

<220>
<223>  Exemplary lipophilic amino acid component (AA)

<400>  212

Cys Ala Ala Ala Cys
1               5


<210>  213
<211>  6
<212>  PRT
<213>  Artificial Sequence

<220>
<223>  Exemplary lipophilic amino acid component (AA)

<400>  213

Cys Ile Ala Ile Ala Cys
1               5


<210>  214
<211>  6
<212>  PRT
<213>  Artificial Sequence

<220>
<223>  Exemplary lipophilic amino acid component (AA)

<400>  214

Cys Ala Ile Ala Ile Cys
1               5


<210>  215
<211>  6
<212>  PRT
<213>  Artificial Sequence

<220>
<223>  Exemplary lipophilic amino acid component (AA)

<400>  215

Cys Ile Ile Ile Ile Cys
1               5


<210>  216
<211>  6
<212>  PRT
<213>  Artificial Sequence

<220>
<223>  Exemplary lipophilic amino acid component (AA)

<400>  216

Cys Ala Ala Ala Ala Cys
1               5


<210>  217

<400>  217
000

<210>  218
<211>  4
<212>  PRT
<213>  Artificial Sequence

<220>
<223>  Exemplary lipophilic amino acid component (AA)

<400>  218

Cys Met Ala Cys
1


<210>  219
<211>  4
<212>  PRT
<213>  Artificial Sequence

<220>
<223>  Exemplary lipophilic amino acid component (AA)

<400>  219

Cys Ala Met Cys
1


<210>  220
<211>  4
<212>  PRT
<213>  Artificial Sequence

<220>
<223>  Exemplary lipophilic amino acid component (AA)

<400>  220

Cys Met Met Cys
1


<210>  221
<211>  4
<212>  PRT
<213>  Artificial Sequence

<220>
<223>  Exemplary lipophilic amino acid component (AA)

<400>  221

Cys Ala Ala Cys
1


<210>  222
<211>  5
<212>  PRT

<213>  Artificial Sequence

<220>
<223>  Exemplary lipophilic amino acid component (AA)

<400>  222

Cys Met Ala Met Cys
1               5


<210>  223
<211>  5
<212>  PRT
<213>  Artificial Sequence

<220>
<223>  Exemplary lipophilic amino acid component (AA)

<400>  223

Cys Ala Met Ala Cys
1               5


<210>  224
<211>  5
<212>  PRT
<213>  Artificial Sequence

<220>
<223>  Exemplary lipophilic amino acid component (AA)

<400>  224

Cys Met Met Met Cys
1               5


<210>  225
<211>  5
<212>  PRT
<213>  Artificial Sequence

<220>
<223>  Exemplary lipophilic amino acid component (AA)

<400>  225

Cys Ala Ala Ala Cys
1               5


<210>  226
<211>  6
<212>  PRT
<213>  Artificial Sequence

<220>
<223>  Exemplary lipophilic amino acid component (AA)

<400>  226

Cys Met Ala Met Ala Cys
1               5


<210>  227
<211>  6
<212>  PRT
<213>  Artificial Sequence

<220>
<223>  Exemplary lipophilic amino acid component (AA)

<400>  227

Cys Ala Met Ala Met Cys
1               5


<210>  228
<211>  6
<212>  PRT
<213>  Artificial Sequence

<220>
<223>  Exemplary lipophilic amino acid component (AA)

<400>  228

Cys Met Met Met Met Cys
1               5


<210>  229
<211>  6
<212>  PRT
<213>  Artificial Sequence

<220>
<223>  Exemplary lipophilic amino acid component (AA)

<400>  229

Cys Ala Ala Ala Ala Cys
1               5


<210>  230

<400>  230
000

<210>  231

<400>  231
000

<210>  232

<400>  232
000

<210>  233

<400>  233
000

<210>  234

<400>  234
000

<210>  235

<400>  235
000

<210>  236

<400>  236
000

<210>  237

<400>  237
000

<210>  238
<211>  4
<212>  PRT
<213>  Artificial Sequence

<220>
<223>  Exemplary weak basic amino acid component (AA)

<400>  238

Asp His Asp His
1

<210>  239
<211>  4
<212>  PRT
<213>  Artificial Sequence

<220>
<223>  Exemplary weak basic amino acid component (AA)

<400>  239

His Asp His Asp
1

<210>  240
<211>  4
<212>  PRT
<213>  Artificial Sequence

<220>
<223>  Exemplary weak basic amino acid component (AA)

<400>  240

Asp Asp Asp Asp
1

<210>  241
<211>  4
<212>  PRT
<213>  Artificial Sequence

<220>
<223>  Exemplary weak basic amino acid component (AA)

<400>  241

His His His His
1


<210>  242

<400>  242
000

<210>  243

<400>  243
000

<210>  244
<211>  4
<212>  PRT
<213>  Artificial Sequence

<220>
<223>  Exemplary weak basic amino acid component (AA)

<400>  244

Cys Asp His Cys
1


<210>  245
<211>  4
<212>  PRT
<213>  Artificial Sequence

<220>
<223>  Exemplary weak basic amino acid component (AA)

<400>  245

Cys His Asp Cys
1


<210>  246
<211>  4
<212>  PRT
<213>  Artificial Sequence

<220>
<223>  Exemplary weak basic amino acid component (AA)

<400>  246

Cys Asp Asp Cys
1

<210> 247
<211> 4
<212> PRT
<213> Artificial Sequence

<220>
<223> Exemplary weak basic amino acid component (AA)

<400> 247

Cys His His Cys
1

<210> 248
<211> 5
<212> PRT
<213> Artificial Sequence

<220>
<223> Exemplary weak basic amino acid component (AA)

<400> 248

Cys Asp His Asp Cys
1               5

<210> 249
<211> 5
<212> PRT
<213> Artificial Sequence

<220>
<223> Exemplary weak basic amino acid component (AA)

<400> 249

Cys His Asp His Cys
1               5

<210> 250
<211> 5
<212> PRT
<213> Artificial Sequence

<220>
<223> Exemplary weak basic amino acid component (AA)

<400> 250

Cys Asp Asp Asp Cys
1               5

<210> 251
<211> 5

<212>  PRT
<213>  Artificial Sequence

<220>
<223>  Exemplary weak basic amino acid component (AA)

<400>  251

Cys His His His Cys
1               5


<210>  252
<211>  6
<212>  PRT
<213>  Artificial Sequence

<220>
<223>  Exemplary weak basic amino acid component (AA)

<400>  252

Cys Asp His Asp His Cys
1               5


<210>  253
<211>  6
<212>  PRT
<213>  Artificial Sequence

<220>
<223>  Exemplary weak basic amino acid component (AA)

<400>  253

Cys His Asp His Asp Cys
1               5


<210>  254
<211>  6
<212>  PRT
<213>  Artificial Sequence

<220>
<223>  Exemplary weak basic amino acid component (AA)

<400>  254

Cys Asp Asp Asp Asp Cys
1               5


<210>  255
<211>  6
<212>  PRT
<213>  Artificial Sequence

<220>
<223>  Exemplary weak basic amino acid component (AA)

<400>  255

Cys His His His His Cys
1               5



<210>  256
<211>  4
<212>  PRT
<213>  Artificial Sequence

<220>
<223>  Description of sequence: signal peptide, localization signal or
       sequence or nuclear localization signal

<400>  256

Lys Asp Glu Leu
1



<210>  257
<211>  4
<212>  PRT
<213>  Artificial Sequence

<220>
<223>  Description of sequence: signal peptide, localization signal or
       sequence or nuclear localization signal

<400>  257

Asp Asp Glu Leu
1



<210>  258
<211>  4
<212>  PRT
<213>  Artificial Sequence

<220>
<223>  Description of sequence: signal peptide, localization signal or
       sequence or nuclear localization signal

<400>  258

Asp Glu Glu Leu
1



<210>  259
<211>  4
<212>  PRT
<213>  Artificial Sequence

<220>
<223>  Description of sequence: signal peptide, localization signal or
       sequence or nuclear localization signal

<400>  259

Gln Glu Asp Leu
1

<210>  260
<211>  4
<212>  PRT
<213>  Artificial Sequence

<220>
<223>  Description of sequence: signal peptide, localization signal or
       sequence or nuclear localization signal

<400>  260

Arg Asp Glu Leu
1


<210>  261
<211>  8
<212>  PRT
<213>  Artificial Sequence

<220>
<223>  Description of sequence: signal peptide, localization signal or
       sequence or nuclear localization signal

<400>  261

Gly Gln Asn Leu Ser Thr Ser Asn
1               5


<210>  262
<211>  7
<212>  PRT
<213>  Artificial Sequence

<220>
<223>  Description of sequence: signal peptide, localization signal or
       sequence or nuclear localization signal

<400>  262

Pro Lys Lys Lys Arg Lys Val
1               5


<210>  263
<211>  7
<212>  PRT
<213>  Artificial Sequence

<220>
<223>  Description of sequence: signal peptide, localization signal or
       sequence or nuclear localization signal

<400>  263

Pro Gln Lys Lys Ile Lys Ser
1               5


<210>  264

<211>  5
<212>  PRT
<213>  Artificial Sequence

<220>
<223>  Description of sequence: signal peptide, localization signal or
       sequence or nuclear localization signal

<400>  264

Gln Pro Lys Lys Pro
1               5


<210>  265
<211>  4
<212>  PRT
<213>  Artificial Sequence

<220>
<223>  Description of sequence: signal peptide, localization signal or
       sequence or nuclear localization signal

<400>  265

Arg Lys Lys Arg
1


<210>  266
<211>  12
<212>  PRT
<213>  Artificial Sequence

<220>
<223>  Description of sequence: signal peptide, localization signal or
       sequence or nuclear localization signal

<400>  266

Arg Lys Lys Arg Arg Gln Arg Arg Arg Ala His Gln
1               5                   10


<210>  267
<211>  16
<212>  PRT
<213>  Artificial Sequence

<220>
<223>  Description of sequence: signal peptide, localization signal or
       sequence or nuclear localization signal

<400>  267

Arg Gln Ala Arg Arg Asn Arg Arg Arg Arg Trp Arg Glu Arg Gln Arg
1               5                   10                  15


<210>  268
<211>  19
<212>  PRT
<213>  Artificial Sequence

<220>
<223>  Description of sequence: signal peptide, localization signal or
       sequence or nuclear localization signal

<400>  268

Met Pro Leu Thr Arg Arg Arg Pro Ala Ala Ser Gln Ala Leu Ala Pro
1               5                   10                  15

Pro Thr Pro

<210>  269
<211>  8
<212>  PRT
<213>  Artificial Sequence

<220>
<223>  Description of sequence: signal peptide, localization signal or
       sequence or nuclear localization signal

<400>  269

Gly Ala Ala Leu Thr Ile Leu Val
1               5

<210>  270
<211>  8
<212>  PRT
<213>  Artificial Sequence

<220>
<223>  Description of sequence: signal peptide, localization signal or
       sequence or nuclear localization signal

<400>  270

Gly Ala Ala Leu Thr Leu Leu Gly
1               5

<210>  271
<211>  15
<212>  PRT
<213>  Artificial Sequence

<220>
<223>  Description of sequence: signal peptide, localization signal or
       sequence or nuclear localization signal

<400>  271

Met Asp Asp Gln Arg Asp Leu Ile Ser Asn Asn Glu Gln Leu Pro
1               5                   10                  15

<210>  272
<211>  32
<212>  PRT

<213>  Artificial Sequence

<220>
<223>  Description of sequence: signal peptide, localization signal or
       sequence or nuclear localization signal


<220>
<221>  misc_feature
<222>  (7)..(8)
<223>  Xaa can be any naturally occurring amino acid

<220>
<221>  misc_feature
<222>  (32)..(32)
<223>  Xaa can be any naturally occurring amino acid

<400>  272

Met Leu Phe Asn Leu Arg Xaa Xaa Leu Asn Asn Ala Ala Phe Arg His
1               5                   10                  15


Gly His Asn Phe Met Val Arg Asn Phe Arg Cys Gly Gln Pro Leu Xaa
            20                  25                  30


<210>  273
<211>  8
<212>  PRT
<213>  Artificial Sequence

<220>
<223>  Description of sequence: signal peptide, localization signal or
       sequence or nuclear localization signal

<400>  273

Gly Cys Val Cys Ser Ser Asn Pro
1               5


<210>  274
<211>  8
<212>  PRT
<213>  Artificial Sequence

<220>
<223>  Description of sequence: signal peptide, localization signal or
       sequence or nuclear localization signal

<400>  274

Gly Gln Thr Val Thr Thr Pro Leu
1               5


<210>  275
<211>  8
<212>  PRT
<213>  Artificial Sequence

<220>

<223>  Description of sequence: signal peptide, localization signal or
       sequence or nuclear localization signal

<400>  275

Gly Gln Glu Leu Ser Gln His Glu
1               5


<210>  276
<211>  8
<212>  PRT
<213>  Artificial Sequence

<220>
<223>  Description of sequence: signal peptide, localization signal or
       sequence or nuclear localization signal

<400>  276

Gly Asn Ser Pro Ser Tyr Asn Pro
1               5


<210>  277
<211>  8
<212>  PRT
<213>  Artificial Sequence

<220>
<223>  Description of sequence: signal peptide, localization signal or
       sequence or nuclear localization signal

<400>  277

Gly Val Ser Gly Ser Lys Gly Gln
1               5


<210>  278
<211>  8
<212>  PRT
<213>  Artificial Sequence

<220>
<223>  Description of sequence: signal peptide, localization signal or
       sequence or nuclear localization signal

<400>  278

Gly Gln Thr Ile Thr Thr Pro Leu
1               5


<210>  279
<211>  8
<212>  PRT
<213>  Artificial Sequence

<220>
<223>  Description of sequence: signal peptide, localization signal or
       sequence or nuclear localization signal

<400>  279

Gly Gln Thr Leu Thr Thr Pro Leu
1               5


<210>  280
<211>  8
<212>  PRT
<213>  Artificial Sequence

<220>
<223>  Description of sequence: signal peptide, localization signal or
       sequence or nuclear localization signal

<400>  280

Gly Gln Ile Phe Ser Arg Ser Ala
1               5


<210>  281
<211>  8
<212>  PRT
<213>  Artificial Sequence

<220>
<223>  Description of sequence: signal peptide, localization signal or
       sequence or nuclear localization signal

<400>  281

Gly Gln Ile His Gly Leu Ser Pro
1               5


<210>  282
<211>  8
<212>  PRT
<213>  Artificial Sequence

<220>
<223>  Description of sequence: signal peptide, localization signal or
       sequence or nuclear localization signal

<400>  282

Gly Ala Arg Ala Ser Val Leu Ser
1               5


<210>  283
<211>  8
<212>  PRT
<213>  Artificial Sequence

<220>
<223>  Description of sequence: signal peptide, localization signal or
       sequence or nuclear localization signal

<400>  283

Gly Cys Thr Leu Ser Ala Glu Glu

1               5


<210>  284
<211>  8
<212>  PRT
<213>  Artificial Sequence

<220>
<223>  Description of sequence: signal peptide, localization signal or
       sequence or nuclear localization signal

<400>  284

Gly Ala Gln Val Ser Ser Gln Lys
1               5


<210>  285
<211>  8
<212>  PRT
<213>  Artificial Sequence

<220>
<223>  Description of sequence: signal peptide, localization signal or
       sequence or nuclear localization signal

<400>  285

Gly Ala Gln Leu Ser Arg Asn Thr
1               5


<210>  286
<211>  8
<212>  PRT
<213>  Artificial Sequence

<220>
<223>  Description of sequence: signal peptide, localization signal or
       sequence or nuclear localization signal

<400>  286

Gly Asn Ala Ala Ala Ala Lys Lys
1               5


<210>  287
<211>  8
<212>  PRT
<213>  Artificial Sequence

<220>
<223>  Description of sequence: signal peptide, localization signal or
       sequence or nuclear localization signal

<400>  287

Gly Asn Glu Ala Ser Tyr Pro Leu
1               5

<210>  288
<211>  8
<212>  PRT
<213>  Artificial Sequence

<220>
<223>  Description of sequence: signal peptide, localization signal or
       sequence or nuclear localization signal

<400>  288

Gly Ser Ser Lys Ser Lys Pro Lys
1               5


<210>  289
<211>  20
<212>  RNA
<213>  Artificial

<220>
<223>  nucleic acid sequence according to formula (II)

<400>  289
gguuuuuuuu uuuuuuuggg                                                   20


<210>  290
<211>  20
<212>  RNA
<213>  Artificial

<220>
<223>  nucleic acid sequence according to formula (II)

<400>  290
ggggguuuuu uuuuuggggg                                                   20


<210>  291
<211>  40
<212>  RNA
<213>  Artificial

<220>
<223>  nucleic acid sequence according to formula (II)

<400>  291
ggggguuuuu uuuuuuuuuu uuuuuuuuuu uuuuuggggg                             40


<210>  292
<211>  39
<212>  RNA
<213>  Artificial

<220>
<223>  nucleic acid sequence according to formula (II)

<400>  292
gugugugugu guuuuuuuuu uuuuuuugug ugugugugu                              39

<210>  293
<211>  39
<212>  RNA
<213>  Artificial

<220>
<223>  nucleic acid sequence according to formula (II)

<400>  293
gguugguugg uuuuuuuuuu uuuuuuuggu ugguugguu                              39


<210>  294
<211>  20
<212>  RNA
<213>  Artificial

<220>
<223>  nucleic acid sequence according to formula (II)

<400>  294
gggggggggu uugggggggg                                                   20


<210>  295
<211>  20
<212>  RNA
<213>  Artificial

<220>
<223>  nucleic acid sequence according to formula (II)

<400>  295
   gggggggguu uugggggggg                                                     20


<210>  296
<211>  20
<212>  RNA
<213>  Artificial

<220>
<223>  nucleic acid sequence according to formula (II)

<400>  296
   ggggggguuu uuuggggggg                                                   20


<210>  297
<211>  20
<212>  RNA
<213>  Artificial

<220>
<223>  nucleic acid sequence according to formula (II)

<400>  297
   ggggggguuu uuuugggggg                                                   20


<210>  298
<211>  20
<212>  RNA

<213>  Artificial

<220>
<223>  nucleic acid sequence according to formula (II)

<400>  298
   ggggggtuuu uuuugggggg                                                    20


<210>  299
<211>  20
<212>  RNA
<213>  Artificial

<220>
<223>  nucleic acid sequence according to formula (II)

<400>  299
   ggggggtuuu uuuuuggggg                                                    20


<210>  300
<211>  20
<212>  RNA
<213>  Artificial

<220>
<223>  nucleic acid sequence according to formula (II)

<400>  300
   ggggggtuuu uuuuuugggg                                                    20


<210>  301
<211>  20
<212>  RNA
<213>  Artificial

<220>
<223>  nucleic acid sequence according to formula (II)

<400>  301
   gggggtuuuu uuuuuugggg                                                    20


<210>  302
<211>  20
<212>  RNA
<213>  Artificial

<220>
<223>  nucleic acid sequence according to formula (II)

<400>  302
   gggggtuuuu uuuuuuuggg                                                    20


<210>  303
<211>  20
<212>  RNA
<213>  Artificial

<220>

<223>  nucleic acid sequence according to formula (II)

<400>  303
ggggguuuuu uuuuuuuggg                                                    20


<210>  304
<211>  20
<212>  RNA
<213>  Artificial

<220>
<223>  nucleic acid sequence according to formula (II)

<400>  304
ggggguuuuu uuuuuuuugg                                                    20


<210>  305
<211>  20
<212>  RNA
<213>  Artificial

<220>
<223>  nucleic acid sequence according to formula (II)

<400>  305
gguuuuuuuu uuuuuuuugg                                                    20


<210>  306
<211>  20
<212>  RNA
<213>  Artificial

<220>
<223>  nucleic acid sequence according to formula (II)

<400>  306
guuuuuuuuu uuuuuuuuug                                                    20


<210>  307
<211>  22
<212>  RNA
<213>  Artificial

<220>
<223>  nucleic acid sequence according to formula (II)

<400>  307
gggggggggg uuuggggggg gg                                                 22


<210>  308
<211>  22
<212>  RNA
<213>  Artificial

<220>
<223>  nucleic acid sequence according to formula (II)

<400>  308

gggggggggu uuuggggggg gg                                              22


<210>  309
<211>  22
<212>  RNA
<213>  Artificial

<220>
<223>  nucleic acid sequence according to formula (II)

<400>  309
   gggggggguu uuuugggggg gg                                              22


<210>  310
<211>  22
<212>  RNA
<213>  Artificial

<220>
<223>  nucleic acid sequence according to formula (II)

<400>  310
   gggggggguu uuuuuggggg gg                                              22


<210>  311
<211>  22
<212>  RNA
<213>  Artificial

<220>
<223>  nucleic acid sequence according to formula (II)

<400>  311
   ggggggguuu uuuuuggggg gg                                              22


<210>  312
<211>  22
<212>  RNA
<213>  Artificial

<220>
<223>  nucleic acid sequence according to formula (II)

<400>  312
   ggggggguuu uuuuuugggg gg                                              22


<210>  313
<211>  22
<212>  RNA
<213>  Artificial

<220>
<223>  nucleic acid sequence according to formula (II)

<400>  313
   ggggggguuu uuuuuuuggg gg                                              22

<210>  314
<211>  22
<212>  RNA
<213>  Artificial

<220>
<223>  nucleic acid sequence according to formula (II)

<400>  314
   gggggguuuu uuuuuuuggg gg                                                22

<210>  315
<211>  22
<212>  RNA
<213>  Artificial

<220>
<223>  nucleic acid sequence according to formula (II)

<400>  315
   gggggguuuu uuuuuuuugg gg                                                22

<210>  316
<211>  22
<212>  RNA
<213>  Artificial

<220>
<223>  nucleic acid sequence according to formula (II)

<400>  316
   ggggguuuuu uuuuuuuugg gg                                                22

<210>  317
<211>  22
<212>  RNA
<213>  Artificial

<220>
<223>  nucleic acid sequence according to formula (II)

<400>  317
   ggggguuuuu uuuuuuuuug gg                                                22

<210>  318
<211>  22
<212>  RNA
<213>  Artificial

<220>
<223>  nucleic acid sequence according to formula (II)

<400>  318
   ggguuuuuuu uuuuuuuuug gg                                                22

<210>  319
<211>  22
<212>  RNA

<213>  Artificial

<220>
<223>  nucleic acid sequence according to formula (II)

<400>  319
gguuuuuuuu uuuuuuuuuu gg                                                22


<210>  320
<211>  24
<212>  RNA
<213>  Artificial

<220>
<223>  nucleic acid sequence according to formula (II)

<400>  320
gggggggggg guuugggggg gggg                                              24


<210>  321
<211>  24
<212>  RNA
<213>  Artificial

<220>
<223>  nucleic acid sequence according to formula (II)

<400>  321
gggggggggg uuuugggggg gggg                                              24


<210>  322
<211>  24
<212>  RNA
<213>  Artificial

<220>
<223>  nucleic acid sequence according to formula (II)

<400>  322
gggggggggu uuuuuggggg gggg                                              24


<210>  323
<211>  24
<212>  RNA
<213>  Artificial

<220>
<223>  nucleic acid sequence according to formula (II)

<400>  323
gggggggggu uuuuuugggg gggg                                              24


<210>  324
<211>  24
<212>  RNA
<213>  Artificial

<220>

<223>  nucleic acid sequence according to formula (II)

<400>  324
gggggggguu uuuuuugggg gggg                                              24


<210>  325
<211>  24
<212>  RNA
<213>  Artificial

<220>
<223>  nucleic acid sequence according to formula (II)

<400>  325
gggggggguu uuuuuuuggg gggg                                              24


<210>  326
<211>  24
<212>  RNA
<213>  Artificial

<220>
<223>  nucleic acid sequence according to formula (II)

<400>  326
gggggggguu uuuuuuuugg gggg                                              24


<210>  327
<211>  24
<212>  RNA
<213>  Artificial

<220>
<223>  nucleic acid sequence according to formula (II)

<400>  327
ggggggguuu uuuuuuuugg gggg                                              24


<210>  328
<211>  24
<212>  RNA
<213>  Artificial

<220>
<223>  nucleic acid sequence according to formula (II)

<400>  328
ggggggguuu uuuuuuuuug gggg                                              24


<210>  329
<211>  24
<212>  RNA
<213>  Artificial

<220>
<223>  nucleic acid sequence according to formula (II)

<400>  329

gggggguuuu uuuuuuuuug gggg                                              24


<210>  330
<211>  24
<212>  RNA
<213>  Artificial

<220>
<223>  nucleic acid sequence according to formula (II)

<400>  330
gggggguuuu uuuuuuuuuu gggg                                              24


<210>  331
<211>  24
<212>  RNA
<213>  Artificial

<220>
<223>  nucleic acid sequence according to formula (II)

<400>  331
gggguuuuuu uuuuuuuuuu gggg                                              24


<210>  332
<211>  24
<212>  RNA
<213>  Artificial

<220>
<223>  nucleic acid sequence according to formula (II)

<400>  332
ggguuuuuuu uuuuuuuuuu uggg                                              24


<210>  333
<211>  32
<212>  RNA
<213>  Artificial

<220>
<223>  nucleic acid sequence according to formula (II)

<400>  333
guuuuuuuuu uuuuuuuuuu uuuuuuuuuu ug                                     32


<210>  334
<211>  34
<212>  RNA
<213>  Artificial

<220>
<223>  nucleic acid sequence according to formula (II)

<400>  334
gguuuuuuuu uuuuuuuuuu uuuuuuuuuu uugg                                   34

<210>  335
<211>  36
<212>  RNA
<213>  Artificial

<220>
<223>  nucleic acid sequence according to formula (II)

<400>  335
   ggguuuuuuu uuuuuuuuuu uuuuuuuuuu uuuggg                                    36


<210>  336
<211>  37
<212>  RNA
<213>  Artificial

<220>
<223>  nucleic acid sequence according to formula (II)

<400>  336
   ggggguuuuu uuuuuuuuuu uuuuuuuuuu uuuuggg                                  37


<210>  337
<211>  39
<212>  RNA
<213>  Artificial

<220>
<223>  nucleic acid sequence according to formula (II)

<400>  337
   gggggguuuu uuuuuuuuuu uuuuuuuuuu uuuuugggg                                39


<210>  338
<211>  41
<212>  RNA
<213>  Artificial

<220>
<223>  nucleic acid sequence according to formula (II)

<400>  338
   ggggggguuu uuuuuuuuuu uuuuuuuuuu uuuuuugggg g                             41


<210>  339
<211>  43
<212>  RNA
<213>  Artificial

<220>
<223>  nucleic acid sequence according to formula (II)

<400>  339
   ggggggguuu uuuuuuuuuu uuuuuuuuuu uuuuuuuggg ggg                          43


<210>  340
<211>  45
<212>  RNA

<213>  Artificial

<220>
<223>  nucleic acid sequence according to formula (II)

<400>  340
gggggggguu uuuuuuuuuu uuuuuuuuuu uuuuuuuugg ggggg                    45


<210>  341
<211>  47
<212>  RNA
<213>  Artificial

<220>
<223>  nucleic acid sequence according to formula (II)

<400>  341
gggggggggu uuuuuuuuuu uuuuuuuuuu uuuuuuuuug ggggggg                 47


<210>  342

<400>  342
000

<210>  343

<400>  343
000

<210>  344

<400>  344
000

<210>  345
<211>  10
<212>  RNA
<213>  Artificial

<220>
<223>  nucleic acid sequence according to formula (II)

<400>  345
gguuuuuugg                                                           10


<210>  346
<211>  11
<212>  RNA
<213>  Artificial

<220>
<223>  nucleic acid sequence according to formula (II)

<400>  346
gguuuuuuug g                                                         11


<210>  347
<211>  12
<212>  RNA

<213>  Artificial

<220>
<223>  nucleic acid sequence according to formula (II)

<400>  347
gguuuuuuuu gg                                                          12


<210>  348
<211>  13
<212>  RNA
<213>  Artificial

<220>
<223>  nucleic acid sequence according to formula (II)

<400>  348
gguuuuuuuu ugg                                                         13


<210>  349
<211>  14
<212>  RNA
<213>  Artificial

<220>
<223>  nucleic acid sequence according to formula (II)

<400>  349
gguuuuuuuu uugg                                                        14


<210>  350
<211>  15
<212>  RNA
<213>  Artificial

<220>
<223>  nucleic acid sequence according to formula (II)

<400>  350
gguuuuuuuu uuugg                                                       15


<210>  351
<211>  16
<212>  RNA
<213>  Artificial

<220>
<223>  nucleic acid sequence according to formula (II)

<400>  351
gguuuuuuuu uuuugg                                                      16


<210>  352
<211>  17
<212>  RNA
<213>  Artificial

<220>

<223>  nucleic acid sequence according to formula (II)

<400>  352
gguuuuuuuu uuuuugg                                                      17


<210>  353
<211>  18
<212>  RNA
<213>  Artificial

<220>
<223>  nucleic acid sequence according to formula (II)

<400>  353
gguuuuuuuu uuuuuugg                                                     18


<210>  354
<211>  19
<212>  RNA
<213>  Artificial

<220>
<223>  nucleic acid sequence according to formula (II)

<400>  354
gguuuuuuuu uuuuuuugg                                                    19


<210>  355

<400>  355
000

<210>  356
<211>  10
<212>  RNA
<213>  Artificial

<220>
<223>  nucleic acid sequence according to formula (II)

<400>  356
ggguuuuggg                                                              10


<210>  357
<211>  11
<212>  RNA
<213>  Artificial

<220>
<223>  nucleic acid sequence according to formula (II)

<400>  357
ggguuuuugg g                                                            11


<210>  358
<211>  12
<212>  RNA
<213>  Artificial

<220>
<223>  nucleic acid sequence according to formula (II)

<400>  358
ggguuuuuug gg                                                          12


<210>  359
<211>  13
<212>  RNA
<213>  Artificial

<220>
<223>  nucleic acid sequence according to formula (II)

<400>  359
ggguuuuuuu ggg                                                         13


<210>  360
<211>  14
<212>  RNA
<213>  Artificial

<220>
<223>  nucleic acid sequence according to formula (II)

<400>  360
ggguuuuuuu uggg                                                        14


<210>  361
<211>  15
<212>  RNA
<213>  Artificial

<220>
<223>  nucleic acid sequence according to formula (II)

<400>  361
ggguuuuuuu uuggg                                                       15


<210>  362
<211>  16
<212>  RNA
<213>  Artificial

<220>
<223>  nucleic acid sequence according to formula (II)

<400>  362
ggguuuuuuu uuuggg                                                      16


<210>  363
<211>  17
<212>  RNA
<213>  Artificial

<220>
<223>  nucleic acid sequence according to formula (II)

<400>  363
ggguuuuuuu uuuuggg                                                    17


<210>  364
<211>  18
<212>  RNA
<213>  Artificial

<220>
<223>  nucleic acid sequence according to formula (II)

<400>  364
ggguuuuuuu uuuuuggg                                                   18


<210>  365
<211>  19
<212>  RNA
<213>  Artificial

<220>
<223>  nucleic acid sequence according to formula (II)

<400>  365
ggguuuuuuu uuuuuuggg                                                  19


<210>  366
<211>  57
<212>  RNA
<213>  Artificial

<220>
<223>  nucleic acid sequence according to formula (II)

<400>  366
ggguuuuuuu uuuuuuuugg guuuuuuuuu uuuuuugggu uuuuuuuuuu uuuuggg          57


<210>  367
<211>  42
<212>  RNA
<213>  Artificial

<220>
<223>  nucleic acid sequence according to formula (II)

<400>  367
ggguuuuuuu uuuuuuuugg gggguuuuuu uuuuuuuuug gg                          42


<210>  368
<211>  51
<212>  RNA
<213>  Artificial

<220>
<223>  nucleic acid sequence according to formula (III)

<400>  368
ggguuugggu uuggguuugg guuuggguuu ggguuugggu uuggguuugg g               51

<210>  369
<211>  20
<212>  RNA
<213>  Artificial Sequence

<220>
<223>  nucleic acid sequence according to formula (II) - Short GU rich

<400>  369
gguuuuuuuu uuuuuuuggg                                                    20


<210>  370
<211>  57
<212>  RNA
<213>  Artificial

<220>
<223>  nucleic acid sequence according to formula (III)

<400>  370
cccuuuuuuu uuuuuuuucc cuuuuuuuuu uuuuuucccu uuuuuuuuuu uuuuccc          57


<210>  371
<211>  51
<212>  RNA
<213>  Artificial

<220>
<223>  nucleic acid sequence according to formula (III)

<400>  371
cccuuucccu uucccuuucc cuuucccuuu cccuuucccu uucccuuucc c                51


<210>  372
<211>  42
<212>  RNA
<213>  Artificial

<220>
<223>  nucleic acid sequence according to formula (III)

<400>  372
cccuuuuuuu uuuuuuuucc ccccuuuuuu uuuuuuuuuc cc                          42


<210>  373
<211>  60
<212>  RNA
<213>  Artificial Sequence

<220>
<223>  nucleic acid sequence according to formula (IV)

<400>  373
uagcgaagcu cuuggaccua gguuuuuuuu uuuuuuuggg ugcguuccua gaaguacacg        60


<210>  374

<211>  120
<212>  RNA
<213>  Artificial Sequence

<220>
<223>  nucleic acid sequence according to formula (IV)

<400>  374
uagcgaagcu cuuggaccua gguuuuuuuu uuuuuuuggg ugcguuccua gaaguacacg      60

aucgcuucga gaaccuggau ccaaaaaaaa aaaaaaaccc acgcaaggau cuucaugugc      120


<210>  375
<211>  229
<212>  RNA
<213>  Artificial Sequence

<220>
<223>  nucleic acid sequence according to formula (IV)

<400>  375
gggagaaagc ucaagcuugg agcaaugccc gcacauugag gaaaccgagu ugcauaucuc      60

agaguauugg cccccgugua gguuauucuu gacagacagu ggagcuuauu cacucccagg      120

auccgagucg cauacuacgg uacuggugac agaccuaggu cgucaguuga ccaguccgcc      180

acuagacgug aguccgucaa agcaguuaga uguuacacuc uauuagauc      229


<210>  376
<211>  547
<212>  RNA
<213>  Artificial Sequence

<220>
<223>  nucleic acid sequence according to formula (IV)

<400>  376
gggagaaagc ucaagcuugg agcaaugccc gcacauugag gaaaccgagu ugcauaucuc      60

agaguauugg cccccgugua gguuauucuu gacagacagu ggagcuuauu cacucccagg      120

auccgagucg cauacuacgg uacuggugac agaccuaggu cgucaguuga ccaguccgcc      180

acuagacgug aguccgucaa agcaguuaga uguuacacuc uauuagaucu cggauuacag      240

cuggaaggag caggaguagu guucuugcuc uaaguaccga gugugcccaa uacccgauca      300

gcuuauuaac gaacggcucc uccucuuaga cugcagcgua agugcggaau cuggggauca      360

aauuacugac ugccuggauu acccucggac auauaaccuu guagcacgcu guugcuguau      420

aggugaccaa cgcccacucg aguagaccag cucucuuagu ccggacaaug auaggaggcg      480

cggucaaucu acuucuggcu aguuaagaau aggcugcacc gaccucuaua aguagcgugu      540

ccucuag      547


<210>  377
<211>  1083

<212>  RNA
<213>  Artificial Sequence

<220>
<223>  nucleic acid sequence according to formula (IV)

<400>  377
gggagaaagc ucaagcuugg agcaaugccc gcacauugag gaaaccgagu ugcauaucuc      60

agaguauugg cccccgugua gguuauucuu gacagacagu ggagcuuauu cacucccagg     120

auccgagucg cauacuacgg uacuggugac agaccuaggu cgucaguuga ccaguccgcc     180

acuagacgug aguccgucaa agcaguuaga uguuacacuc uauuagaucu cggauuacag     240

cuggaaggag caggaguagu guucuugcuc uaaguaccga gugugcccaa uacccgauca     300

gcuuauuaac gaacggcucc uccucuuaga cugcagcgua agugcggaau cuggggauca     360

aauuacugac ugccuggauu acccucggac auauaaccuu guagcacgcu guugcuguau     420

aggugaccaa cgcccacucg aguagaccag cucucuuagu ccggacaaug auaggaggcg     480

cggucaaucu acuucuggcu aguuaagaau aggcugcacc gaccucuaua aguagcgugu     540

ccucuagagc uacgcagguu cgcaauaaaa gcguugauua gugugcauag aacagaccuc     600

uuauucggug aaacgccaga augcuaaauu ccaauaacuc uucccaaaac gcguacggcc     660

gaagacgcgc gcuuaucuug uguacguucu cgcacaugga agaaucagcg ggcauggugg     720

uagggcaaua ggggagcugg guagcagcga aaaagggccc cugcgcacgu agcuucgcug     780

uucgucugaa acaacccggc auccguugua gcgaucccgu uaucaguguu auucuugugc     840

gcacuaagau ucauggugua gucgacaaua acagcgucuu ggcagauucu ggucacgugc     900

ccuaugcccg ggcuugugcc ucucaggugc acagcgauac uuaaagccuu caagguacuc     960

gacgugggua ccgauucgug acacuuccua agauuauucc acuguguuag ccccgcaccg    1020

ccgaccuaaa cugguccaau guauacgcau ucgcugagcg gaucgauaau aaaagcuuga    1080

auu                                                                  1083


<210>  378
<211>  229
<212>  RNA
<213>  Artificial Sequence

<220>
<223>  nucleic acid sequence according to formula (IV)

<400>  378
gggagaaagc ucaagcuuau ccaaguaggc uggucaccug uacaacguag ccgguauuuu      60

uuuuuuuuuu uuuuuuuuga ccgucucaag guccaaguua gucugccuau aaaggugcgg     120

auccacagcu gaugaaagac uugugcggua cgguuaaucu ccccuuuuuu uuuuuuuuuu     180

uuuuuaguaa augcgucuac ugaauccagc gaugaugcug gcccagauc                229

<210> 379
<211> 546
<212> RNA
<213> Artificial Sequence

<220>
<223> nucleic acid sequence according to formula (IV)

<400> 379
gggagaaagc ucaagcuuau ccaaguaggc uggucaccug uacaacguag ccgguauuuu 60

uuuuuuuuuu uuuuuuuuga ccgucucaag guccaaguua gucugccuau aaaggugcgg 120

auccacagcu gaugaaagac uugugcggua cgguuaaucu ccccuuuuuu uuuuuuuuuu 180

uuuuuaguaa augcgucuac ugaauccagc gaugaugcug gcccagaucu ucgaccacaa 240

gugcauauag uagucaucga gggucgccuu uuuuuuuuuu uuuuuuuuuu uggcccaguu 300

cugagacuuc gcuagagacu acaguuacag cugcaguagu aaccacugcg gcuauugcag 360

gaaaucccgu ucagguuuuu uuuuuuuuuu uuuuuuccgc ucacuaugau uaagaaccag 420

guggaguguc acugcucucg aggucucacg agagcgcucg auacaguccu uggaagaauc 480

uuuuuuuuuu uuuuuuuuuu uugugcgacg aucacagaga acuucuauuc augcaggucu 540

gcucua 546

<210> 380
<211> 1083
<212> RNA
<213> Artificial Sequence

<220>
<223> nucleic acid sequence according to formula (IV)

<400> 380
gggagaaagc ucaagcuuau ccaaguaggc uggucaccug uacaacguag ccgguauuuu 60

uuuuuuuuuu uuuuuuuuga ccgucucaag guccaaguua gucugccuau aaaggugcgg 120

auccacagcu gaugaaagac uugugcggua cgguuaaucu ccccuuuuuu uuuuuuuuuu 180

uuuuuaguaa augcgucuac ugaauccagc gaugaugcug gcccagaucu ucgaccacaa 240

gugcauauag uagucaucga gggucgccuu uuuuuuuuuu uuuuuuuuuu uggcccaguu 300

cugagacuuc gcuagagacu acaguuacag cugcaguagu aaccacugcg gcuauugcag 360

gaaaucccgu ucagguuuuu uuuuuuuuuu uuuuuuccgc ucacuaugau uaagaaccag 420

guggaguguc acugcucucg aggucucacg agagcgcucg auacaguccu uggaagaauc 480

uuuuuuuuuu uuuuuuuuuu uugugcgacg aucacagaga acuucuauuc augcaggucu 540

gcucuagaac gaacugaccu gacgccugaa cuuaugagcg ugcguauuuu uuuuuuuuuu 600

uuuuuuuuuc cucccaacaa augucgauca auagcugggc uguuggagac gcgucagcaa 660

augccguggc uccauaggac guguagacuu cuauuuuuuu uuuuuuuuuu uuuucccggg 720

accacaaaua auauucuugc uugguugggc gcaagggccc cguaucaggu cauaaacggg 780

uacauguugc acaggcuccu uuuuuuuuuu uuuuuuuuuu uucgcugagu uauuccgguc 840

ucaaaagacg gcagacguca gucgacaaca cggucuaaag cagugcuaca aucugccgug 900

uucguguuuu uuuuuuuuuu uuuuuuguga accuacacgg cgugcacugu aguucgcaau 960

ucauagggua ccggcucaga guuaugccuu gguugaaaac ugcccagcau acuuuuuuuu 1020

uuuuuuuuuu uucauauucc caugcuaagc aagggaugcc gcgagucaug uuaagcuuga 1080

auu 1083

<210> 381
<211> 59
<212> RNA
<213> Artificial Sequence

<220>
<223> nucleic acid sequence according to formula (V)

<400> 381
uagcgaagcu cuuggaccua ccuuuuuuuu uuuuuucccu gcguuccuag aaguacacg 59

<210> 382
<211> 120
<212> RNA
<213> Artificial Sequence

<220>
<223> nucleic acid sequence according to formula (V)

<400> 382
uagcgaagcu cuuggaccua ccuuuuuuuu uuuuuuuccc ugcguuccua gaaguacacg 60

aucgcuucga gaaccuggau ggaaaaaaaa aaaaaaaggg acgcaaggau cuucaugugc 120

<210> 383
<211> 15
<212> RNA
<213> Artificial

<220>
<223> Description of sequence: generic stabilizing sequence of the
formula (C/U)CCANxCCC(U/A)PyxUC(C/U)CC

<220>
<221> variation
<222> (1)..(1)
<223> /replace="cytosine" /replace="uracile"

<220>
<221> misc_feature
<222> (1)..(1)
<223> nucleic acid = cytosine or uracil

<220>

<221>  misc_feature
<222>  (5)..(5)
<223>  Nx = a, g, c or u or any other nucleic acid

<220>
<221>  variation
<222>  (5)..(5)
<223>  /replace="cytosine" /replace="uracile" /replace="guanosine"
       /replace="adenosine", or any other nucleic acid

<220>
<221>  repeat_unit
<222>  (5)..(5)
<223>  x = any number

<220>
<221>  misc_feature
<222>  (9)..(9)
<223>  nucleic acid = uracil or adenosine

<220>
<221>  variation
<222>  (9)..(9)
<223>  /replace="uracile" /replace="adonosine"

<220>
<221>  misc_feature
<222>  (10)..(10)
<223>  Py = pyrimidine

<220>
<221>  repeat_unit
<222>  (10)..(10)
<223>  x = any number

<220>
<221>  variation
<222>  (10)..(10)
<223>  /replace="pyrimidine"

<220>
<221>  misc_feature
<222>  (13)..(13)
<223>  nucleic acid = cytosine or uracil

<220>
<221>  variation
<222>  (13)..(13)
<223>  /replace="cytosine" /replace="uracile"

<400>  383
nccancccnn ucncc                                                        15


<210>  384
<211>  1698
<212>  RNA
<213>  Artificial Sequence

<220>
<223>  PR8 H1 mRNA

<400>  384

augaaggcca accugcucgu gcugcugugc gcccucgcgg ccgccgacgc cgacaccauc 60

ugcaucggcu accacgccaa caacagcacc gacacggucg acaccgugcu ggagaagaac 120

gugaccguca cccacuccgu gaaccugcuc gaggacagcc acaacgggaa gcugugccgg 180

cugaagggca ucgcgccccu ccagcugggg aagugcaaca ucgccggcug gcugcucggg 240

aacccggagu gcgacccccu gcugcccgug cgcuccugga gcuacaucgu cgagacgccc 300

aacuccgaga acggcaucug cuacccgggc gacuucaucg acuacgagga gcuccgggag 360

cagcugagcu ccgugagcuc cuucgagcgc uucgagaucu uccccaagga gagcuccugg 420

cccaaccaca acaccaacgg ggugaccgcc gccugcagcc acgagggcaa guccagcuuc 480

uaccggaacc ugcucuggcu gaccgagaag gagggguccu accccaagcu gaagaacagc 540

uacgucaaca agaagggcaa ggaggugcuc gugcuguggg ggauccacca cccgcccaac 600

uccaaggagc agcagaaccu guaccagaac gagaacgcgu acgucagcgu ggugacgucc 660

aacuacaacc gccgguucac ccccgagauc gccgagcgcc ccaagguccg ggaccaggcc 720

ggccgcauga acuacuacug gacccuccug aagccgggcg acaccaucau cuucgaggcc 780

aacgggaacc ugaucgcccc gauguacgcg uucgcccuca gccggggcuu cgggagcggc 840

aucaucacgu ccaacgccag caugcacgag ugcaacacca agugccagac cccccugggc 900

gccaucaacu ccagccugcc cuaccagaac auccacccgg ugaccaucgg ggagugcccc 960

aaguacgugc gcuccgccaa gcuccggaug gucacgggcc ugcgcaacaa ccccagcauc 1020

caguccgggg ggcuguucgg cgcgaucgcc ggguucaucg agggcggcug gaccgggaug 1080

aucgacggcu gguacgggua ccaccaccag aacgagcagg gcagcgggua cgccgccgac 1140

cagaagucca cccagaacgc caucaacggc aucaccaaca aggugaacac ggugaucgag 1200

aagaugaaca uccaguucac cgcggucggc aaggaguuca acaagcucga gaagcgcaug 1260

gagaaccuga acaagaaggu ggacgacggg uuccuggaca ucuggaccua caacgccgag 1320

cuccuggugc ugcucgagaa cgagcggacc cuggacuucc acgacagcaa cgucaagaac 1380

cuguacgaga aggugaaguc ccagcucaag aacaacgcca aggagaucgg caacgggugc 1440

uucgaguucu accacaagug cgacaacgag ugcauggaga gcguccgcaa cggcacguac 1500

gacuacccca aguacuccga ggagagcaag cugaaccggg agaaggugga cggggugaag 1560

cuggagucca ugggcaucua ccagauccuc gccaucuaca gcaccgucgc cuccagccug 1620

gugcugcugg ugucccucgg cgcgaucagc uucuggaugu gcagcaacgg gucccugcag 1680

ugccgcaucu gcaucuga 1698

<210> 385
<211> 1161
<212> RNA
<213> Artificial Sequence

<220>
<223>  Ovalbumin (control) mRNA

<400>  385
augggcagca ucggggccgc gucgauggag uucugcuucg acguguucaa ggagcugaag      60

guccaccacg ccaacgagaa caucuucuac ugcccgaucg ccaucaugag cgcgcucgcc     120

augguguacc ugggcgccaa ggacagcacc cggacgcaga ucaacaaggu gguccgcuuc     180

gacaagcugc ccggcuucgg ggacucgauc gaggcgcagu gcggcaccag cgugaacgug     240

cacagcucgc uccgggacau ccugaaccag aucaccaagc cgaacgacgu cuacagcuuc     300

agccuggccu cgcggcucua cgccgaggag cgcuacccga uccugcccga guaccugcag     360

ugcgugaagg agcucuaccg gggcgggcug gagccgauca acuuccagac ggcggccgac     420

caggcccggg agcugaucaa cagcugggug gagagccaga ccaacggcau cauccgcaac     480

guccuccagc cgucgagcgu ggacagccag accgcgaugg ugcuggucaa cgccaucgug     540

uucaagggcc ugugggagaa gacguucaag gacgaggaca cccaggccau gcccuuccgg     600

gugaccgagc aggagucgaa gccgguccag augauguacc agaucgggcu cuuccgggug     660

gcgagcaugg ccagcgagaa gaugaagauc cuggagcugc cguucgccuc gggcacgaug     720

agcaugcucg ugcugcugcc cgacgagguc agcggccucg agcagcugga gucgaucauc     780

aacuucgaga agcugaccga guggaccagc agcaacguga uggaggagcg caagaucaag     840

guguaccucc cgcggaugaa gauggaggag aaguacaacc ugacgucggu ccugauggcg     900

auggggauca ccgacguguu cagcagcucg gccaaccuca gcggcaucag cucggccgag     960

agccugaaga ucagccaggc ggugcacgcc gcccacgcgg agaucaacga ggccggccgg    1020

gaggucgugg ggucggccga ggcgggcgug gacgccgcca gcgucagcga ggaguuccgc    1080

gcggaccacc cguuccuguu cugcaucaag cacaucgcca ccaacgccgu gcucuucuuc    1140

ggccggugcg ugucgcccug a                                              1161


<210>  386
<211>  990
<212>  DNA
<213>  Artificial Sequence

<220>
<223>  RNActiveII KLK3(GC) = PSA

<400>  386
gggagaaagc ttaccatgtg ggtgccggtc gtgttcctga ccctcagcgt gacgtggatc      60

ggcgccgcgc ccctgatcct gtcgcggatc gtggggggct gggagtgcga gaagcacagc     120

cagccctggc aggtgctggt ggccagccgc ggccgggccg tgtgcggcgg cgtgctggtg     180

cacccccagt gggtgctgac cgccgcccac tgcatccgga acaagagcgt catcctgctg     240

ggccggcaca gcctgttcca ccccgaggac accggccagg tgttccaggt gagccacagc 300

ttcccccacc ccctgtacga catgagcctc ctgaagaacc ggttcctgcg gcccggcgac 360

gacagcagcc acgacctgat gctgctgcgg ctgagcgagc ccgccgagct gaccgacgcc 420

gtgaaggtga tggacctgcc gacccaggag cccgccctgg gcaccacctg ctacgccagc 480

ggctggggga gcatcgagcc cgaggagttc ctcaccccca agaagctgca gtgcgtggac 540

ctgcacgtga tcagcaacga cgtgtgcgcc caggtgcacc cccagaaggt gaccaagttc 600

atgctgtgcg ccggccggtg gaccggcggc aagagcacct gcagcggcga cagcggcggc 660

cccctggtct gcaacggcgt gctgcagggc atcaccagct ggggcagcga gccctgcgcc 720

ctgcccgagc gccccagcct gtacaccaag gtggtgcact accggaagtg gatcaaggac 780

accatcgtgg ccaacccgtg accactagtt ataagactga ctagcccgat gggcctccca 840

acgggccctc ctcccctcct tgcaccgaga ttaataaaaa aaaaaaaaaa aaaaaaaaaa 900

aaaaaaaaaa aaaaaaaaaa aaaaaaaaaa aaaaaaaaat attccccccc cccccccccc 960

cccccccccc ccctctagac aattggaatt 990

<210> 387
<211> 2457
<212> DNA
<213> Artificial Sequence

<220>
<223> RNActiveII FOLH1(GC) = PSMA

<400> 387
gggagaaagc ttaccatgtg gaacctgctc cacgagaccg acagcgccgt ggcgacggcc 60

cggcgcccgc ggtggctgtg cgccggcgcc ctggtcctgg ccgggggctt cttcctgctg 120

ggcttcctgt tcggctggtt catcaagtcg agcaacgagg ccaccaacat cacccccaag 180

cacaacatga aggccttcct cgacgagctg aaggccgaga acatcaagaa gttcctgtac 240

aacttcaccc agatccccca cctggccggg accgagcaga acttccagct ggccaagcag 300

atccagagcc agtggaagga gttcggcctg gactcggtgg agctggcgca ctacgacgtg 360

ctgctcagct accccaacaa gacccacccc aactacatca gcatcatcaa cgaggacggc 420

aacgagatct tcaacaccag cctgttcgag cccccgcccc ccggctacga gaacgtgtcg 480

gacatcgtgc cccccttcag cgccttcagc ccgcagggca tgcccgaggg ggacctggtg 540

tacgtgaact acgcccggac ggaggacttc ttcaagctgg agcgcgacat gaagatcaac 600

tgcagcggca agatcgtgat cgcccggtac ggcaaggtgt tccggggcaa caaggtgaag 660

aacgcccagc tggccggggc caagggcgtg atcctgtact cggaccccgc cgactacttc 720

gcccccggcg tgaagagcta ccccgacggc tggaacctgc ccggcggggg cgtccagcgc 780

ggcaacatcc tcaacctgaa cggcgccggc gacccgctga cccccgggta ccccgcgaac 840

gagtacgcct accggcgggg catcgccgag gccgtgggcc tgcccagcat ccccgtgcac 900

ccgatcggct actacgacgc ccagaagctg ctggagaaga tgggcgggag cgccccgccc 960

gactcgagct ggcggggcag cctgaaggtg ccctacaacg tgggccccgg cttcaccggg 1020

aacttctcga cccagaaggt gaagatgcac atccacagca ccaacgaggt gacccgcatc 1080

tacaacgtga tcggcaccct gcggggcgcc gtggagcccg accggtacgt gatcctcggc 1140

gggcaccgcg acagctgggt gttcggcggc atcgaccccc agagcggcgc cgccgtggtc 1200

cacgagatcg tgcggtcgtt cggcaccctg aagaaggagg ggtggcggcc ccgccggacg 1260

atcctgttcg ccagctggga cgcggaggag ttcggcctgc tgggcagcac cgagtgggcc 1320

gaggagaaca gccggctgct gcaggagcgg ggcgtggcct acatcaacgc cgactcgagc 1380

atcgagggca actacaccct ccgcgtggac tgcaccccgc tgatgtacag cctggtgcac 1440

aacctgacca aggagctgaa gagccccgac gaggggttcg agggcaagtc gctgtacgag 1500

agctggacca agaagagccc ctcgcccgag ttcagcggca tgccccggat cagcaagctg 1560

ggcagcggga acgacttcga ggtgttcttc cagcggctgg gcatcgcctc gggccgcgcc 1620

cggtacacca agaactggga gacgaacaag ttcagcggct accccctcta ccacagcgtg 1680

tacgagacct acgagctggt ggagaagttc tacgacccca tgttcaagta ccacctgacc 1740

gtggcccagg tgcggggcgg gatggtgttc gagctggcca acagcatcgt gctgcccttc 1800

gactgccgcg actacgccgt cgtgctgcgg aagtacgccg acaagatcta ctcgatcagc 1860

atgaagcacc cccaggagat gaagacctac agcgtgagct tcgactcgct gttcagcgcg 1920

gtgaagaact tcaccgagat cgccagcaag ttctcggagc ggctccagga cttcgacaag 1980

agcaacccga tcgtgctgcg catgatgaac gaccagctga tgttcctgga gcgggccttc 2040

atcgaccccc tgggcctgcc cgaccggccc ttctaccggc acgtgatcta cgcccccagc 2100

agccacaaca agtacgccgg cgagtcgttc ccggggatct acgacgccct gttcgacatc 2160

gagagcaagg tggaccccag caaggcctgg ggcgaggtga agcgccagat ctacgtggcc 2220

gccttcaccg tgcaggccgc ggccgagacc ctgagcgagg tggcctgacc actagttata 2280

agactgacta gcccgatggg cctcccaacg ggccctcctc ccctccttgc accgagatta 2340

ataaaaaaaa aaaaaaaaaa aaaaaaaaaa aaaaaaaaaa aaaaaaaaaa aaaaaaaaaa 2400

aaaaaatatt cccccccccc cccccccccc cccccccccc tctagacaat tggaatt 2457

<210> 388
<211> 576
<212> DNA
<213> Artificial Sequence

<220>
<223> RNActiveII PSCA(GC)

<400>  388
gggagaaagc ttaccatgaa ggccgtgctg ctcgcgctgc tgatggccgg cctggccctg      60

cagccgggga ccgccctgct gtgctacagc tgcaaggccc aggtctcgaa cgaggactgc     120

ctgcaggtgg agaactgcac gcagctgggc gagcagtgct ggaccgcccg gatccgcgcc     180

gtgggcctgc tcaccgtgat cagcaagggc tgcagcctga actgcgtgga cgacagccag     240

gactactacg tgggcaagaa gaacatcacc tgctgcgaca ccgacctgtg caacgccagc     300

ggcgcccacg ccctgcagcc cgcggccgcc atcctggccc tgctgcccgc cctgggcctg     360

ctgctctggg gccccggcca gctgtgacca ctagttataa gactgactag cccgatgggc     420

ctcccaacgg gccctcctcc cctccttgca ccgagattaa taaaaaaaaa aaaaaaaaaa     480

aaaaaaaaaa aaaaaaaaaa aaaaaaaaaa aaaaaaaaaa aaaaatattc cccccccccc     540

cccccccccc ccccccccct ctagacaatt ggaatt                              576


<210>  389
<211>  1224
<212>  DNA
<213>  Artificial Sequence

<220>
<223>  RNActive II STEAP (GC) = STEAP1

<400>  389
gggagaaagc ttaccatgga gagccggaag gacatcacca accaggagga gctgtggaag      60

atgaagccgc gccggaacct cgaggaggac gactacctgc acaaggacac gggcgagacc     120

tcgatgctga agcggcccgt gctcctgcac ctgcaccaga ccgcccacgc ggacgagttc     180

gactgcccga gcgagctcca gcacacgcag gagctgttcc cgcagtggca cctgcccatc     240

aagatcgccg ccatcatcgc gagcctcacc ttcctgtaca ccctgctccg cgaggtcatc     300

cacccgctgg ccacgtcgca ccagcagtac ttctacaaga tcccgatcct ggtgatcaac     360

aaggtgctcc ccatggtcag catcaccctg ctggccctcg tgtacctgcc gggggtgatc     420

gcggccatcg tccagctgca caacggcacc aagtacaaga agttcccgca ctggctcgac     480

aagtggatgc tgacgcggaa gcagttcggc ctgctcagct tcttcttcgc cgtgctgcac     540

gcgatctact cgctgagcta ccccatgcgg cgcagctacc ggtacaagct cctgaactgg     600

gcctaccagc aggtgcagca gaacaaggag gacgcctgga tcgagcacga cgtctggcgg     660

atggagatct acgtgtcgct ggggatcgtg ggcctcgcga tcctggccct gctcgccgtc     720

accagcatcc cgagcgtgtc ggacagcctg acctggcgcg agttccacta catccagagc     780

aagctgggca tcgtgtcgct cctgctgggg acgatccacg cgctcatctt cgcctggaac     840

aagtggatcg acatcaagca gttcgtctgg tacaccccgc ccaccttcat gatcgccgtg     900

ttcctgccga tcgtggtcct gatcttcaag agcatcctct tcctgccgtg cctgcggaag     960

aagatcctca agatccggca cggctgggag gacgtgacga agatcaacaa gaccgagatc 1020

tgcagccagc tgtgaccact agttataaga ctgactagcc cgatgggcct cccaacgggc 1080

cctcctcccc tccttgcacc gagattaata aaaaaaaaaa aaaaaaaaaa aaaaaaaaaa 1140

aaaaaaaaaa aaaaaaaaaa aaaaaaaaaa aaatattccc cccccccccc cccccccccc 1200

cccccctctc agacaattgg aatt 1224